# EUROPEAN PATENT SPECIFICATION

(11) **EP 0 398 231 B1**
(45) Date of publication and mention of the grant of the patent: **16.07.1997**
(21) Application number: 90109066.2
(22) Date of filing: 14.05.1990
(51) Int. Cl.: C07H 19/10, C07H 19/11, C07H 19/20, C07H 19/213, A61K 31/70, C07D 473/30, C07D 405/04, A61K 31/505, A61K 31/52, C07H 21/04, C07F 9/6561, C07F 9/6571, C07F 9/6512, C07F 9/6558

(54) **Nucleoside analogs**
Nucleosidanaloge
Analogues nucléosides

(30) Priority: 15.05.1989 US 352303; 22.02.1990 US 481569
(43) Date of publication of application: 22.11.1990
(73) Proprietor: INSTITUTE OF ORGANIC CHEMISTRY AND BIOCHEMISTRY OF THE ACADEMY OF SCIENCES OF THE CZECH REPUBLIC, 166 10 Praha 6 (CZ); Rega Stichting Vzw., B- 3000 Leuven (BE)
(72) Inventor: Kim, Choung Un, Madison, Connecticut 06443 (US); Martin, John C., Cheshire, Connecticut 06410 (US); Misco, Peter F., Durham, Connecticut 06422 (US); Luh, Bing Yu, Killingworth, Connecticut 06417 (US)
(74) Representative: Kinzebach, Werner, Dr.

(56) References cited:
- EP-A- 0 044 527
- WO-A-84/04748
- WO-A-89/12061
- FR-A- 2 539 132
- US-A- 3 457 255
- JOURNAL OF MEDICINAL CHEMISTRY vol. 29, no. 5, 1986, WASHINGTON US pages 671 - 5 E.J.PRISBE ET AL. 'Synthesis of Antiherpes Virus Activity of Phosphate and Phosphonate Derivatives of 9-((1,3-Dihydro xy-2-propoxy)methyl)guanine.'
- CHEMICAL ABSTRACTS, vol. 107, 1987, Columbus, Ohio, US; abstract no. 236377g, R.A.FOGUET ET AL. 'Process for the Preparation of 9-(2-Hydroxyethoxymethyl)guanine, used as an Antiviral Agent.' page 757 ;column 1 ;
- CHEMICAL ABSTRACTS, vol. 111, 1989, Columbus, Ohio, US; abstract no. 205325s, Y.KANEKO 'Light Fading Inhibitor for Color Photographic Material.' page 716 ;column 1 ;
- J.A.C.S. 94(9), 3213-3218, 1973

## Description

### BACKGROUND OF THE INVENTION

### Field of the Invention

The present invention concerns nucleotide analogs and their compositions and use. In particular, the invention concerns antiviral (including antiretroviral) and antitumor phosphonomethoxymethyloxymethyl purine/pyrimidine derivatives and 5'-phosphonomethoxytetrahydrofur-2'-yl purine/pyrimidine derivatives.

### Background Information

Infectious viral diseases are recognized as an important medical problem. Progress against infectious viral disease requires the development of drugs with selective antiviral activity while remaining benign to normal cell lines. A number of antiviral agents currently under study which seem to possess some selectivity are nucleoside analogs. In general, these compounds are structural analogs of the naturally occurring nucleosides. Structural modification in either the purine or pyrimidine base nucleus and/or the saccharide component results in a synthetically modified nucleoside derivative which, when incorporated into a viral nucleic acid forming process, acts to disrupt further synthesis of viral nucleic acid.

Effectiveness of these antiviral agents depends on selective conversion by viral enzymes, but not by host enzymes, to the corresponding nucleotide analog which is then converted to the triphosphate and incorporation into viral nucleic acid occurs. A problem with this antiviral strategy has been the emergence of certain viral strains whose enzymes poorly promote phosphorylation of the nucleoside analogs. To circumvent this problem, intact nucleotide analogs appear to be potentially quite useful as antivirals for incorporation into viral nucleic acid.

Reist and Sturm in WO 84/04748, published December 6, 1984, disclosed new phosphonic acid analogs of nucleoside phosphates which are useful as antivirals for incorporation into viral DNA. The structural formula for these compounds is shown below as 1*'*.

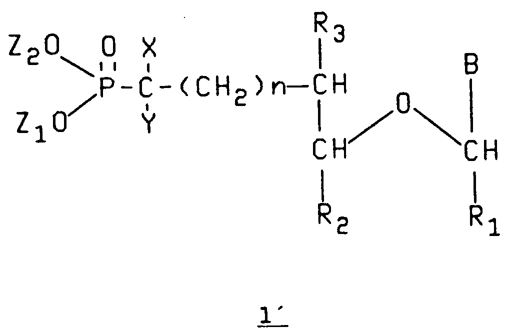

In the Reist and Sturm compounds, B is a purine or pyrimidine base: R₁ and R₂ together complete a beta-pentofuranose sugar or R₁ is H and R₂ is H or hydroxmethyl; R₃ is H or OH: X is H, OH or together with Y is carbonyl oxygen and Y can also be H; Z₁ and Z₂ are H or alkyl.

Similarly, synthesis and anti-Herpes-Virus activity of phosphate and phosphonate derivatives of 9-[(1,3-dihydroxy-2-propoxy)methyl]quanine (Formula 2*'* was disclosed by Prisbe, et al., in J. Med. Chem., 1986, 29, 671.

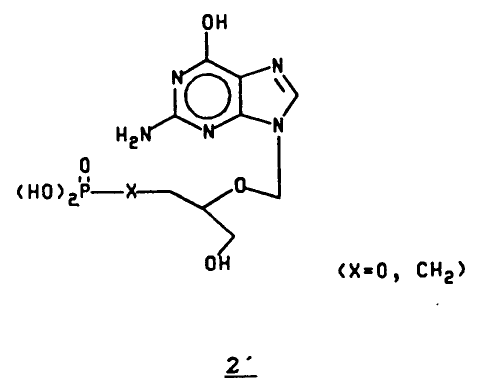

Other phosphonate nucleotide analogs of the Formula 2*'* type wherein X=CH₂ have been described by R. M. Riggs et al., Nucleosides and Nucleotides, 8(5&6, 1119-1120 (1989); D.H.R. Bouton, et al., Tetrahedron Letters, Vol. 30, No. 37, pp 4969-4972 (1972); and H. Tanaka, et al., Tetrahedron Letters, 30, 2567-2570 (1989).

Adenine phosphonic analogs (Formula 3*'*) and their syntheses are disclosed in the UK Patent Application of Holy, et al., GB 2,134,907A published 8/22/84.

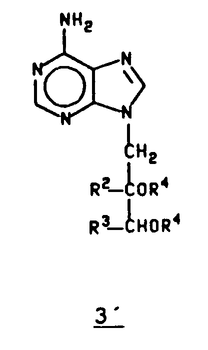

In formula 3*'*, R₂ and R₃ are H or together complete a ribonucleoside ring; and both R₄ are alternately a hydrogen and -CH₂P(O) (OH)₂ group.

A preferred example of one of these compounds, known as (S)-HPMPA (Formula 4*'*) was disclosed by DeClercq, et al., in Nature, 1986, 323, pp. 464-467 and earlier by Holy, et al., Nucleic Acids Research, Symposium Series No. 14, 1984, pp. 277-278. Phosphonate compounds which are HPMPA analogs are described in South African Patent 1987/8607. In applicant's hands, (S)-HPMPA is only slightly active in mice inoculated with Herpes simplex virus - 2. In a 21 day protocol 30% of a group of animals treated i.p. with 50 mg/kg/day of (S)-HPMPA survived.

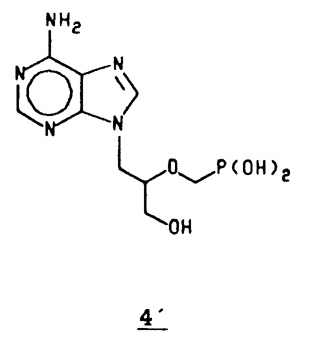

There is no teaching contained in those references, or a suggested combination thereof, which would make obvious the compounds, compositions, and uses involved in the present invention.

### SUMMARY OF THE INVENTION

Phosphonomethoxymethoxymethyl purine and pyrimidine derivatives, 5'-phosphonomethoxytetrahydro-fur-2'-yl-9-purine and 1-pyrimidine derivatives, phosphonomethoxymethoxymethyl 9-purine and 1-pyrimidine derivatives having a cyclic phosphonate group and 5'-phosphonomethoxytetrahydro-fur-2'-yl-9-purine and 1-pyrimidine derivatives having a cyclic phosphonate group have been synthesized and found to possess useful antiviral and antitumor activity.

The present invention concerns a phosphonomethoxymethoxymethyl purine/pyrimidine derivative of the formula 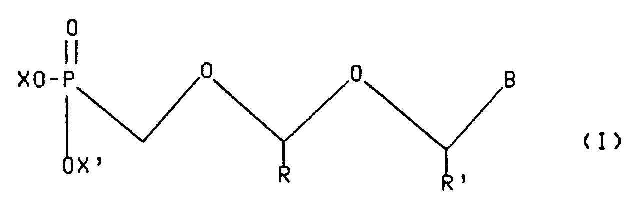 wherein
X and X*'* independently are hydrogen or alkyl having 1 to 6 carbon atoms, or the cation of a salt-forming base
R and R*'* independently are hydrogen, alkyl having 1 to 6 carbon atoms, hydroxyalkyl with 1 to 6 carbon atoms, alkanoyl having 2 to 7 carbon atoms, and B is a (9-substituted) purine or (1-substituted) pyrimidine base selected from the group consisting of xanthine, substituted xanthine, for example, hypoxanthine, guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine, and pharmaceutically acceptable salts thereof.

The present invention also concerns a 5*'*-phosphonomethoxytetrahydro(or dihydro)fur-2*'*-yl-purine or pyrimidine derivative of the formula 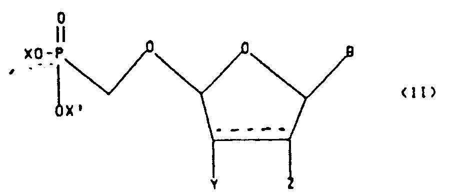 wherein
X and X*'* independently are hydrogen or alkyl having 1 to 6 carbon atoms, or the cation of a salt-forming base the broken line represents an optional bond,
Y and Z independently are unsubstituted or substituted alkyl with 1 to 6 carbon atoms or together they constitute an oxygen atom or methylene group in which event the broken line is absent, and
B is a 9-substituted purine or a 1-substituted pyrimidine base selected from the group consisting of xanthine, substituted xanthine, for example, hypoxanthanine, guanine, substituted guanine, for example, 8-bromoguanine, 8-chloroguanine, 8-methylguanine and 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine, and pharmaceutically acceptable salts thereof.

The invention also concerns to a 5*'*-phosphonomethoxytetrahydrofur-2*'*-yl-purine or pyrimidine derivative having a cyclic phosphonate group of the formula 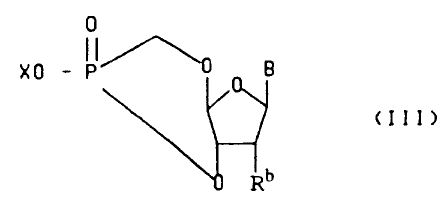 wherein
X is hydrogen or alkyl with 1 to 6 carbon atoms,
R^{b} is H or OH
and B is a (9-substituted) purine or a (1-substituted) pyrimidine base selected from the group consisting of xanthine, substituted xanthine, for example, hypoxanthine, guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine,
and pharmaceutically acceptable salts thereof.

The invention is further directed to a phosphonomethoxymethoxymethyl-9-substituted purine or 1-substituted pyrimidine derivatives having a cyclic phosphonate group of the formula 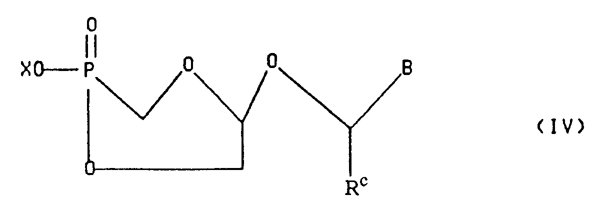 wherein
X is hydrogen, alkyl with 1 to 6 carbon atoms,
R^{c} is hydrogen, alkyl with 1 to 6 carbon atoms or alkanoyl having 2 to 7 carbon atoms, and B is a purine or pyrimidine base selected from the group consisting of xanthine, substituted xanthine, for example, hypoxanthine, guanine, substituted guanine, for example, 8-bromoguanine, 8-chlorogranine, 8-aminoguanine, 8-hydrazinoguanine, 8-hydroxyguanine, 8-methylguanine, 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine, are pharmaceutically acceptable salts thereof.

The present invention also concerns the following intermediates: 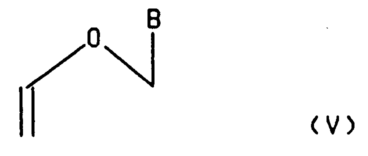 , wherein
B is a 9-substituted purine or a 1-substituted pyrimidine base selected from the group consisting of xanthine, substituted xanthine, for example, hypoxanthine, guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine; 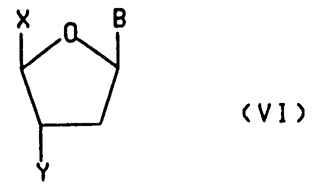 , wherein
X is a halogen, for example, chlorine, bromine, iodine or fluorine,
Y is phenylthio, phenylseleno or a halogen atom, for example, chlorine, bromine, iodine or fluorine, and B is a 9-substituted purine or 1-substituted pyrimidine base selected from the group consisting of xanthine, substituted xanthine, for example, hypoxanthine, guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, for example 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine; 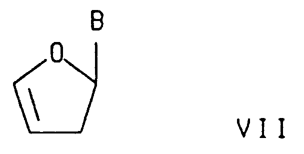 . wherein
B is guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, cystosine or substituted cytosine, for example, 5-ethylcytosine and 5-methylcytosine, 5-substituted uracil, for example, 5-chlorouracil, 5-bromouracil, 5-ethyluracil, 5-iodouracil, 5-propyluracil and 5-vinyluracil, except 5-fluorouracil and substituted adenine, for example, 3-deazaadenine, and 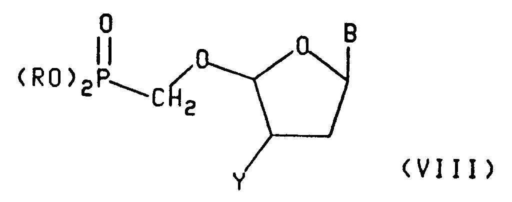 wherein
Y is a halogen, for example chlorine, fluorine, bromine or iodine, phenylthio, or phenylselano, R is hydrogen or alkyl with 1 to 6 carbon atoms and B is a purine or pyrimidine base selected from the group consisting of xanthine, substituted xanthine, for example, hypoxanthine, guanine, substituted guanine, for example, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, for example, 2-aminopurine, 2,6-diaminopurine, cytosine, substituted cytosine, for example, 5-ethylcytosine and 5-methylcytosine, thymine, uracil, 5-substituted uracil, for example, 5-chlorouracil, 5-bromouracil, 5-ethyluracil, 5-iodouracil, 5-propyluracil and 5-vinyluracil, adenine and substituted adenine, for example, 3-deazaadenine.

The present invention further relates to the following processes: 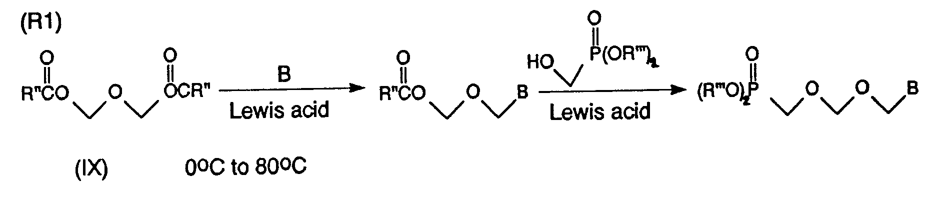 wherein
R'' is an alkyl having 1 to 6 carbon atoms or an unsubstituted aryl or an aryl substituted by a substituent such as a halogen, e.g., bromine or chlorine, nitro, alkyl having 1 to 6 carbon atoms or an alkoxy having 1 to 6 carbon atoms, B is a silylated purine or pyrimidine base, and R''' is hydrogen or alkyl with 1 to 6 carbon atoms; preferably the molar ratio of compound (IX) to B is 1:1; the corresponding substituted derivatives (R and R' in formula I other than hydrogen) are obtained in an analogous manner. 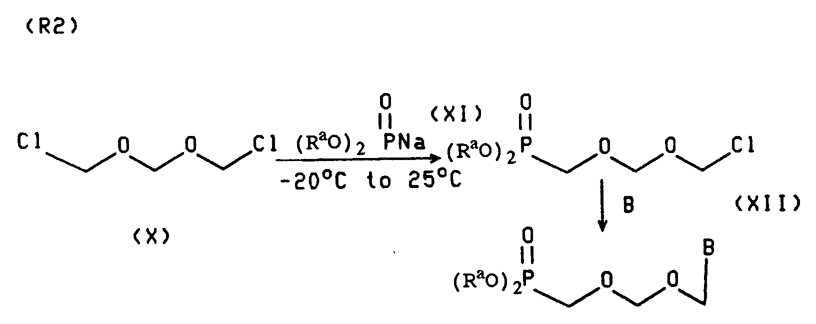 wherein
R^{a} is an alkyl having 1 to 6 carbon atoms and B is a purine or pyrimidine base; preferably compound (X) is 5 to 10 times in excess of compounds (XI) and the preferred molar ratio of compound (XII) to B is 1:1, the corressponding substituted derivatives (R and R' in formula I other than hydrogen) are obtained in an analogous manner. 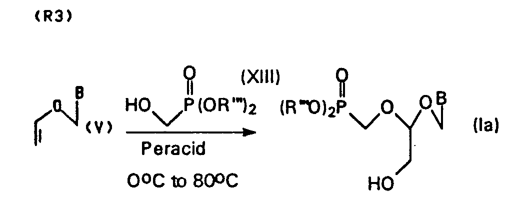 wherein
compound (XIII) is preferably in a 5 to 10 times excess of compound (V); 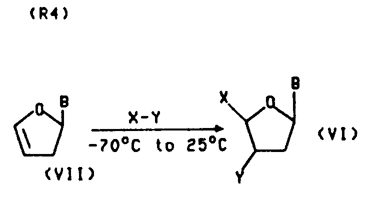 wherein
X-Y is a halogen,e.g., Br₂, Cl₂, phenyl-Se-Z, phenyl-S-Z (wherein Z= halogen) and B is a purine or pyrimidine base; preferably the molar ratio of compound (VII) to X-Y is 1:1; 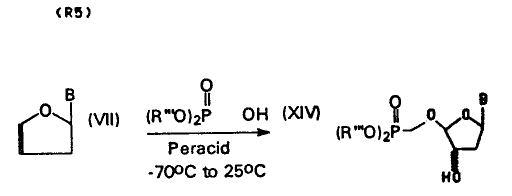 wherein
R''' is hydrogen or alkyl with 1 to 6 carbon atoms; preferably compound (XIV) is 5 to 10 times in excess of compound (V).

### DETAILED DESCRIPTION OF THE INVENTION

The compounds of the present invention can exist as optical isomers and both racemic and diasteromeric mixtures of these isomers which may exist for certain compounds, as well as the individual optical isomers are all within the scope of the present invention. While the racemic mixtures can be separated into their individual isomers through well-known techniques such as, for example, the separation of diastereomeric salts formed with optically active adjuncts, e.g., acids or bases followed by conversion back to the optically active substrates; in most instances, for the compounds of the present invention, the preferred optical isomer can be synthesized by means of stereospecific reactions, beginning with the appropriate stereoisomer of the desired starting material.

As indicated above, the present invention also pertains to pharmaceutically acceptable non-toxic salts of these compounds, containing, for example, Na⁺, Li⁺, K⁺, Ca⁺⁺ and Mg⁺⁺. Such salts may include those derived by combination of appropriate cations such as alkali and alkaline earth metal ions or ammonium and quaternary amino ions with the acid anion moiety of the phosphonic acid group. Metal salts can be prepared by reacting the metal hydroxide with a compound of this invention. Examples of metal salts which can be prepared in this way are salts containing Li⁺, Na⁺, and K⁺. A less soluble metal salt can be precipatated from the solution of a more soluble salt by addition of the suitable metal compound. In addition, salts may be formed from acid addition of certain organic and inorganic acids, e.g., HCl, HBr, H₂SO₄ or organic sulfonic acids, with basic centers of the purine, specifically guanine, or pyrimidine base. Finally, it is to be understood that compounds of the present invention in their un-ionized, as well as zwitterionic form, and/or in the form, of solvates are also considered part of the present invention.

Compounds of the present invention also exist in subclasses, with two broad subclasses being those wherein B is either a purine or a pyrimidine base. Of these broad subclasses there are preferred classes wherein the purine base is a guanine or a substituted guanine moiety and where the pyrimidine bases are either thymine or cytosine. The most preferred class of compounds are those wherein B is guanine or substituted guanine.

Compounds of the present invention may also be subclassed according to the structure of the phosphonate moiety. These classes are comprised of the diester, the monoester, and the diacid. Preferred subclasses of the phosphonate moiety are the monoester and the diacid.

The compounds of this invention, including the physiologically acceptable salts thereof, have desirable antiviral and antitumor activity. They exhibit activity against viruses, for example, Herpes Simplex virus I, Herpes Simplex virus II, cytomegalovirus, Varicella Zoster virus, influenza virus, vaccinia, polio, rubella, small pox, cowpox, Epstein-Barr virus, measles virus, human respiratory virus, papillomavirus and sinbis virus, just to mention a few and also against retroviruses, for example, human immunodeficiency virus (HIV). The inventive compounds also have an antitumor effect. They are active against murine leukemia P388 and other experimental tumors.

As mentioned above, the compounds of the present invention are useful active ingredients in human and veterinary medicine for the treatment and prophylaxis of diseases caused by retroviruses. Examples of fields of indication in human medicine regarding retroviruses are as follows:
(1) the treatment or prophylaxis of human retrovirus infections;
(2) the treatment or prophylaxis of diseases caused by HIV (virus of human immune deficiency; previously called HTLV III/LAV or AIDS) and the stages associated therewith such as ARC (AIDS related complex) and LAS (lymph adenopathy syndrome) and the immune weakness and encephalopathy caused by this retrovirus;
(3) the treatment or prophylaxis of HTLV I infection or HTLV II infection;
(4) the treatment or prophylaxis of the AIDS carrier state (AIDS transmitter state); and
(5) the treatment or prophylaxis of diseases caused by hepatitis B virus.

Examples of indications in veterinary medicine are as follows:
(1) Maedivisna (in sheep and goats),
(2) progressive pneumonia virus (PPV) (in sheep and goats),
(3) caprine arthritis encephalitis virus (in sheep and goats),
(4) Zwoegerziekte virus (in sheep),
(5) infectious virus of anemia (of the horse), and
(6) infections caused by cat leukemia virus.

For use against viral infections and against tumors, the compounds of this invention can be formulated into pharmaceutical preparations. Such preparations are composed of one or more of the inventive compounds in association with a pharmaceutically acceptable carrier. The reference Remington's Pharmaceutical Sciences, 17th Edition by A.R. Gennaro (Mack Publishing Company, 1985) discloses typical carriers and methods of preparation.

For antiviral purposes, the compounds may be administered topically or systemically to warn blooded animals, e.g., humans. For antitumor use, systemic, and preferably, parenteral administration is employed. By systemic administration is intended, oral, rectal, and parenteral (i.e., intramuscular, intravenous, subcutaneous and nasal) routes. Generally, it will be found that when a compound of the present invention is administered orally, a larger quantity of the reactive agent is required to produce the same effect as the smaller quantity given parenterally. In accordance with good clinical practice, it is preferred to administer the instant compounds at a concentration level that will produce effective antiviral or antitumor effect without causing any harmful or untoward side effects.

Therapeutically and prophylactically the instant compounds are given as pharmaceutical compositions comprised of an effective antiviral or antitumor amount of a compound according to the invention or a pharmaceutically acceptable salt thereof and a pharmaceutically acceptable carrier, as stated hereinabove. Pharmaceutical compositions for effecting such treatment will contain a major or minor amount, e.g., from 95 to 0.5% of at least one compound of the present invention in combination with a pharmaceutical carrier, the carrier comprising one or more solid, semi-solid, or liquid diluents, fillers and formulation adjuvants which are non-toxic, inert and pharmaceutically acceptable. Such pharmaceutical compositions are preferable in dosage unit form; i.e., physically discrete units containing a predetermined amount of the drug corresponding to a fraction or multiple of the dose which is calculated to produce the desired therapeutic response. Other therapeutic agents can also be present. Pharmaceutical compositions providing from about 1 to 50 mg of the active ingredient per unit dose are preferred and are conventionally prepared as tablets, lozenges, capsules, powders, aqueous or oily suspensions, syrups, elixirs, and aqueous solutions. Preferred oral compositions are in the form of tablets or capsules and may contain conventional excipients such as binding agents, (e.g., syrup, acacia, gelatin, sorbitol, tragacanth or polyvinylpyrrolidone), fillers (e.g., lactose, sugar, corn starch, calcium phosphate, sorbitol, or glycine), lubricants (e.g., magnesium stearate, talc, polyethylene glycol or silica), disintegrants (e.g., starch) and wetting agents (e.g., sodium lauryl sulfate). Solutions or suspensions of an inventive compound with conventional pharmaceutical vehicles are employed for parenteral compositions, such as an aqueous solution for intravenous injection or an oily suspension for intramuscular injection. Such compositions having the desired clarity, stability and adaptability for parenteral use are obtained by dissolving from 0.1% to 10% by weight of an active inventive compound in water or a vehicle comprising a polyhydric aliphatic alcohol such as glycerine, propylene glycol, and polyethylene glycol or mixtures thereof. The polyethylene glycols comprise a mixture of non-volatile, usually liquid, polyethylene glycols which are soluble in both water and organic liquids and have molecular weights from about 200 to 1500.

Considering the biological activities possessed by the compounds of the instant invention, it can be seen that these compounds have antitumor and antiviral properties, particularly suited to their use in combating viral infections or tumors. Thus, another aspect of the instant invention concerns a process for treating viral (including retroviral) infections or tumors in a mammal in need of such treatment which comprises systemic or topical administration to such mammal of an effective dose of an inventive compound or a pharmaceutically acceptable salt thereof. On the basis of testing, an effective dose could be expected to be from about 0.01 to about 30 mg/kg body weight with about 1 to about 20 mg/kg body weight a preferred dosage range. It is envisioned that for clinical antiviral application compounds of the instant invention will be administered in the same manner as for the reference drug acyclovir. For clinical applications, however, the dosage and dosage regimen must in each case be carefully adjusted, utilizing sound professional judgment and consideration of the age, weight and condition of the recipient, the route of administration and the nature and gravity of the illness. Generally a daily oral dose will comprise from about 150 to about 750 mg, preferable 250-500 mg of an inventive compound administered from one to three times a day. In some instances, a sufficient therapeutic effect can be obtained at lower doses, while in others, larger doses will be required.

In the reaction (process) (R1) described above, non-limiting examples of Lewis acids include BF₃ether, TiCl₄ and BCl₃.

In the reactions (processes) (R3) and (R5) described above, non-limiting examples of peracids include the following: m-chloroperbenzoic acid, trifluoroperacetic acid and perbenzoic acid.

The reactions (processes) (R1) to (R5) described above are preferably conducted at atmospheric pressure and preferably conducted in the presence of a solvent, e.g., CH₃CN, CH₂Cl₂, ClCH₂CH₂Cl, CHCl₃, THF, dioxane, diethylether, benzene or toluene.

### Description of the Specific Embodiments

The compounds which constitute this invention and their methods of preparation will appear more fully from a consideration of the following examples which are given for the purpose of illustration only and are not to be construed as limiting the invention in sphere or scope. In addition to the compounds described in the following examples, further compounds encompassed by the present invention are as follows:
9-[(2-Hydroxy-1-phosphonomethoxyethoxy)methyl]adenine disodium salt 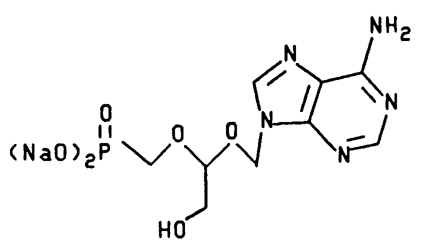
9-[(2-Hydroxy-1-phosphonomethoxyethoxy)methyl]cytosine disodium salt 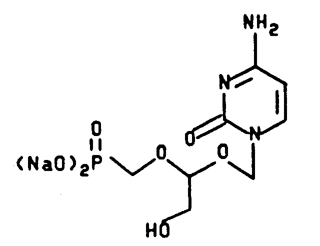
9-[(2-Hydroxy-1-phosphonomethoxyethoxymethyl]thymine disodium salt

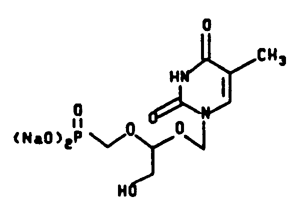

In the following examples, all temperatures are understood to be in degrees C when not specified. The nuclear magnetic resonance (NMR) spectral characteristics refer to chemical shifts (δ) expressed in parts per milion (ppm) versus tetramethylsilane (TMS) as a reference standard. The relative area reported for the various shifts in the proton NMR spectral data corresponds to the number of hydrogen atoms of a particular functional type in the molecule. The nature of the shifts as to multiplicity is reported as broad singlet (bs), singlet (s), multiplet (m), doublet (d), doublet of doublets (dd), triplet (t) or quartet (q). Abbreviations employed are:
- ACV: (acyclovir)
- BID: (twice a day)
- CDCl₃: (deuterochloroform)
- DMf: (dimethylformamide
- DMSO-d₆: (perdeuterodimethylsulfoxide)
- EMEM: (Earle's Minimum Essential medium)
- Et: (ethyl)
- HIV: (human immune deficiency
- HSV: (Herpes simplex virus)
- MuLV: (murine leukemia virus)
- NOE: (Nuclear Overhouser Effect)
- PFU: (plague forming units)
- TMS: (trimethylsilyl)
- Me: (methyl)
- Ac: (acetyl)
- Pv: (pivaloyl)
- Ph: (phenyl)
All compounds gave satisfactory elemental analyses.

The invention will now be described with reference to the following non-limiting examples.

### Examples:

### Example 1: Bisbenzoyloxymethyl ether (1)

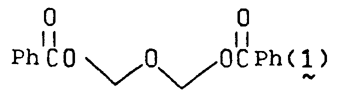

To a suspension of sodium benzoate (5.0 g, 34.7 mmol) in DMF (70 ml) was added bischloromethyl ether (20 g, 17.3 mmol) and the mixture was heated at 70°C for 16 hours. The insoluble material was removed by filtration. The filtrate was concentrated in vacuo to give a white crystal which was recrystallized from ether-pentane: yield 4.5 g (91%); mp 39°C.
- Analysis:: Calc. for C₁₆H₁₄O₅: C, 67.12; H, 4.92.
- Found:: C, 66.87; H, 4.94.

¹H-NMR (200 MHz, CDCl3): δ 5.66 (s, 4H), 7.75-8.05 (m,10H).

### Example 2: 1-[(Benzoyloxymethyoxy)methyl]thymine (2)

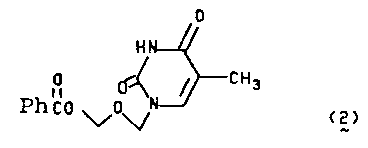

(1) A suspension of thymine (12.6 g, 0.1 mole), ammonium sulfate (300 mg) and trimethylsilyl chloride (2.5 ml) in hexamethyldisilazane (150 mL) was heated at 140°C for 16 hours under nitrogen. The volatiles were removed in vacuo at 50°C and the residual oil was dissolved in xylene (30 ml) and concentrated to dryness.
(2) To a solution of the silylated thymine in CH₂Cl₂ (200 ml) was added bisbenzoyloxymethyl ether (30 g, 0.1 mol) and trimethylsilyl trifluoromethanesulfonate (50 ml). The solution was stirred for 8 hours at 25°C under nitrogen. The reaction was diluted with ethyl acetate (400 ml) and washed with aqueous sodium carbonate, brine, dried (MgSO₄), filtered and concentrated in vacuo. The crude oily material was purified by silica gel column chromatography using CH₂Cl₂-5% MeOH as an eluent to give the title compound as white crystals: yield 14.5 g (50%); mp 141-143°C.

- Analysis:: Calc. for C₁₄H₁₄N₂O₅: C, 57.93; H, 4.82; N, 9.65.
- Found:: C, 57.59; H, 4.90; N, 9.52.

¹³C-NMR (50.3 MHz, d₆DMSO): δ 70.779, 72.477, 72.915, 73.349, 82.985, 105.563, 123.376, 124.076, 124.368, 128.383, 134.397, 146.260, 159.451, 160.211.
1H-NMR (CDCl₃): δ 1.82 (s,3H), 5.38 (s, 2H), 5.62 (s, 2H), 7.10 (s,1H), 7.4-8.0 (m,5H).

### Example 3:

### 1-[(Diethylphosphonomethoxy)methoxymethyl]thymine (3)

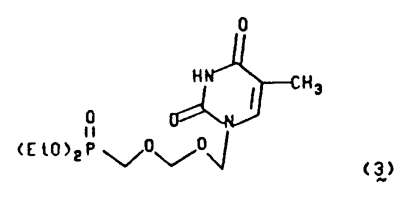

To a solution of 1-[3(benzoyloxy)methoxymethyl]thymine (2.9 g, 10 mmol) and diethylphosphonomethanol (1.85 g, 11 mmol) in benzene (180 ml) was added trimethylsilyl trifluoromethanesulfonate (0.05 ml) via a syringe under nitrogen. The solution was heated at 85°C for 20 minutes. After cooling to room temperature, ethyl acetate (50 ml) was added and washed with aqueous bicarbonate, brine dried (MgSO₄), filtered and concentrated in vacuo. The resultant yellow oil was purified by silica gel column chromatography using CH₂Cl₂-5% MeOH as an eluent to give the title compound as a white oil: yield 980 mg (30%).
H¹-NMR (200 MHz, CDCl₃): δ 1.39 (t, J = 6.6 Hz, 6H), 1.98 (s, 3H), 3.85 (d, J = 9.9 Hz, 2H), 4.1-4.3 (m, 4H), 4.82 (s, 2H), 5.20 (s, 2H), 7.20 (s, 1H), 9.0 (broad s, 1H).

### Example 4:

### 1-[3-(Ethylphosphonomethoxy)methyloxymethyl)]thymine sodium salt (4)

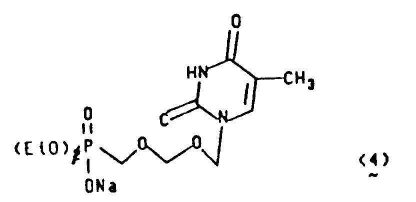

A solution of 1-[(diethylphosphonomethoxy)methoxymethyl]thymine (400 mg, 1.2 mmol) in 1N NaOH (8 ml) was stirred for 3 hours at 25°C. The solution was concentrated in vacuo and the resultant solid was purified by C-18 reverse phase column chromatography using water as an eluent under 0.56 bar (8 psi) pressure. The fractions having ultraviolet absorption were checked with HPLC, combined and lyophilized to give the title compound as a white amorphous powder: yield 220 mg (55%).
Analysis: Calc. for C₁₀H₁₆N₂O₇PNa H₂O: C, 34.48; H, 5.17; N, 8.05. Found: C, 34.92; H, 5.37; N, 8.35.
UV (H₂O): λ max 226 nm (ε =6966).
¹³C-NMR (50.3 MHz, D₂O): δ 11.451, 15.843, 61.067, 61.826, 63.585, 75.247, 94.631, 111.049, 141.277, 155.20, 170.907.
¹H-NMR (200 MHz, D₂O): δ 1.19 (t, J = 6.8 Hz, 3H), 1.81 (s, 3H), 3.62 (d, J = 8.9 Hz, 2H), 3.8-4.1 (m, 2H), 3.62 (s, 2H), 4.78 (s, 2H), 5.20 (s, 2H), 7.45 (s, 1H).

### Example 5: 1-[(Phosphonomethoxy)methoxymethyl]thymine disodium salt (5)

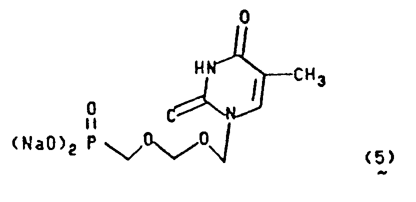

To a solution of 1-[3'-(ethylphosphonomethoxy) methoxymethyl]thymine sodium salt (300 mg, 0.9 mmol) in dry DMF (5 ml) was added bromotrimethylsilane (1.5 ml) under nitrogen. After stirring 3 hours at 25°C, volatiles were removed in vacuo and the residue was dissolved in aqueous saturated bicarbonate and re-evaporated in vacuo to a solid foam. Purification of this material by a C-18 reverse phase column chromatography using water as eluent under 0.56 bar (8 psi) pressure and lyophilization of combined fractions gave the title compound as a white amorphous foam: Yield 140 mg (48%).
Analysis: Calcd. for C₈N₁₁N₂O₇PNa₂: C, 29.64; H, 3.42; N, 8.64. Found: C, 29.91; H, 3.61; N, 9.16.
UV (H₂O): λ max 266 nm (ε = 8100).
¹³H-NMR (200 MHz, D₂O): δ 1.75 (s, 3H), 3.27 (d, J = 8.5 Hz, 2H), 4.71 (s, 2H), 5.11 (s, 2H), 7.74 (s, 1H).

### Example 6:

### 2-Amino-6-chloro-9-[(diethylphosphonomethoxy)methoxymethyl]purine (6)

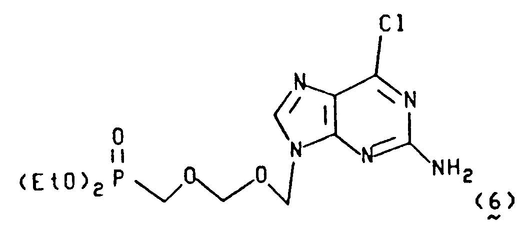

To a suspension of 60% sodium hydride in mineral oil (1.4 g, 34.5 mmol) in n-pentane (100 ml) at 0°C was added dropwise diethyl phosphate (4.4 mL, 34.5 mmol) under nitrogen. After stirring for 1 hour at 0°C, a solution of bis(chloromethoxy)methane (25 g, 172 mmol) (prepared according to the literature procedure: P.R. Strapp, J. Org. Chem., 34, 1143 (1969) in n-pentane (50 ml) was added at -70°C. The mixture was stirred for 90 minutes at 0°C, and then the solvent was evaporated under reduced pressure. The residual oil was dissolved in xylene and volatiles were removed in vacuo to give crude chloromethoxy-(diethoxyphosphonomethoxy)methane. Without further purification, this material was used for the next reaction.

To a suspension of 60% sodium hydride in mineral oil (1.4 g, 34.5 mmol) in DMF (100 ml) was added 2-amino-6-chloropurine (5.78 g, 34.2 mmol) and the mixture was stirred for 1 hour at 25°C. To the resulting yellow solution was added dropwise a solution of above chloromethoxy(diethylphosphonomethoxy)methane in DMF (20 ml) under nitrogen. After stirring 15 hours at 25°C, volatiles were removed in vacuo. The residual oil was suspended in ethyl acetate (100 ml), washed with water (30 ml), brine and dried (MgSO₄). The solvent was removed under reduced pressure, and the residual oil was chromatographed on silica gel using CH₂Cl₂-3% MeOH as an eluent to give the title compound as a colorless oil: yield 3.0 g (23%).
¹H-NMR (300 MHz, CDCl₃): δ 1.395 (t, J = 6.9 Hz, 6H), 3.850 (d, J = 9.0 Hz, 2H), 4.05-4.20 (m, 4H), 4.697 (s, 2H), 5.323 (broad s, 2H) 5.578 (s, 2H, 7.889 (s, 1H).

### Example 7:

### 9-[(Methylphosphonomethoxy)methoxymethyl]guanine sodium salt (7)

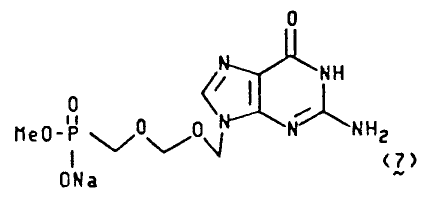

To a solution of 2-amino-6-chloro-9-[3-(diethylphosphonomethoxy)methoxy-methyl] purine (325 mg, 0.84 mmol) in methanol (5 ml) was added 1N sodium methoxide in methanol (10 ml). The solution was heated at 80°C for 1 hour under nitrogen. Volatiles were removed under reduced pressure. The residual oil was then dissolved in water (10 ml) and the solution was heated at 100°C for 1 hour. The pH of the solution was carefully adjusted to 8.0 at 0°C by dropwise addition of 1N-HCl. Water was then evaporated in vacuo and the residual oil was purified by a C₁₈ reverse phase column using water as an eluent to give the title compound as a white solid: yield 185 mg(60%).
Analysis: Calcd. for C₉H₁N₅O₆PNa 4H₂O: C, 26.13; H, 5.12; N, 16.95. Found: C, 26.05; H, 4.99; N, 16.64.
UV (H₂O): λ max 254 nm (ε = 14,372), 274 nm (ε = 9.788).
¹³C-NMR (75.47 MHz, D₂O): δ 51.875, 60.464. 63.627, 70.005, 94.711, 94.952, 116.171, 139.925, 151.665, 154.428, 159.278.
¹H-NMR (300 MHz, D₂O): 3.677 (d, J = 10.3Hz, 3H), 3.620 (d, J = 9.0 Hz, 2H), 4.817 (s, 2H), 5.539 (s, 2H), 7.882 (s, 2H).

### Example 8: 9-[3-(Phosphonomethoxy)methoxymethyl]guanine disodium salt (8)

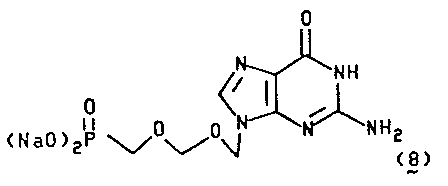

To a solution of 9-[(methylphosphonomethoxy)methoxymethyl]guanine (1.5 g, 4.4 mmol) in DMF (5 ml) was added bromotrimethylsilane ( 5 ml) under nitrogen. After stirring 3 hours at 25°C, the volatiles were removed in vacuo and the residue was neutralized to pH 8.0 by addition of aqueous saturated sodium bicarbonate. Water was then evaporated in vacuo, and the residue was purified by a C₁₈ reverse phase column using water as an eluent under 0.56 bar (8 psi) pressure to give the title compound as a white powder: yield 900 mg (59%).
Analysis: Calcd. for C₈H₁₀N₅O₆PNa 3H₂O: C, 23.84; H, 4.01; N, 17.37. Found: C,23.99; H, 3.92; H, 17.21
UV (H₂O): λ max 252 nm (ε = 12,113), 274 nm (ε = 8,201).
¹³C-NMR (75.47 MHz, D₂O): δ 67.016, 69.018, 70,746, 95.680, 95.831, 118.192, 141.812, 153.576, 157.386, 162.493. ¹H-NMR (300 MHz, D₂O): δ 3.525 (d, J = 8.9 Hz, 2H), 4.766 (s, 2H), 5.539 (s, 2H), 7.892 (s, 1H).

### Example 9:

### 9-[(Diethylphosphonomethoxy)methoxymethyl]adenine (9)

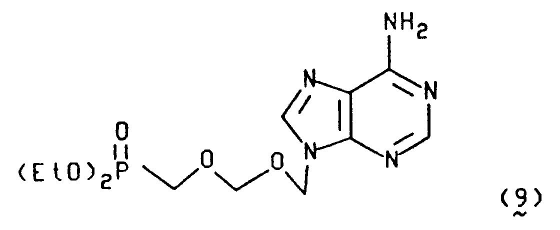

To a suspension of 60% sodium hydride in mineral oil (1.4 g, 34.5 mmol) in DMF (100 ml) was added adenine (4.7 g, 34.5 mmol) and the mixture was stirred at 80°C for 1 hour. To the resulting yellow solution was added dropwise a solution of chloromethoxy-(diethoxyphosphinolmethoxy)methane [(prepared from diethylphosphate (4.4 ml, 34.5 mmol) and bis-(chloromethoxy)methane (25 g, 172 mmol)] in DMF (20 ml) under nitrogen. After stirring at 25°C for 15 hours, volatiles were removed in vacuo, and the resulting oily residue was purified by silica gel column chromatography using CH₂Cl₂-10% MeOH as an eluent to obtain the title compound as a colorless oil: yield 6.0 g (50%).
¹H-NMR (300 MHz, CDCl₃): δ 1.390, (t, J = 6.7 Hz, 6H), 3.821 (d, J = 9.2 Hz, 2H), 4.05-4.18 (m, 4H), 4.785 (s, 2H), 5.690 (s, 2H), 6.20 (broad s, 2H), 7.921 (s, 1H), 8.295 (s, 1H).

### Example 10: 9-[3-(Phosphonomethoxy)methoxymethyl]adenine disodium salt (10)

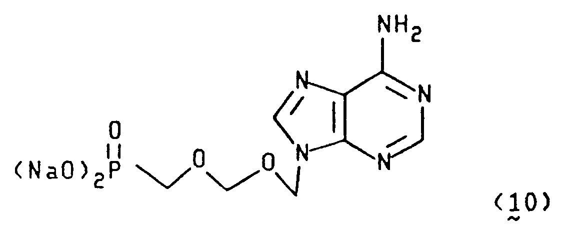

To a solution of 9-[(diethylphosphonomethoxy)methoxymethyl]adenine (600 mg, 1.7 mmol) in DMF (4 mL) was added bromotrimethylsilane (5 ml) under nitrogen. After stirring 3 hours at 25°C, the volatiles were removed in vacuo and the residue was neutralized to pH 8.0 by addition of aqueous saturated sodium bicarbonate. Water was then evaporated in vacuo, and the residue was purified by a C₁₈ reverse phase column using water as an eluent under 0.56 bar (8 psi) pressure to give the title compound as a white powder: yield 280 mg (50%).
Analysis: Calcd for C₈N₁₀N₅O₅PNa₂ (3H₂O + 0.2 mol NaCl): C, 22.08; H, 4.72; N, 16.10. Found: C, 22.15; H, 4.64; N, 16.26.
UV (H₂O): λ max 260 nm (ε = 12,016)
¹³C-NMR (75.47 MHz, D₂O): δ 66.913, 68.917, 71.033, 95.729, 95.940, 120.228, 144.611, 150.754, 154.766, 157.370.
¹H-NMR (300 MHz, D₂O): 3.486 (d, J = 8.9 Hz, 2H), 4.779 (s, 2H), 5.710 (s, 2H), 8.177 (s, 1H), 8.226 (s, 1H).

### Example 11:

### 1-[(Diethylphosphonomethoxy)methoxymethyl]cytosine (11)

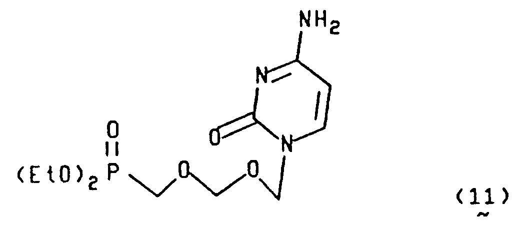

To a suspension of 60% sodium hydride in mineral oil (700 mg, 17 mmol) in DMF (50 ml) was added cytosine (1.9g, 17 mmol) and the mixture was heated at 80°C for 2 hours under nitrogen. To the resulting yellow solution was added dropwise a solution of chloromethoxy(diethylphosphonomethoxy)methane [prepared from diethylphosphate (2.4 g, 17 mmol) and bis(chloromethoxy)methane (12.5 g, 86 mmol)] in DMF (10 ml) under nitrogen. After stirring 15 hours at 25°C, the volatiles were removed in vacuo. The residue was dissolved in ethyl acetate (120 mL) and water (30 ml). The organic phase was washed with brine and dried (MgSO₄). After removal of the solvent in vacuo, the residual oil was chromatographed on silica gel using CH₂Cl₂-10% MeOH as an eluent to give the title compound as a white oil: yield 1.2 g (22%).
¹H-NMR (300 MHz, CDCl₃): δ 1.390 (t, J = 6.9 Hz, 6H), 1.90 (broad s, 2H), 3.815 (d, J = 9.0 Hz, 2H), 4.05-4.20 (m, 4H), 4.752 (s, 2H), 5.20 (s, 1H), 5.853 (d, J = 7.4 Hz, 1H), 7.312 (d, J = 7.4 Hz, 1H).

### Example 12: 1-[(Phosphonomethoxy)methoxymethyl]cytosine disodium salt (12)

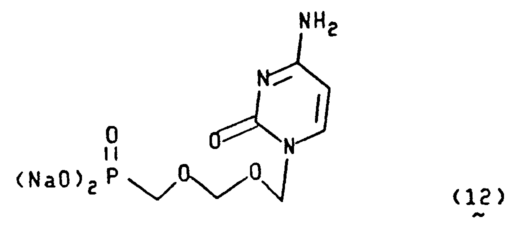

To a solution of 1-[diethylphosphonomethoxy)methoxymethyl]cytosine (1.2 g, 3.7 mmol) in DMF (5 ml) was added bromotrimethylsilane (5 ml) under nitrogen. After stirring 3 hours at 25°C, the volatiles were removed in vacuo and the residue was neutralized to pH of 8.0 by the addition of aqueous saturated sodium bicarbonate. Water was then evaporated in vacuo and the residue was purified by a C₁₈ reverse phase column using water as eluent under 0.56 bar (8 psi) pressure to give the title compound as a white solid: yield 460 mg (47%).
Analysis: Calcd. for C₇H₁₁N₃O₆PNa₂ (3 H₂O + 5% NaCl); C, 21.99; H, 4.22; N, 10.99. Found: C, 21.72; H, 4.65; N, 10.78.
UV (H₂O): λ max 268 nm (ε = 8,245)
¹³C-NMR (75.47 MHz, D₂O): δ 66.876, 68.873, 77.710, 96.141, 96.284, 98.178, 148.355, 160.409, 162.543. ¹H-NMR (300MHz, D₂O): δ 3.560 (d, J = 9.0 Hz, 2H), 4.849 (s, 2H), 5.313 (s, 2H), 6.03 (d, J= 7.3 Hz, 1H), 7.714 (d, J = 7.3 Hz, 1H).

### Example 13: [2-(Phenylselenyl)ethoxy]methyl chloride (13)

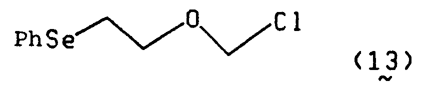

To a solution of 2-(phenylselenyl)ethanol (4.0 g, 20 mmol) [prepared according to the literature procedure: P. Rollin, V.V Bencomo, P. Sinay, Synthesis, 13 (1984] in CH₂Cl₂ 15 ml) was added paraformaldehyde (620 mg, 20 mmol). HCl gas was then bubbled into the solution at 5°C for 2 hours. The solution was dried (MgSO₄), and the solvent was removed under reduced pressure to give the title compound as a colorless oil in a quantitative yield.
¹H-NMR (300 MHZ, CDCl₃: δ 3.059 (t, J = 7.0 Hz, 2H), 3.882 (t, J = 7.0 Hz, 2H), 5.449 (s, 2H), 7.2-7.5 (m, 5H).

### Example 14:

### 2-Amino-6-chloro-9-[(2-(phenylselenyl)ethoxy)methyl]purine (14)

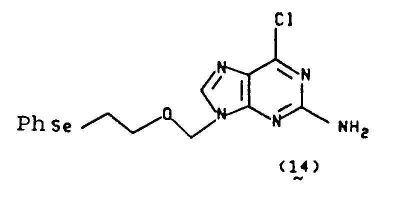

A mixture of 2-amino-6-chloropurine (20 g, 118 mmol) add ammonium sulfate (400 mg) in hexamethyldisilazane (400 ml) and chlorotrimethylsilane (6ml) was heated at 145°C for 5 hours under nitrogen. Volatiles were removed in vacuo and the residue was evaporated with xylene twice, and further dried in vacuo for 3 hours. The crude silylated 2-amino-6-chloro-purine (15 g, 72 mmol) and mercuric cyanide (15 g, 59 mmol) in benzene (900 ml) was heated at reflux for 30 minutes, then a solution of 2-(phenylselenyl)ethoxymethyl chloride (17 g, 68 mmol) in benzene (100 ml) was added. The mixture was refluxed for 3 hours, and then allowed to stir for 15 hours at 25°C. The reaction was diluted with CH₂Cl₂ (300 ml), and then quenched with aqueous saturated bicarbonate (2 l). The organic phase was washed with 2N potassium iodide (200 ml), dried (MgSO₄) and the solvents were removed in vacuo. The residual oil was chromatographed on silica gel using CH₂Cl₂-5% MeOH as an eluent to provide the title compound as a slightly yellow foam: yield 15.0 g (63%).
Analysis: Calcd. for C₁₄H₁₄N₅OClSe 1/2 H₂O C, 42.93; H, 3.86; N, 17.88. Found: C, 42.92: H, 3.80: N, 17.59
H¹-NMR (300 MHz, CDCl₃): δ 2.961 (t, J = 6.9 Hz, 2H), 3.704 (t, J - 6.9 Hz, 2H), 5.196 (broad s, 2H), 5.420 (s, 2H), 7.1-7.4 (m,5H), 7.806 (s, 1H).
¹³C-NMR (75.47 MHz, CDCl₃): δ 26.041, 69.144, 72.710, 127.206, 128.653, 128.846, 131.232, 136.062, 144.946, 151.190, 151.833, 152.298.

### Example 15: 2-Acetamino-6-chloro-9-[(2-(phenylselenyl)ethoxy)methyl]purine (15)

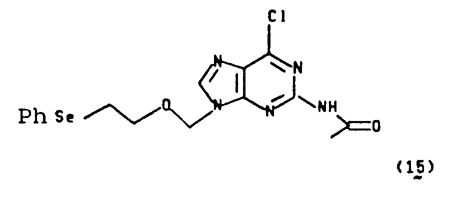

A solution of 2-amino-6-chloro-9-[(2-phenylselenyl)ethoxy)methyl]purine (8 g, 21 mmol) in acetic anhydride (80 ml) was heated at 55°C for 40 hours. Volatiles were removed in vacuo and the residual oil was purified by silica gel column chromatography using CH₂Cl₂-40% EtOAc as an eluent to give the title compound as a yellow powder: yield 5.8 g (65%).
Analysis: Calcd. for C₁₆H₁₆N₂O₂ClSe: C, 45.25: H, 3.80; N, 16.49. Found: C, 45.12; H, 3.90; N, 16.47.
¹H-NMR (300 MHz, CDCl₃): δ 2.49 (s, 3H), 2.961 (t, J = 6.9 Hz, 2H), 3.756 (t, J = 6.9 Hz, 2H), 5.541 (s, 2H), 7.2-7.4 (m, 5H), 8.063 (s, 1H).

### Example 16:

### 2-Acetamino-6-chloro-9-(vinyloxymethyl)purine (16)

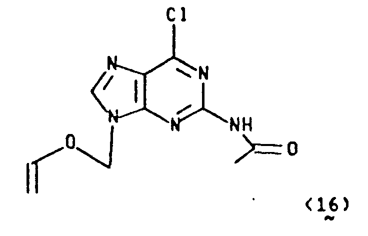

To a solution of 2-acetamino-6-chloro-9-[(2-(phenylselenyl)ethoxy)methyl]purine (424 mg, 1 mmol) in methanol (20 ml) was added sodium bicarbonate (92 mg, 1.1 mmol) and sodium periodate (320 mg, 1.5 mmol). After stirring at 25°C for 30 minutes the mixture was filtered and evaporated to dryness. The residue was dissolved in dioxane (20 ml) and the solution was heated at 80°C for 20 minutes under nitrogen. The solution was evaporated in vacuo and the residual oil was chromatographed on silica gel using CH₂Cl₂-20% MeOH as an eluent to give the title compound as a slightly yellow foam: yield 220 mg (60%).
Analysis: Calcd. for C₁₀H₁₀N₅O₂Cl: C, 44.88; H, 3.77; N, 26.17.
Found: C, 44.57; H, 3.83; N, 25.82.

### Example 17:

### 2-Acetamino-6-chloro-9-[(1-(dimethylphosphonomethoxy)ethoxy)methyl]purine (17)

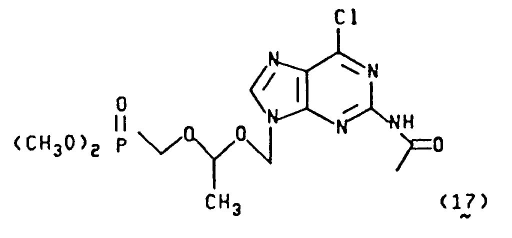

To a solution of 2-acetamino-6-chloro-9-(vinyloxy)methyl-purine (2.2 g, 6.0 mmol) and dimethylphosphonomethanol (1.67 g, 12.0 mmol) in chloroform (100 ml) was added 120 mg of methanesulfonic acid. After heating at 60°C for 2 hours, the solvent was removed in vacuo and the residual oil was chromatographed on silica gel using CH₂Cl₂-10% MeOH as an eluent to give the title compound as a colorless oil: yield 1.2 g (50%).
¹³C NMR (75.47 MHz, CDCl₃): 18.794, 25.054, 53.064, 53.218, 55.743, 59.056, 68.071, 99.254, 99.502, 127.921, 144.576, 151.598, 152.645, 170.299.
¹H-NMR (300 MHz, CDCl₃): δ 1.347 (d, J = 8.1 Hz, 3H), 2.519 (s, 3H), 3.852 (d, J = 16.2 Hz, 6H), 5.029 (q, J = 8.1 Hz, 1H), 5.648 (d, J = 15.6 Hz, 1H), 5.791 (d, J = 15.6 Hz, 1H), 7.275 (s, 1H), 8.201 (s, 1H).

### Example 18:

### 9-[(1-(Phosphonomethoxyethoxy)methyl]guanine disodium salt (18)

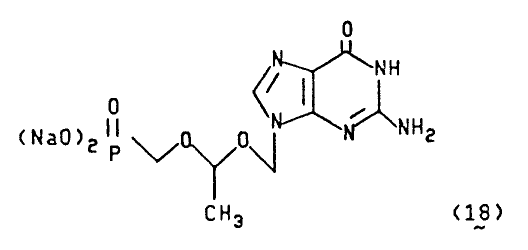

To a solution of 2-acetamino-6-chloro-9-[(1-dimethylphosphonomethoxy)ethoxy)methyl]purine (1.2 g, 2.95 mmol) in methanol (5 ml) was added 1N sodium methoxide in methanol (10 ml). After stirring at 25°C for 1 hour, water (10 ml) was added and the solution was heated at 90°C for 1 hour under nitrogen. Volatiles were removed in vacuo and the residual oil was purified by C₁₈ reverse phase column chromatography using water as an eluent under 0.56 bar (8 psi) pressure. Each 10 ml fraction was assayed by high pressure liquid chromatography. The combined fractions were lyophilized to give a white solid. This material was dissolved in DMF (20 ml) followed by bromotrimethylsilane (5 ml). After stirring 2 hours at 25°C, volatiles were removed in vacuo and the residue was purified by a C₁₈ reverse phase column using water as an eluent under 0.56 bar (8 psi) pressure to give the title compound as white amorphous powder after lyophilization: yield 245 mg (24%).
Analysis: Calcd. for C₉H₁₂N₅O₆PNa₂ 4H₂O: C, 25.78, H, 4.80; N, 16.70.
Found: C, 25.93; H, 4.44; N, 16.91.
UV (H₂O): λ max 252 nm (ε=9751).
¹³C-NMR (75.47 MHz, D₂O): δ 20.859, 64.079, 66.088, 70.423, 102.054, 102.241, 119.11, 140.287, 153.110, 162.211, 168.712.
¹H-NMR (300 MHz, D₂O): δ 1.195 (d, J = 6.3 Hz, 3H), 3.305 (dd, J = 8.9, 8.4 Hz, 1H), 3.496 (dd, J = 8.9, 8.4 Hz, 1H), 4.874, (q, J = 6.3 Hz, 1H), 5.475 (dd, J = 14.0, 11.1 Hz, 1H), 5.523 (dd, J = 14.0, 11.1 Hz, 1H), 7.790 (s, 1H).

### Example 19:

### 1-(5-Methoxytetrahydro-2-furyl)thymine (19)

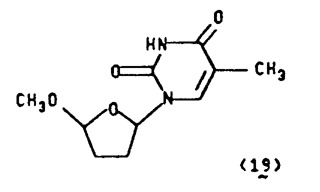

To a suspension of thymine (2.5 g, 20 mmol) in hexamethyldisilazane (30 ml) was added ammonium sulfate (50 mg) and chlorotrimethylsilane (0.5 ml) and the mixture was heated at 145°C for 4 hours under nitrogen. The excess hexamethyldisilazane was removed at reduced pressure, and the residual oil was dissolved in xylene and evaporated in vacuo to give a colorless viscous oil. To this silylated thymine in dichloroethane (40 ml) was added 2,5-dimethoxytetrahydrofuran (7 ml). After cooling the solution to -30°C, tin tetrachloride (2.3 ml) was added dropwise via a syringe under nitrogen. The mixture was allowed to warm to -10°C and was then poured into ice cold aqueous sodium bicarbonate (100 ml) and ethyl acetate (150 ml). The mixture was filtered and the organic phase was separated and dried (MgSO₄). The solvent was removed under reduced pressure and the residual oil was chromatographed on silica gel using CH₂Cl₂-5% MeOH as an eluent to give the title compound as a cis/trans mixture in a ratio of 1:1 as shown by analytical HPLC and ¹H-NMR: yield 3.4 g (75%).
Analysis: Calcd. for C₁₀H₁₄N₂O₄: C, 53.08; H, 6.24; N, 12.39.
Found: C, 52.81; H, 6.22; N, 12.38.
UV (EtOH): λmax 266 nm (ε = 9076).
¹H-NMR (300 MHz, CDCl₃): δ 1.4-2.1 (m, 7H), 3.40 and 3.425 (two s, 3H), 5.20 and 5.328 (two broad s, 1h), 6.208 and 6.417 (two dd, J = 3.5, 7.0 HZ and 7.2, 7.2 Hz, 1H), 7.021 and 7.40 (broad s, 1H).

The cis/trans (19A/19B) mixture was separated by a careful silica gel column chromatography. Thus, the cis isomer 19A was eluted first with CH₂Cl₂-3% MeOH and obtained as a white needle. The X-ray crystallography and the NOE (Nuclear Overhauser Effect) nmr confirmed the cis stereo-chemical arrangement of 19A. mp 153-154°C.
- Analysis:: Calcd. for C₁₀H₁₄N₂O₄: C, 53.09; H, 6.24; N, 12.38.
- Found:: C, 52.92; H, 6.20; N, 12.10.

¹³C-NMR (75.47 MHz, CDCl₃): δ 12.634, 29.417, 32.157, 55.202, 105.883, 111.411, 135.952, 139.553, 150.938, 163.906.
¹H-NMR (300 MHz, CDCl₃): δ 1.950 (s, 3H), 1.9-2.3 (m, 4H), 3.40 (s, 3H), 5.20 (t, J = 2.9 Hz, 1H), 6.417 (dd, J = 4.0, 7.2 Hz, 1H), 7.40 (s, 1H), 8.781 (s, 1H).

Continuing the column with CH₂Cl₂-3% MeOH, the trans isomer 19B was eluted after the cis isomer 19A and was obtained as white needles. The NOE observation of 19A was consistent with the assigned structure: mp 124-125°C.
- Analysis:: Calcd. for C₁₀H₁₄N₂O₄: C, 53.09; H, 6.24; N, 12.38.
- Found:: C 53.10; H, 6.10; N, 12.00.

¹H-NMR (75.47 MHz, CDCl₃): δ 1.920 (s, 3H), 2.0-2.5 (m, 4H), 3.425 (s, 3H), 5.328 (dd, J = 2.5, 6.0 Hz, 1H), 6.208 (dd, J = 3.5, 7.0 Hz, 1H), 7.021 (s, 1H), 8.885 (s, 1H).

### Example 20:

### 1-(5-Dimethylphosphonomethoxytetrahydro-2-furyl)thymine (20)

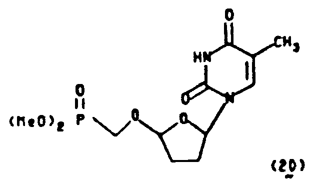

To a solution of 1-(5-methoxytetrahydro-2-furyl)thymine (5.2 g, 23 mmol) and dimethylphosphonomethanol (6.5 g, 44 mmol) in toluene was added acetic acid (5 ml) and p-toluenesulfonic acid monohydrate (500 mg, 2.6 mmol). The solution was heated at 100°C for 2 hours and the resulting insoluble solid was removed by suction filtration. After removal of the solvent under reduced pressure, the residual oil was chromotographed on silica gel using CH₂Cl₂-5% MeOH as an eluent to give the title compound as a cis/trans mixture (6:4): yield 5.0 g (60%).
¹H-NMR (300 MHz, CDCl₃): δ 1.9-2.2 (m, 10H), 3.8-4.1 (m, 6H), 5.198, 5.445 (broad s, 0.6 and 0.4H), 6.250 (dd, J = 2.8, 7.5 Hz, 0.4H), 6.437 (t, J = 7.4 Hz, 0.6H), 7.052 (s, 0.4H), 7.405 (s, 0.6H), 9.60 (broad s, 0.6H), 7.628 (broad s, 0.4H).

### Example 21:

### 1-(5-Methylphosphonomethoxytetrahydro-2-furyl)thymine sodium salt (21)

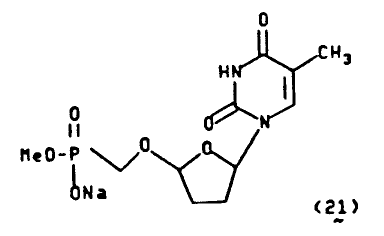

To a solution of 1-(5-dimethylphosphonomethoxytetrahydro-2-furyl)thymine (5 g, 14.6 mmol) in methanol (10 ml) was added 2N sodium hydroxide (20 ml). After stirring 2 hours at 25°C, the reaction was neutralized to pH 8.0 by addition of 3N-HCl with stirring. Water was then evaporated in vacuo and the residual oil was purified by a C₁₈ reverse phase column using water as an eluent to give the title compound as a white powder. This material was shown to be a 1:1 cis/trans (21A/21B) mixture by analytical HPLC and ¹H-NMR: yield 3.2 g (65%).
Analysis: Calcd. for C₁₁H₁₆N₂O₇NaP 2H₂O: C, 34.92; H, 5.29; N, 7.40.
Found: C, 34.93; H, 4.99; N, 7.43.
UV (H₂O): λ max 268 nm (ε = 8668).
¹H-NMR (300 MHz, D₂O): δ 1.842 (s, 1.5H), 1.894 (s, 1.5H), 1.9-2.5 (m,4H), 3.571 (d, J = 9.8 Hz, 1H), 3.589 (d, J = 9.2 Hz, 1H), 3.59-3.85 (m, 2H), 5.239 (d, J = 3.5. Hz, 0.5H), 5.483 (d, J = 4.7 Hz, 0.5H), 6.198 (q, J = 2.9 Hz, 0.5H), 6.331 (t, J = 6.0 Hz, 0.5H), 7.371 (s, 0.5H), 7.561 (s, 0.5H).

The cis/trans mixture was separated by a C₁₈ reverse phase column (100 time weight) using water -3% acetonitrile as an eluent under 0.42 bar (6 psig) pressure. Each 15 ml fraction was assayed by HPLC. The cis isomer 21A was eluted first and obtained as a white powder.
¹H-NMR(300 MHz, D₂O): δ 1.894 (s, 3H), 2.0-2.45 (m,4H), 3.571 (d, J = 9.8 Hz, 2H), 3.595 (dd, J = 7.4, 10.0 Hz, 1H), 3.781 (dd, J = 7.4, 10.8 Hz, 1H), 5.239 (d, J = 3.5 Hz, 1H), 6.331 (t, J = 6.0 Hz, 1H), 7.561 (s, 1H).

After cis/trans mixture fractions, the pure trans isomer 21B was also obtianed as a white powder.
¹H-NMR (300 MHz, D₂O): δ 1.842 (s, 3H), 2.0-2.5 (m, 4H), 3.589 (d, J = 9.2 Hz, 2H), 3.611 (dd, J = 9.2, 10.0 Hz, 1H), 3.840 (dd, J = 9.2, 10.0 Hz, 1H), 5.483 (d, J = 4.3 Hz, 1H), 6.198 (q, J = 2.9 Hz, 1H), 7.371 (s, 1H).

The steroechemical assignment of the cis and trans isomers was confirmed by the NOE (Nuclear Overhauser Effect) nmr.

### Example 22:

### 1-(5-Phosphonomethoxytetrahydro-2-furyl)thymine disodium salt (22)

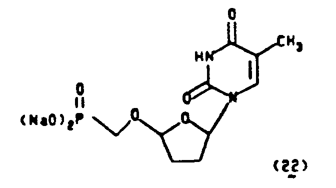

To a solution of 1-(5-methylphosphonotetrahydro-2-furyl)thymine sodium salt (1.2 g, 3.5 mmol) in DMF (15 ml) was added bromotrimethylsilane (10 ml) under nitrogen. After stirring 4 hours at 25°C, the volatiles were removed in vacuo and the residual oil was neutralized to pH 8.0 by the addition of aqueous sodium bicarbonate. Water was then evaporated in vacuo, and the residue was purified by a C₁₈ reverse phase column using water as an eluent under 0.56 bar (8 psi) pressure to give the title compound as a white powder: yield 857 mg (70%).
Analysis: Calcd. for C₁₀H₁₃N₂O₇PNa₂ 5H₂O: C, 27.26; H, 5.20; N, 6.36.
Found: C, 27.14; H, 5.26; N, 6.03.
UV (H₂O): λ max 268 nm (ε = 7.350).
¹H-NMR (300 MHz, D₂O): δ 1.864 (s, 1.5H), 1.928 (s, 1.5H), 1.95-2.90 (m, 4H), 3.4-3.6 (m, 2H), 5.363 (t, J = 3.0 Hz, 0.5H), 5.56 (d, J = 4.2 Hz, 0.5H), 6.212 (dd, J = 2.7, 5.8 Hz, 0.5H), 6.321 (t, J = 3.9 Hz, 0.5H), 7.435 (s, 0.5H), 7.679 (s, 0.5H).

### Example 23:

### 1-[2,3-Dideoxy-4-beta-chloro-3-(phenylselenyl)-beta-D-erythrofuranosyl]thymine (23)

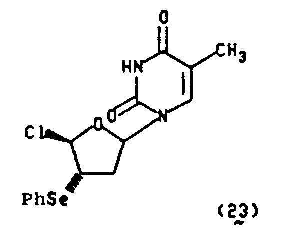

To a solution of 1-(2,3-dideoxy-3,4-didehydro-beta-D-ethyro-furanosyl)thymine (1.94 g, 10 mmol) prepared according to the literature procedure: J. Zemlicka, R. Gasser, J. V. Freisler, J. P. Horwitz, J. Amer. Chem. Soc., 94, 3213 (1972)] in CH₂Cl₂ (30 mL) was added at - 70°C dropwise a solution of phenylselenyl chloride (1.92 g, 10 mmol) in CH₂Cl₂ (5 ml) under nitrogen. After stirring at -70°C for 1 hour, the solvent was removed in vacuo to give the title compound as a yellowish oil. This material was used for the next reaction without further purification.
¹H-NMR (300 MHz, CDCl₃): δ 1.95 (s, 3H), 2.5-2.8 (m, 2H), 4.18 (d, J == 6.5 Hz, 1H), 6.20 (s, 1H), 6.55 (q, J - 6.0, 7.5 Hz, 1H), 7.1-7.7 (m, 6H), 9.30 (broad, 1H).

### Example 24:

### 1-[2,3-Dideoxy-4-beta-(dimethylphosphono)methoxy-3-(phenyselenyl)-beta-D-erythrofuranosyl]thymine (24)

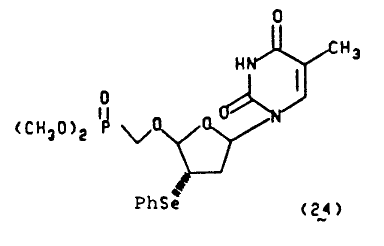

To a solution of 1-[2,3-dideoxy-4-chloro-3-(phenylselenyl)-beta-D-erythro-furanosyl]thymine (3.85 g, 10 mmol) and dimethoxyphosphinylmethanol (1.5 g, 11 mmol) in CH₂Cl₂ (20 ml) was added dropwise at -70°C a solution of silver perchlorate (2.3 g, 11 mmol) in CH₃CN (3 ml) over 3 minutes under nitrogen. The mixture was allowed to warm to 0°C and was then poured into aqueous saturated bicarbonate (10 ml)-brine (15 ml). The organic phase was separated after filtration and dried (MgSO₄). The solvents were removed under reduced pressure, and the residual oil was purified by silica gel column chromatography using CH₂Cl₂-5% MeOH as an eluent to give the title compound as a colorless oil: yield 1.3 g (31%).
¹H-NMR (300 MHz, CDCl₃): δ 1.93 (s, 3H), 2.45 (m, 2H), 3.70 (dd, J = 8.4, 8.0 Hz, 1H), 3.75 (d, J = 12.0 Hz, 6H), 3.85 (d, J = 6.9 Hz, 1H), 3.90 (dd, J = 8.4, 8.0 Hz, 1H), 5.10 (s, 1H), 6.52 (s, 1H), 7.35 (s, 1H), 8.86 (broad s, 1H).

### Example 25:

### 1-[2,3-Dideoxy-2,3-didehydro-4-phosphonomethoxy-beta-D-erythrofuranosyl]thymine disodium salt (25)

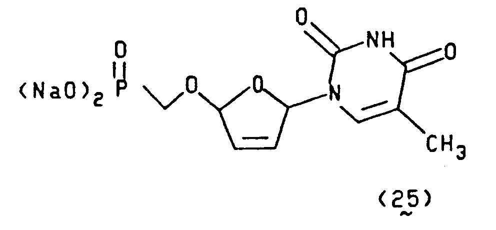

To a solution of 1-[2,3-dideoxy-4-(dimethylphosphono)methoxy-3-(phenylselenyl-beta-D-erythrofuranosyl]thymine (1.15 g, 2.76 mmol) in DMF (4 ml) was added at 5°C bromotrimethylsilane (3 ml) under nitrogen. After stirring for 4 hours at 5°C, volatiles were removed in vacuo and the residue was dissolved in aqueous saturated bicarbonate (3 ml) and evaporated again in vacuo to give a slightly yellow solid.
¹H-NMR (200 MHz, D₂O): δ 1.79 (s, 3H), 2.3-2.5 (m, 2H), 3.27 (1, J = 7.6, 8.4 Hz, 1H), 3.50 (1, J = 7.6, 8.4 Hz, 1H), 3.93 (d, J = 6.9 Hz, 1H), 5.23 (s, 1H), 6.0 (t, J = 6.9 Hz, 1H), 7.53 (s, 1H).

The reaction product from the above example was dissolved in water (5 ml) followed by sodium periodate (1.7 g, 8.0 mmol). After stirring for 30 minutes, the reaction mixture was heated at 80°C for 8 minutes and then filtered. The filtrate was evaporated to dryness and the residual solid was purified by a C₁₈ reverse phase column chromatography using water as an eluent under 0.56 bar (8 psi) pressure. Each 15 ml fraction was assayed by high pressure liquid chromatography. Lyophilization of combined fractions gave the title compound as a white amorphous solid: yield 538 mg (50%); mp 233-237°C.
Analysis: Calcd. for C₁₀H₁₁N₂O₇Na₂P H₂O: C, 32.61; H, 3.51; N, 7.61.
Found: C, 32.31; H, 3.63; N, 7.35.
¹³C-NMR (50.3 MHz, D₂O): δ 13.921, 67.870, 69.848, 89.868, 111.242, 111.364, 113.908, 131.177, 134.655, 139.350.
¹H-NMR (300 MHz, D₂O): δ 1.848 (s, 3H), 3.565 (dd, J = 8.4, 8.7 Hz, 1H), 3.738 (dd, J - 8.4, 8.7 Hz, 1H), 5.987 (s, 1H), 6.180 (d, J = 6.0 Hz, 1H), 6.432 (d, J = 6.0 Hz, 1H), 6.817 (s, 1H), 7.377 (s, 1H).
UV (H₂O): λ max 266 nm (ε = 10.134).

### Example 26:

### 1-[2,3-Dideoxy-2,3-didehydro-4-beta-(dimethylphosphono)methoxy-beta-D-erythrofuranosyl)thymine (26)

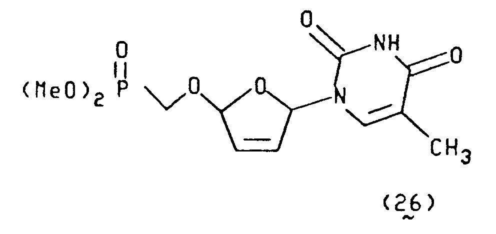

To a solution of 1-(2,3-dideoxy-4-beta-(dimethylphosphono)methoxy-3-(phenylselenyl)-beta-D-erythrofuranosyl] thymine (6.0 g, 12.2 mmol) in methanol (20 ml) was added dropwise a suspended solution of sodium bicarbonate (1.8 g, 21 mmol) and sodium periodate (3.2 g, 15 mmol) in water (20 ml). After stirring at 25°C for 1 hour, the mixture was heated at 80°C for 60 minutes. Volatiles were removed in vacuo and the residue was suspended in CH₂Cl₂ (120 ml). After removal of insoluble material, the organic phase was dried (MgSO₄) and evaporated in vacuo. The residue was chromatographed on silica gel using CH₂Cl₂-5% MeOH as eluent to give the title compound as a white amorphous powder: yield 3.4 g (85%).
Analysis: Calcd. for C₁₂H₁₇N₂O₇P: C, 43.38; H, 6.16; N, 8.43.
Found: C, 43.53; H, 5.20; N, 8.26.
¹³C-NMR (75.47 MHz, CDCl₃): δ 12.339, 52.889, 52.976, 53.080, 60.491, 62.738, 87.837, 108.402, 108.569, 111.653, 130.613, 131.675, 135.435, 150.480, 163.503.
¹H-NMR (300 MHz, CDCl₃): δ 1.862 (s, 3H), 3.748 (d, J = 12.8 Hz, 3H), 3.814 (d, J = 12.8 Hz, 1H), 3.826 (dd, J = 8.9, 8.4 Hz, 1H), 3.902 (dd, J = 8.9, 8.4 Hz, 1H), 5.711 (s, 1H), 6.075 (d, J = 5.7 Hz, 1H), 6.233 (d, J = 5.7 Hz, 1H), 6.915 (s, 1H), 7.129 (s, 1H), 8.95 (broad s, 1H).

### Example 27:

### 1-[2,3-Dideoxy-2,3-didehydro-4-beta-(methylphosphono) methoxy-beta-D-erthyrofuranosyl]thymine sodium salt (27)

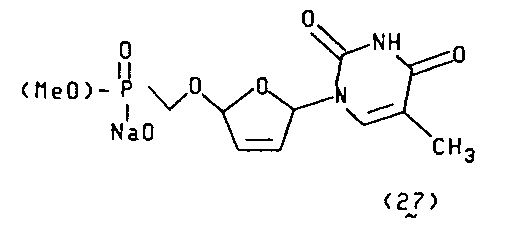

A solution of 1-[2,3-dideoxy-2,3-dihydro-4-beta-(dimethylphosphono)methoxy-beta-D-2-erthyrofuranosyl]thymine (180 mg, 0.54 mmol) in 1N-NaOH (2 ml) was stirred at 25°C for 2 hours. The reaction was carefully neutralized to pH 8.0 by dropwise addition of 1N-HCl with good stirring. Water was then evaporated in vacuo and the residue was purified by a C₁₈ reverse phase column using water -3% acetonitrile as an eluent to give the title compound as a white solid: yield 125 mg (68%).
Analysis: Calcd. for C₁₁H₁₄N₂O₇PNa 1.5 H₂O: C, 34.28; H, 4.93; N, 7.27.
Found: C, 34.02; H, 49.4; N, 7.19.
UV (H₂O): λ max 269 nm (ε=8160)
¹H-NMR (300 MHz, D₂O): δ 1.847 (s, 3H), 3.525 (d, J = 11.0 Hz, 3H), 3.917 (dd, J = 13.6, 17.0 Hz, 1H), 3.765 (dd, J = 13.6, 17.0 Hz, 1H), 5.849 (s, 1H), 6.198 (d, J = 4.6 Hz, 1H), 6.415 (d, J = 4.6 Hz, 1H), 6.847 (s, 1H), 7.374 (s, 1H).

### Example 28:

### 1-[2,3-Dideoxy-4-beta-(methylphosphono)methoxy-beta-D-erythrofuranosyl]thymine sodium salt (28)

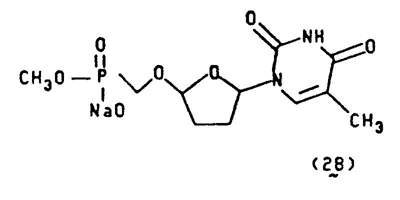

To a solution of 1-[2,3-dideoxy-2,3-didehydro-4-beta-(methylphosphono)methoxy-beta-D-erythrofuranosyl]thymine sodium salt (300 mg, 0.9 mmol) in water (20 ml) was added 10% palladium on active carbon (200 mg) and hydrogenated for 30 minutes under 2.49 bar (35 psi) H₂ pressure. The catalyst was filtered and washed with methanol (30 ml). The combined filtrate and wash was evaporated in vacuo and the residue was purified by a C₁₈ reverse phase column using water -2% acetonitrile under 0.56 bar (8 psi) pressure to give the title compound as a white solid: yield 250 mg (84%).

This material showed an identical nmr with compound 21A which was prepared from 2,5-dimethoxytetrahydrofuran and the stereochemical assignment of compound 21A was confirmed by the NOE.
¹H-NMR (300 MHz, D₂O): δ 1.943 (s, 3H), 2.168 (m, 2H), 2.418 (m, 2H, 3.608 (d, J = 6.9 Hz, 3H), 3.68 (dd, J = 13.5, 18.0 Hz, 1H), 3.85 (dd, J = 13.5, 18.0 Hz), 5.303 (s, 1H), 6.390 (d, J = 6.3 Hz, 1H), 7.627 (s, 1H).

In a manner similar to the above Example 28, the thymine-containing reactant can be replaced with a corresponding adenine, guanine or cytosine reactant to produce 1-[2,3-dideoxy-2,3-didehydro-4-beta-(methylphosphono)methoxy-beta-D-erythrofuranosyl] adenine sodium salt, or 1-[2,3-dideoxy-2,3-didehydro-4-beta-(methylphosphono)methoxy-beta-D-erythrofuranosyl] guanine sodium salt, or 1-[2,3-dideoxy-2,3-didehydro-4-beta-(methylphosphono)methoxy-beta-D-erythrofuranosyl cytosine sodium salt.

### Example 29:

### 1-[4-beta-(Dimethylphosphono)methoxy-beta-D-erythrofuranosyl]thymine (29)

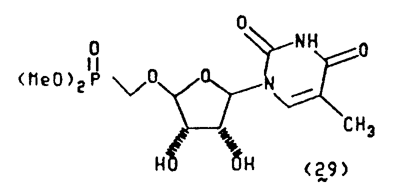

To a solution of 1-[2,3-dideoxy-2,3-didehydro-4-beta-(dimethylphosphono)methoxy-beta-D-erythrofuranosyl]thymine (3.31 g, 10 mmol) in pyridine (20 ml) was added osmium tetroxide (2.54g, 10mmol at 0°C and stirred for 2 hours. hours. The solvent was removed in vacuo. The residue was dissolved in ethyl acetate (100 ml), washed with 10% phosphoric acid (30 ml), water (20 ml), aqueous sodium bicarbonate (20 ml), brine and dried MgSO₄). The solvent was removed in vacuo, and the residual oil was chromatographed on silica gel using CH₂Cl₂-5% MeOH as an eluent to give the title compound as a white powder: yield 2.56 g (70%).
Analysis: Calcd. for CH₁₂N₁₉N₂O₉P: C, 39.35; H, 5.23; N, 7.65.
Found: C, 38.98; H, 5.09; N, 7.42.
¹³C-NMR (75.47 MHZ, d₆-DMSO): δ 11.861, 52.672, 53.797, 59.114, 61.411, 72.608, 73.316, 73.399, 87.419, 107.607, 107.863, 110.810, 135.217, 150.973, 163.581.
¹H-NMR (300 MHz, d₆-DMSO): δ 1.675 (s, 3H), 3.540 (d, J = 10.5 Hz, 6H), 3.736 (1, J = 4.0 Hz, 1H), 3.817 (d, J = 9.6 Hz, 2H), 4.03 (dd, J = 6.0, 11.2 Hz, 1H), 4.763 (s, 1H), 5.35 (d, J = 4.0 Hz, 1H), 5.45 (d, J = 7.0 Hz, 1H), 5.919 (d, J = 6.9 Hz, 1H), 7.128 (s, 1H), 11.228 (broad s, 1H).

In a manner similar to the above Example 29, the thymine-containing reactant can be replaced with a corresponding adenine, guanine or cytosine reactant to produce 9-[4-beta-(dimethylphosphono)methoxy-beta-D-erythrofuranosyl]adenine, or 9-[4-beta-(dimethylphosphono) methoxy-beta-D-erythrofuranosyl]guanine, or 1-[4-beta-(dimethylphosphono)methoxy-beta-D-erythrofuranosyl]cytosine.

### Example 30:

### 1-[4-(Methylphosphono)methoxy-beta-D-erythrofuranosyl]thymine Sodium Salt (30)

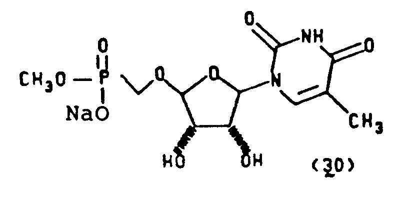

To a solution of 1-[4-beta-(dimethylphosphono)methoxy-beta-D-erythrofuranosyl]thymine (200 mg, 0.5 mmol) in 1N-NaOH (2 ml) was stirred at 25°C for 2 hours. The reaction was carefully neutralized to pH 9.0 by dropwise addition of 1N-HCl with stirring. Water was then evaporated in vacuo and the residual oil was purified by a C₁₈ reverse phase column using water-2% acetonitrile as an eluent under 8 psi pressure to give the title compound as a white amorphous powder: yield 114 mg (56%).
Analysis Calcd. for C₁₁H₁₅N₂O₉PNa 2H₂O: C, 32.27; H, 4.64; N, 6.84.
Found: C, 31.94; H, 4.32; N, 6.86.
UV (H₂O): λ max 268 nm (ε = 8153).
¹³C-NMR (75.47 MHz, D₂): δ 35.644, 35.719, 45.366, 47.479, 57.032, 57.411, 71.915, 92.426, 92.599, 96.463, 120.503, 137.007, 144.422, 151.377.
¹H-NMR (300 MHz, D₂O): δ 1.922 (s, 3H), 3.615 (D, J = 10.1 Hz, 3H), 3.687 (dd,J = 11.3, 11.1 Hz, 1h), 3.885 (dd, J = 11.3, 11.1 Hz, 1H), 4.220 (d, J = 4.3 Hz, 1H), 4.574 (dd, J = 6.7, 4.3 Hz, 1H), 5.09 (s, 1H), 6.175 (d, J = 6.7, Hz, 1H), 7.485 (s, 1H).

In a manner similar to the above Example 30, the thymine-containing reactant can be replaced with a corresponding adenine, guanine or cytosine reactant to produce 9-[4-beta-(methylphosphono)methoxy-beta-D-erythrofuranosyl]adenine, or 9-[4-beta-(methylphosphono)methoxy-beta-D-erythrofuranosyl]guanine, or 1-[4-beta-(methylphosphono)methoxy-beta-D-erythrofuranosyl]cytosine.

### Example 31:

### 1-(4-beta-Phosphonomethoxy-beta-D-erythrofuranosyl)thymine disodium salt (31)

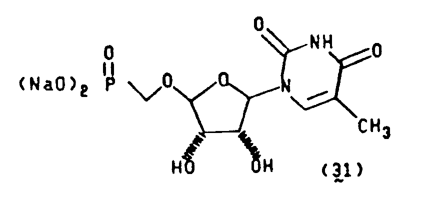

To a solution of 1-[4-beta-(diemthylphosphono)methoxy-beta-D-erythrofuranosyl]thymine (320 mg, 0.87 mmol) in DMF (2 ml) was added at 0°C bromotrimethylsilane (1.4 ml) under nitrogen. After stirring 90 minutes at 0°C, volatiles were removed in vacuo and the residue was neutralized to pH 8.0 by addition of aqueous saturated sodium bicarbonate. Water was then evaporated in vacuo and the residual solid was purified by a C₁₈ reverse phase column using water-2% acetonitrile as an eluent to give the title compound as a white solid: yield 153 mg (46%). mp >250°C. (decomposition).
Analysis: Calcd. for C₁₀H₁₃N₂O₉PNa 2H₂O: C, 27.52; H, 4.35; N, 6.42.
Found: C, 27.05; H, 3.99; N, 6.12.
UV (H₂O): λ max 268 nm (ε = 7,568).
¹³C-NMR (75.47 MHz, D₂O): δ 49.590, 51.582, 57.347, 57.541, 71.861, 93.034, 93.169, 95.541, 121.178, 136.393, 144.222, 150.601.
¹H-NMR (300 MHz, D₂O): δ 1.936 (s, 3H), 3.469 (dd, J = 12.3, 12.6 Hz, 1H), 3.756 (dd, J = 12.3, 12.6 Hz, 1H), 4.258 (d, J = 4.5 Hz, 1H), 4.578 (dd, J = 4.5, 6.2 Hz, 1H), 5.175 (s, 1h), 6.182 (d, J = 6.2 Hz, 1H), 7.591 (s, 1H).

In a manner similar to the above Example 31, the thymine-containing reactant can be replaced with a corresponding adenine, guanine or cytosine reactant to produce 9-[4-beta-phosphonomethoxy-beta-D-erythrofuranosyl]adenine disodium salt, or 9-[4-beta-phosphonomethoxy-beta-D-erythrofuranosyl]guanine disodium salt, or 1-[4-beta-phosphonomethoxy-beta-D-erythrofuranosyl] cytosine disodium salt.

### Example 32:

### 1-[2-Deoxy-4-beta-(diethylphosphono)methoxy-beta-D-erythrofuranosyl]thymine (32A) and the trans isomer (32B).

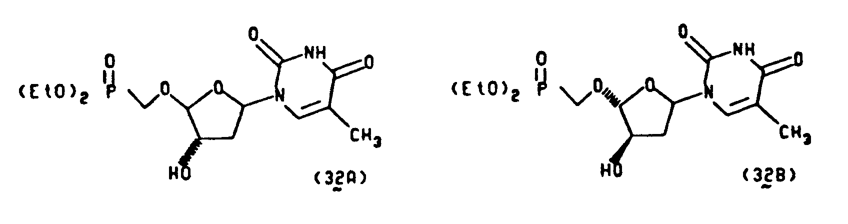

To a solution of 1-(2,3-dideoxy-3,4-didehydro-beta-D-erythrofuranosyl)thymine (2.4 g, 12.4 mmol) and diethylphosphonomethanol (17.4 g, 103 mmol) in CH₂Cl₂ ( 2 ml) was added 80-85% 3-chloroperoxybenzoic acid (17.4 g, 13.14 mmol) at 5°C. After stirring for 60 minutes at 25°C, the reaction mixture was purified by column chromatography on silica gel using CH₂Cl₂-3% MeOH to obtain the crude product, which was carefully rechromatographed on silica gel to separate the two isomers. Using CH₂Cl₂-1% MeOH, the minor isomer B was first eluted and obtained as a colorless oil: yield 75 mg (1.7%).
¹H-NMR (300 MHz, CDCl₃) of 32B: δ 1.287 (t, J = 6.9 Hz, 6H), 1.900 (s, 3H), 1.97-2.58 (m, 2H), 3.807 (dd, J = 9.0, 13.8 Hz), 4.016 (dd, J = 9.0, 13.8 Hz, 1H), 4.138 (m, 4H), 4.30 (m, 1H), 4.934 (d, J = 4.2 Hz, 1H), 6.321 (t, J = 6.6 Hz, 1H), 7.417 (s, 1H), 9.80 (broad s, 1H).

The silica gel column was continuously eluted with CH₂Cl₂-3% MeOH to obtain the major isomer 32A as a colorless oil: yield 735 mg (17%).
¹H-NMR (300 MHz, CDCl₃) of 32A: δ 1.310 (t, J = 7.5 Hz, 6H), 1.872 (s, 3H), 1.95 (m, 1H), 2.70 (m, 1H), 3.780 (dd, J = 9.3, 13.8 Hz, 1H), 3.928 (dd, J = 9.3, 13.8 Hz, 1H), 4.139 (m, 4H), 4.330 (d, J = 5.7 Hz, 1H), 5.268 (s, 1H), 6.238 (dd, J = 2.7, 8.4 Hz, 1H), 7.624 (s, 1H), 9.176 (broad s, 1H).

The stereochemical assignment of 32A and 32B was consistent with nmr NOE observation.

In a manner similar to the above Example 32, 1-(2,3-deoxy-3,4-didehydro-beta-D-erythrofuranosyl)thymine can be replaced with a corresponding adenine, guanine or cytosine reactant to produce 9-[2-deoxy-4-beta-(diethylphosphono)methoxy-beta-D-erythrofuranosyl]adenine, 9-[2-deoxy-4-beta-(methylphosphono)methoxy-beta-D-erythrofuranosyl]adenine sodium salt, or 9-[2-deoxy-4-beta-(diethylphosphono)methoxy-beta-D-erythrofuranosyl]guanine, or 1-[2-deoxy-4-beta-(diethylphosphono)methoxy-beta-D-erythrofuranosyl]cytosine.

### Example 33:

### 1-(2-Deoxy-4-beta-phosphonomethoxy-beta-D-erythrofuranosyl)thymine disodium salt (33)

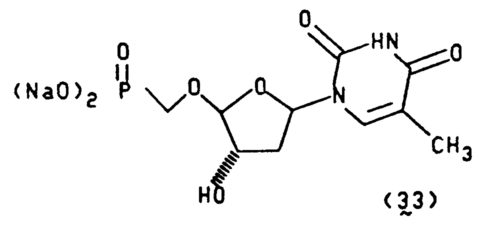

To a solution of 1-[2-deoxy-4-(diethylphosphono)methoxy-beta-D-erythrofuranosyl]thymine (480 mg, 1.3 mmol) in DMF (2 ml) was added bromotrimethylsilane (2 ml) under nitrogen. After stirring 3 hours at 25°C, volatiles were removed in vacuo and the residue was carefully neutralized to pH 8.5 by addition of aqueous saturated sodium bicarbonate. Water was evaporated to dryness and the residual solid was purified by a C₁₈ reverse phase column using water-3% acetonitrile as a eluent to give the title compound as a white powder: yield 165 mg (40%).
Analysis: Calcd. for C₁₀H₁₃N₂O₈PNa₂ 3H₂O: C, 28.57; H, 4.52; N, 6.67.
Found: C, 28,48; H, 4.49; N, 6.52.
UV (H₂O): λmax 268 nm (ε = 7,602).
¹³C-NMR (75.47 MHz, D₂O): δ 20.803, 48.289, 50.291, 56.714, 69.570, 94.015, 94.157, 94.994, 122.321, 135.750, 150.715.
¹H-NMR (300 MHZ, D₂O): δ 1.853 (s, 3H), 1.924 (dd, J = 2.6, 13.4 Hz, 1H), 2.8=785 (dd, J = 2.6, 5.3, 8.0 Hz, 1H), 3.415 (dd, J = 8.9, 12.4 Hz, 1H), 3.669 (dd, J = 8.9, 12.4 Hz, 1H), 4.372 (d, J = 5.3 Hz, 1H), 4.372 (d, J = 5.3 Hz, 1H), 4.372 (d, J = 5.3 Hz, 1H), 5.310 (s, 1H), 6.285 (dd, J = 2.6, 8.0 Hz, 1H), 7.776 (s, 1H).

In a manner similar to the above Example 33, the thymine-containing reactant can be replaced with a corresponding adenine, guanine or cytosine reactant to produce 9-(2-deoxy-4-beta-phosphonomethoxy-beta-D-erythrofuranosyl)adenine disodium salt, or 9-(2-deoxy-4-beta-phosponomethoxy-beta-D-erythrofuranosyl)-guanine disodium salt, or 1-(2-deoxy-4-beta-phosphonomethoxy-beta-D-erythrofuranosyl)cytosine disodium salt.

### Example 34:

### 2-Acetamino-6-diphenylcarbamoyloxy-9-[2-(phenylselenyl) ethoxymethyl]purine (34):

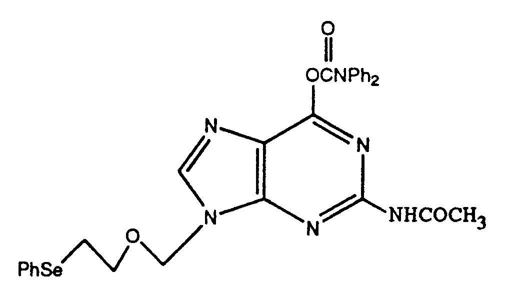

A mixture of 2-acetamino-6-diphenylcarbamoylpurine (36.7g, 94.6 mmol) [prepared according to the following literature procedure: R. Zou and M.J. Ropbins, Can. J. Chem., 65, No. 6, 1436 (1987)] and N,O-bis(trimethylsilyl)acetamide (47.6 ml, 193 mmol) in dry dichloroethane (700 ml) was heated at 80°C for 60 minutes. Volatiles were removed in vacuo and the residue was evaporated with toluene twice. The silylated purine and mercuric cyanide (29.6g, 117 mmol) in benzene (800 ml) was heated at reflux for 60 minutes, then a solution of 2-(phenylselenyl)ethoxymethyl chloride (24g, 94.5 mmol) in benzene (100 ml) was added dropwise. The mixture was refluxed for 4 hours and then allowed to stir for 15 hours at 25°C. The reaction was diluted with CH₂Cl₂ (500ml) and quenched with aqueous saturated bicarbonate (1l). The organic phase was washed with 2N potassium iodide (200 ml), dried (MgSO₄) and the solvents were removed in vacuo. The residual oil was chromatographed on silica gel using CH₂Cl₂-5% MeOH as an eluent to provide the title compound as a slightly yellow powder: yield 22g (39%).
Analysis: Calcd. for C₂₉H₂₅N₆O₄Se: C, 57.91, H, 4.36; N, 13.98. Found: C, 57.76; H, 4.46; N, 13.48.
¹H-NMR (300 MHz, CDCl₃): δ 2.459 (s, 3H), 2.951 (t,J = 6.9 Hz, 2H), 3.714 (t, J = 6.9 Hz, 2H), 5.477 (s, 2H), 7.07-7.7 (m, 15H), 8.001 (s, 1H), 8.171 (s, 1H).
¹³C-NMR (75.45 MHz; CDCl₃): δ 25.133, 26.196, 69.192, 72.602, 120.363, 126.970, 127.014, 129.174, 141.686, 143.734, 150.247, 152.487, 155.232, 156.247, 156.297, 170.793.

### Example 35: 2-Acetamino-6-diphenylcarbamoyl-9-(vinyloxymethyl) purine (35):

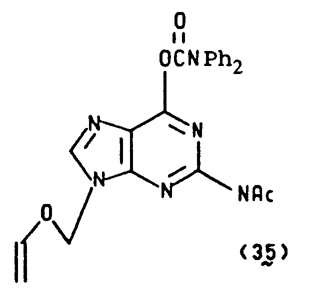

To a solution of 2-acetamino-6-diphenylcarbamoyl-9-[2-(phenylselenyl)-ethoxymethyl]purine (4.92g, 8.16 mmol) in dioxane (80 ml) was added to 30% H₂O₂ (4 ml, 35 mmol) and sodium bicarbonate (2.1g, 24.5 mmol). The mixture was heated at 60°C for 20 minutes. The reaction was then concentrated to about 10mL, diluted with ethyl acetate (100 ml), dried (MgSO₄) and the solvents were removed in vacuo. The residue was dissolved in dioxane (40 mL), diisopropylethylamine (1.27g, 10 mmol) was added and the solution was heated at 80°C for 30 minutes under nitrogen. The solvent was evaporated in vacuo and the residual oil was chromatographed on silica gel using CH₂Cl₂-ethyl acetate (1:1) as an eluent to give the title compound as a yellowish powder: yield 2.3g (65%).
Analysis: Calcd. for C₂₃H₂₀N₆O₄ 0.5H₂O: C, 60.98; H, 4.67; N, 18.55. Found: C, 61.20; H, 4.76; N, 18.84.
¹H-NMR (300 MHz, CDCl₃): δ 2.485 (s, 3H), 4.170 (dd, J = 2.7, 6.6 Hz, 1H), 44.473 (dd, J = 2.7, 14.1 Hz, 1H), 6.395 (dd, J = 6.6, 14.1 Hz, 1H). 7.0-7.5 (m, 10H), 7.961 (s, 1H), 7,996 (s, 1H).
¹³C-NMR (75.47 MHz, CDCl₃): δ 25.121, 70.572, 92.427, 126.272, 126.344, 126.443, 126.496,, 126.566, 126.644, 141.628, 143.226, 148.925, 152.552, 170.505.

### Example 36:

### 2-Acetamino-6-diphenylcarbamoyloxy-9-[(2-hydroxy-1-(dimethylphonomethoxy)ethoxymethyl]purine (36):

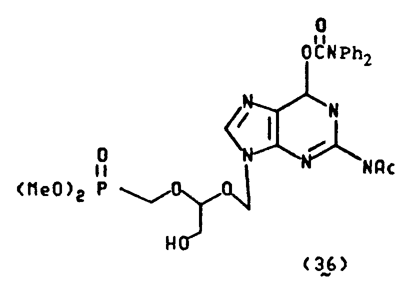

To a suspension of 2-acetamino-6-diphenylcarbamoyloxy-9-(vinyloxymethyl)purine (1.0g, 2.25 mmol) and dimethylphosphonomethanol (6 ml) in CH₂Cl₂ (6 ml) was added 80-85% m-chloroperbenzoic acid (611 mg, 3 mmol). After stirring for 18 hours at 25°C, the clear solution was diluted with CH₂Cl₂ (100 ml) and washed with ice cold 1N-NaOH (4 mL) and brine (20 ml). The organic phase was washed again with brine (20 ml), dried (MgSO₄) and the solvent was removed in vacuo. The residual oil was chromatographed on silica gel using using CH₂Cl₂-5% MeOH as an eluent to give the title compound as a colorless oil: yield 360 mg (27%).
Analysis: Calcd. for C₂₆H₂₉N₆O₉P 0.5CH₂Cl₂: C, 49.22; H, 4.64; N, 13.00. Found: C, 49.89; H, 4.47; N, 12.76.
¹H-NMR (300 MHz, CDCl₃): δ 2.384 (s, 3H), 3.582 (dd. J = 4.5, 12.5 Hz, 1H), 3.722 (dd, J =4.5, 12.5 Hz, 1H), 3.768 (dd, J = 2.9, 10.7 Hz, 6H), 3.798 (dd, J = 8.9, 14.0 Hz, 1H), 3.994 (dd, J = 8.9, 14.0 Hz, 1H), 4.910 (t, J =4.9 Hz, 1H), 5.649 (d, J = 10.8 Hz, 1H), 5.723 (d, J =10.8 Hz, 1H), 7.0-7.4 (m, 10H), 8.032 (s, 1H), 8.662 (s, 1H).

### Example 37: 9[(2-Hydroxy-1-(methylphosphonomethoxy)ethoxymethyl]guanine ammonium salt (37):

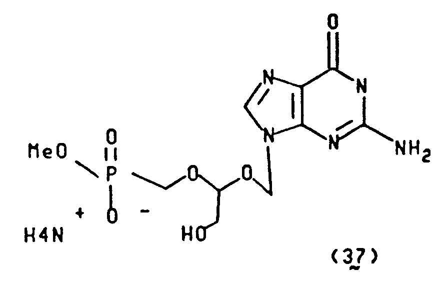

A solution of 2-acetamino-6-diphenylcarbamoyloxy-9[(2-hydroxy-1-(dimethylphosphonomethoxy)ethoxy)methyl]guanine (2.9g, 4.8 mmol) in methanol (300 ml) and 28% NH₄OH (300 ml) was heated at 60°C for 90 minutes. The solution was concentrated in vacuo and the residual oil was purified by C-18 reverse phase column chromatography using water as eluent under 0.56 bar (8 psi) pressure. The fractions having ultraviolet fractions were checked with HPLC, combined and lyophilized to give the title compound as a white powder: yield 1.15g (65%).
Analysis: Calcd. for C₁₀H₁₉N₆O₇P H₂O: C, 31.26; H, 5.51; N, 21.87. Found: C, 31.63; H, 5.43; N, 21.72.
¹H-NMR (300 MHz, D₂O): δ 3.549 (d, J = 10.5 Hz, 3H), 3.571 (dd, J = 4.8, 13.2 Hz, 1H), 3.675 (dd, J = 9.3, 13.2 Hz, 1H), 3.4-3.6 (m, 2H), 4.844 (t, J = 3.9 Hz, 1H), 5.573 (d, J = 11.4 Hz, 1H), 5.638 (d, J = 11.4 Hz, 1H), 7.934 (s, 1H).
UV (H₂O): λ max 252 nm (ε = 13, 871).

### Example 38: 9[(2-Hydroxy-1-(phosphonomethoxy)ethoxy)methyl]guanine disodium salt (38):

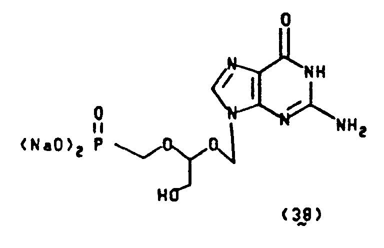

To a solution of 9-[2-hydroxy-1-(methylphosphonomethoxy)ethoxy)methyl]guanine ammonium salt (780 mg, 2.0 mmol) in dry DMF (20 ml) was added at 5°C bromotrimethysilllyl (8 ml, 60 mmol) under nitrogen. After stirring for 3 hours at 5°C, volatiles were removed in vacuo and the residue was dissolved in aqueous saturated bicarbonate and re-evaporated in vacuo to a solid. Purification of this material by C-18 reverse phase column chromatography using water as eluent under 0.56 bar (8 psi) pressure and lyophilization of combined fractions gave the title compound as a white powder: yield 520 mg (62%).
Analysis: Calcd. for C₉H₁₂N₅O₇PNa₂ 2H₂O: C, 26.04; H, 3.89; N, 16.87; Found: C, 26.25; H, 4.05; N, 16.89.
UV (H₂O): λ max 252nm (ε = 15,150).
1H-NMR (300MHz, D2O): δ 3.40-3.50 (m, 2H), 3.605 (dd, J = 5.4, 11.6 Hz, 1H), 3.698 (dd, J =9.0, 11.6 Hz, 1H), 4.877 (t, J = 4.5 Hz, 1H), 5.665 (d, J = 11.3 Hz, 1H), 5.725 (d, J = 11.3 Hz, 1H), 7.999 (s, 1H).
¹³C-NMR (75.47 MHz, D₂O): δ 63.268, 65.832, 67.834, 71.636, 104.561, 104.712, 117.980, 141.823, 153.521, 156.320, 161.129.

### Example 39: 1-(5-Methoxytetrahydro-2-furyl)thymine (3A)

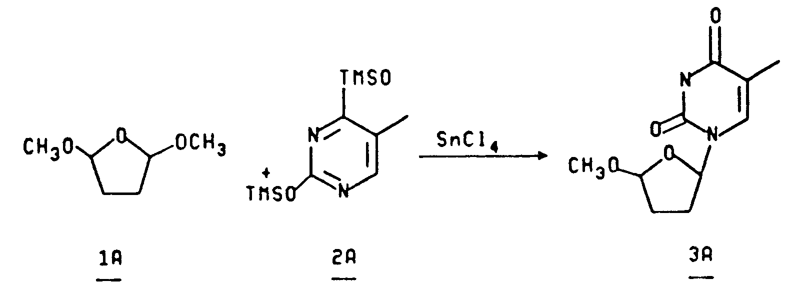

To a suspension of 2.5 g (20 mmol) of dry, powdered thymine in 30 ml of hexamethyldisilazane was added 50 mg of ammonium sulfate and 0.5 ml of trimethylsilyl chloride. The mixture was heated at 140-145°C (for 4 hours to obtain a clear solution). The excess hexamethyldisilazane was removed at reduced pressure, then the residual white oil was dissolved in xylene and evaporated in high vacuum to give a colorles viscous oil. The crude silyated thimine was dissolved in 40 ml of dichloroethane and cooled to -30°, followed by 7.8g (60 mmol) of 2.5 dimethoxytetrahydrofuran. To this solution was added 2.3 mL of tin tetrachoride via a syringe over 2 minutes, then stirred for 10 minutes under nitrogen. The mixture reaction was poured into ice-cold aqueous NaHCO₃ (100 ml)-ethylacetate (100 ml). The milky solution was filtered through celite and the organic layer was separated and dried over MgSO₄. Evaporation of the dried solvents gave a yellow oil which was chromatographed on SiO₂ (CH₂Cl₂-MeOH) to give 4.3 g (95%) of 3A as a colorless oil. This oil was a mixture of the two isomers (cis/trans) in a ratio of 1:1 as seen by analytical HPLC and ¹H-NMR.
¹H-NMR (CDCl₃) δ 1.4-2.2 (m, 7H), 3.40 and 3.42 (two s, 3H), 5.2 and 5.25 (two broad s, 1H), 6.20 and 6.41 (two q, 1H, J = 3.5, 7.0 Hz, and 4.0, 7.2 Hz), 7.02 and 7.40 (two s, 1H)

### Example 40:

### 1-(5-Diethylphosphonomethoxytetrahydro-2-furyl)thymine (5A)

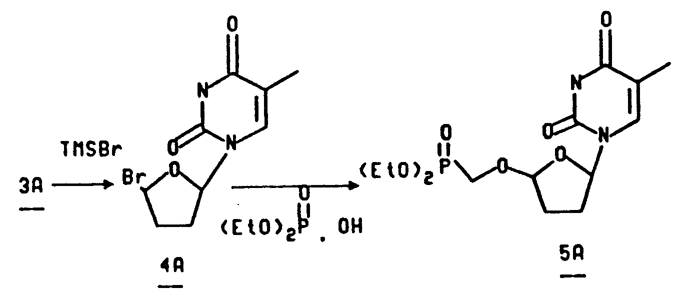

To a solution of 600 mg (2.65 mmol) of 3A (Example 39) in 15 ml of methylene chloride was added 0.6 ml of trimethylsilyl bromide and heated at 40-45°C for 10 minutes under nitrogen. The reaction was evaporated in vacuo to give 4AW as a yellow oil which was dissolved in 20 ml of methylne chloride followed by 440 mg (2.60 mmol) of diethylphosphonomethyl alcohol. This solution was cooled to -10°C followed by 0.6 ml of triethylamine and stirred for 15 minutes without the cooling bath. After dilution with 40 ml of ethylacetate, the reaction was washed with water and brine. Evaporation of the dried (MgSO₄) solvent gave a yellow oil which was chromtographed over SiO₂(CH₂CH₂-MeOH) to give 180mg (18.5%) of 5A as a white oil. This oil was a mixture of the two isomers cis/trans in a ratio of 1:1, as seen by analytical HPLC and ¹H NMR; ¹H-NMR(CDCl₃) δ 1.25 (t, 6H, J = 7.0 Hz), 1.95 and 2.0 (two S, 3H), 3.8-4.0 (m, 2H),4.0-4.2 (m, 4H), 5.2 and 5.3 (two broad s, 1H), 6.2 and 6.42 (q,q, 1H, J =3.5, 7.0 Hz and 4.0, 7.2 Hz), 7.0 and 7.4 (two s, 1H).

### Example 41: Testing and evaluation of compounds against herpes virus

### A. Plaque Reduction Assay

Herpes simplex virus (HSV) strains were grown and titered at 37°C in vero cells (African Green Monkey Kidney cells) and used for virus work before the tenth passage.

Cells were grown and maintained in Earle's Minimum Essential Medium (EMEM), Gibco Laboratories, supplemented with 0.75% sodium bicarbonate, 2mM 1-glutamine, Pen-strep. and 5-10% fetal calf serum.

The titer of HSV strains is determined by a plaque titration method (Roizman and Roane, Virology, 115:75-79, 1961). Tissue culture 24-well petri dishes are seeded with cells and used for assays when approximately 75% monolayer. Volumes (0.1ml) of logarithmic dilutions of the virus strain are inoculated onto each of triplicate wells, and absorbed for one hour with intermittent shaking. The inoculum thereafter is removed, and 1 ml of 5-10% EMEM containing 0.3% human immune serum globulin is added. After a 48 hour incubation period at 37°C in a 5% CO₂ atmosphere, the overlay medium is removed and the cell sheets stained with Giemsa stain. The number of plaques is counted, the triplicate is averaged, and the number of plaque-forming units per ml is calculated.

The compounds are tested for activity against the herpes simplex stains using a stock solution of each compound freshly prepared. Appropriate dilution of each compound are made in 10% EMEM before usage. The antiviral efficacy of each compound is determined using the plaque reduction assay described above. Briefly, tissue culture 24-well plates, with approximately 50 plaque forming units of HSV per 0.1 ml, and the virus absorbed for 1 hour, with intermittent shaking. After removal of the inoculum, 1 ml of 10% EMEM containing two-fold dilutions of the appropriate drug are added in triplicates. Triplicate wells/plate receives no drug and are used as a virus control. After a 48-hour incubation period, at 37°C in a 5% CO₂ atmosphere, the overlay medium is removed, the cells are stained as described above, and plaques are counted. The counts of triplicate wells are averaged, and the number of plaques in the presence of each drug dilution are calculated.

The antiviral potency of the drug is determined by ID₅₀, the drug concentration necessary to reduce the number of plaques by 50% of those in the virus control cultures.

The results are shown in Table 1 herein below.

**Table 1**

| Antiviral Test REsults of Compound 8 (see Example 8) against HSV-1 and HSV-2 | | |
|---|---|---|
| Compound | ID₅₀(µg/ml) | |
| | HVS-1 | HVS-2 |
| ACV (Acyclovir) | 0.5 | 0.5 |
| Compound 8 | 2.6 | 11 |

### Example 42: Comparison of Compound 8 and Acyclovir (ACV) In Vivo

Groups of ten mice were inoculated intraperitoneally with from 200 to 600 PFU/0.2ml of Herpes simplex virus-1 (HL-34 strain). Different doses of test compound were administered to separate groups of animals on a BID basis for five consecutive days commencing three hours after inoculation. Treatment was by an intraperitoneal route. The experiment was teminated 21 days post inoculation and the number of survivals in each group was counted. The mean survival time (MST, days) was calculated.

The results are shown in Table 2. Compound 8 is equally as active as ACV.

**Table 2**

| Antiviral Effect of Compound 8 Against HSV-1 Systemic Infection in Mice | | | |
|---|---|---|---|
| Compound | Dose (mg/kg/d) | Route | Survival |
| Compound 8 | 300 | i.p. | 10/10 |
| | 100 | i.p. | 9/10 |
| | 10 | i.p. | 3/10 |
| ACV | 200 | i.p. | 6/7 |
| | 100 | i.p. | 7/10 |
| Treatment was initiated 3 hours post-infection and was given BID for 5 consecutive days. | | | |

### Example 43: Testing andevaluating of compounds against Murine Retroviruses:

The compounds were evaluated for antiviral activity against murine leukemia virus (MuLV) strains using the UV-XC plaque assay (Rowe, et al., Virology, 42:1136, 1970).

The MuLV strains were grown in feral mouse cells (SC-1) and used for antiviral tests using the UV-XC plaque assay. Briefly, SC-1 cells are grown as monolayers in 4-well tissue culture plates and inoculated with approximately 50-100 plaque forming units of MuLV in 0.5 ml of 5% EMEM containing 20 µg/ml DEAE/Dextran. After 1 hour adsorbtion, the inoculum is removed and 5 ml of 5% EMEM containing three-fold dilutions of the appropriate drug are added. Five days later, the cultures are UV-irradiated with an ultraviolet lamp and rat XC sarcoma cells are added to the cultures. Three-four days after UV-irradiation, the cell cultures are stained with Giemsa stain and the plaques are enumerated. Antiviral activity is expressed in terms of the reduction in the mean number of UX-XC plaques counted in the drug treated, virus-infected cultures compared with mean number of plaques counted in untreated, virus-infected control cultures.

### Example 44: In Vitro Evaluation Against HIV.

The HIV in vitro assay described as follows was used: The anti-HIV/LAV activity is measured in cultures of CEM-F cells. The CEM cells are infected with approximately 30 TCID₅₀ (50% tissue culture infectious dose of HIV (LAV strain). The cells are then incubated for 45 minutes at 37°C. The test compounds in culture medium are added at various concentrations to the infected cells and then incubated for a further 8 days. After 8 days the antiviral activity was evaluated in the culture media supernatant for p-24 gag protein by an enzyme capture assay (ELISA). The antiviral activity was expressed as the dose that inhibits 50% of the virus expression (ID₅₀ in µg/mL) as detected by the assay described.

**Table 3**

| Antiviral Test Results of Compound 25 (see Example 25) against Retroviruses | | |
|---|---|---|
| Compound | ID₅₀(µg/mL) | |
| | R-MuLV | HIV |
| AZT | 0.05 | 0.1 |
| Compound 25 | 0.3 | 1.8 |

It will be appreciated that the instant specification and claims are set forth by way of illustration and not limitation and that various modifications and changes may be made without departing form the spirit and scope of the present invention.

### Example 45

### 6-N-Pivaloyl-9-(2,3-dideoxy-3,4-didehydro-β-D-erythrofuranosyl)-adenine

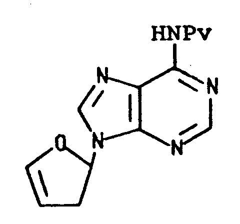

To a solution of 9-(2,3-dideoxy-3,4-didehydro-β-D-erythrofuranosyl)adenine (2.0 g, 10 mmol) [prepared according to the literature procedure: J. Zemlicka, R. Gasser, J. V. Freisler, J. P. Horwitz, J. Amer. Chem. Soc., 94, 3213 (1972)] in 1,2-dichloroethane (10 ml) was added pyridine (1 ml), dimethylaminopyridine (170 mg) and pivaloyl chloride (1.5 g, 12 mmol). The resulting solution was heated at 55-60°C for 6 h under nitrogen. The mixture was then concentrated in vacuo, taken up in CH₂Cl₂ and washed with water, 20% H₃PO₄ and brine, dried over MgSO₄, and evaporated in vacuo. The residual oil was chromatographed on silica gel using CH₂Cl₂-3% MeOH as eluent to give 2 (2.45 g, 85%) as a white powder.
¹H NMR (CDCl₃) δ 1.24 (s, 9H), 2.29 (ddd, J=3.5, 5.0, 17.1 Hz, 1H), 3.33 (dddd, J=2.4, 5.0, 9.4, 17.1 Hz, 1H), 5.24 (dd, J=2.4, 5.0 Hz, 1H), 6.41 (dd, J=3.5, 9.4 Hz, 1H), 6.48 (dd, J=2.4, 5.0 Hz, 1H), 8.18 (s, 1H), 8.73 (s, 1H).

### Example 46

### 6-N-Pivaloyl-9-[2,3-dideoxy-4-β-chloro-3-α-(phenylselenyl)-β-D-erythrofuranosyl]adenine

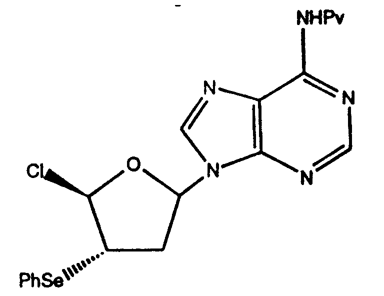

To a solution of the product of Example 45 (3.5 g, 12.2 mmol) in CH₂Cl₂ (40 ml) was added at -25°C a solution of phenylselenyl chloride (2.7 g, 14.0 mmol) in CH₂Cl₂ (7 ml) over 5 min under nitrogen. After stirring for 30 min at -25°C, the solvent was removed in vacuo to give 3 as a yellow oil. This material was used promptly for the next reaction:
¹H NMR (CDCl₃) δ 1.41 (s, 9H), 2.88 (m, 1H), 3.23 (m, 1H), 4.36 (d, J=6.3 Hz, 1H), 6.29 (s, 1H), 6.80 (dd, J=6.6, 8.4 Hz, 1H), 8.53 (s, 1H), 8.77 (s, 1H).

### Example 47

### 6-N-Pivaloyl-9-[2,3-dideoxy-4-β-dimethylphosphono)methoxy-3-α-(phenylselenyl)-β-D-erythrofuranosyl]adenine

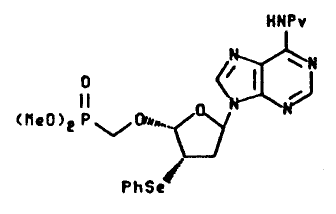

To a solution of the product of Example 46 (approximately 12 mmol) and dimethyl hydroxymethylphosphonate (16.8 g, 120 mmol) [prepared according to the literature procedure: D.P. Philion and S.S. Andres, Tetrahedron Lett. 27, 1477 (1986)] in CH₂Cl₂ (15 ml) was added at -25°C a suspended solution of silver perchlorate (4.0 g, 20 mmol) in CH₂Cl₂ (10 ml) and dimethyl hydroxymethylphosphonate (5 ml) over 5 min under nitrogen. The mixture was allowed to warm to 0°C, stirred for 60 min and was then poured into CH₂Cl₂ (100 ml)-aqueous bicarbonate (100 ml)-brine (50 ml). The organic phase was separated after filtration, fried over MgSO₄ and evaporated. The residual oil was chromatographed on silica gel using CH₂Cl₂-5% MeOH as eluent to give 4 (3.2 g, 45%) as a colorless oil:
¹H NMR (CDCl₃) δ 1.25 (s, 9H), 2.61 (ddd, J=2.7, 6.6, 14.4 Hz, 1H), 2.96 (ddd, J=6.6, 7.5, 14.4 Hz, 1H), 3.68 (d, J=11 Hz, 6H), 3.7-4.0 (m, 3H), 5.24 (s, 1H), 8.27 (s, 1H), 8.67 (s, 1H).

### Example 48

### 6-N-Pivaloyl-9-[2,3-dideoxy-2,3-didehydro-4-β-(dimethylphosphono)methoxy-β-D-erythrofuranosyl]adenine

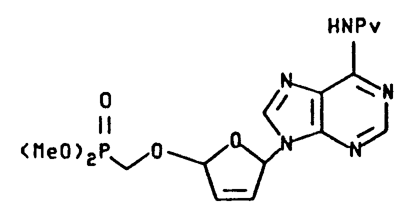

To a solution of the product of Example 47 (3.3 g, 5.6 mmol) in dioxane (30 ml) was added a solution of sodium periodate (6.3 g, 30 mmol) in water (30 ml) and the resulting solution was stirred at 23°C for 4 h. CH₂Cl₂ (200 ml) was added and the mixture was filtered through celite. The organic phase was washed with water, brine, dried over MgSO₄ and evaporated in vacuo. The residual oil was chromatographed on silica gel using CH₂Cl₂-5% MeOH as eluent to give 5 (1.4 g, 57%) as a colorless oil:
¹H NMR (CDCl₃) δ 1.31 (s, 6H), 3.64 (d, J=10.8 Hz, 3H), 3.71 (d, J=10.8 Hz, 3H), 3.84 (dd, J=8.7, 9.9 Hz, 1H), 5.87 (s, 1H), 6.27 (d, J=6.0 Hz, 1H), 6.34 (dd, J=1.5, 6.0 Hz, 1H), 7.00 (d, J=1.5 Hz, 1H), 8.04 (s, 1H), 8.66 (s, 1H);
¹³C NMR (CDCl₃) δ 27.524, 40.607, 53.318 (d, J=6.2 Hz), 61.598 (d, J-165 Hz), 86.247, 109.202, 123.406, 130.657, 132.679, 141.966, 150.205, 152.056, 176,547.

### Example 49

### 9-[2,3-Dideoxy-2,3-didehydro-4-β-(methylphosphono)methoxy-β-D-erythrofuranosyl]adenine ammonium salt.

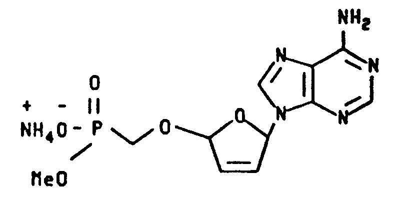

A solution of the product of Example 48 (330 mg, 0.78 mmol) and sodium methoxide (250 mg, 4.6 mmol) in methanol (10 ml) was stirred at 23°C for 18 h. The reaction was carefully neutralized to pH 5.0 by dropwise addition of 2N-HCl in an ice bath. The pH of the solution was readjusted to 8.0 by concentrated NH₄OH and volatiles were removed in vacuo. The solid residue was purified by C₁₈ reverse-phase column using water as eluent under 0.56 bar (8 psi) pressure to give 6 (141 mg, 49%) as a white amorphous powder:
¹H NMR (D₂O) δ 3.34 (d, J=10.5 Hz, 3H), 3.65 (dd, J=9.2, 13.2 Hz, 1H), 3.80 (dd, J=9.2, 13.2 Hz, 1H), 5.99 (s, 1H), 6.57 (s, 2H), 6.83 (s, 1H), 8.11 (s, 1H), 8.14 (s, 1H);
¹³C NMR (D₂O) 58.542 (d, J=3.0 Hz), 68.483 (d, J-150 Hz), 92.797, 116.319, 125.121, 136.147, 139.719, 147.131, 155.275, 159.781, 162.359;
UV max (H₂O) 260 nm (ξ 12,964).
Anal. Calcd for CH11^{H}13^{N}5^{O}5 PNa H₂O: C, 35.97; H, 3.90; N, 1907.
Found: C, 35.53; H, 3.71; N, 18.78.

### Example 50

### 9-[2,3-Dideoxy-2,3-didehydro-4-β-phosphonomethoxy-β-D-erythrofuranosyl]adenine ammonium salt

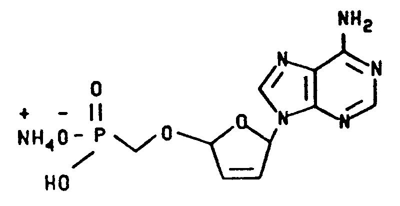

A solution of the product of Example 49 (310 mg, 0.9 mmol) and trimethylsilylbromide (1.0 ml) in DMF (4 ml) was stirred at 0°C for 3 h under nitrogen. Volatiles were removed in vacuo and the residue was dissolved in concentrated NH₄OH (2 ml). Water was evaporated in vacuo and the residual solid was purified by C₁₈ reverse-phase column using water as eluent under 0.56 bar (8 psi) pressure to give 7 (128 mg, 43%) as a white amorphous powder.
UV max (H₂O) 260 nm (ξ 14,982);
¹H NMR (D₂O) δ 3.59 (dd, J=9.3, 22.2 Hz, 1H), 3.69 (dd, J=9.3, 22.2 Hz, 1H), 5.99 (s, 1H), 6.46 (d, J=6.0 Hz, 1H), 6.50 (d, J=6.0 Hz, 1H), 6.83 (s, 1H), 7.87 (s, 1H), 8.13 (s, 1H);
¹³C NMR (D₂O) δ 70.756 (d, J-150 Hz), 93.065, 116.510, 122.434, 136.579, 139.835, 147.763, 155.424, 158.990, 161.809.
Anal. Calcd for C₁₀H₁₅N₆O₅P 3H₂O: C, 31.25; H, 5.46; N, 21.87.
Found: C, 31.32; H, 5.85; N, 22.15.

This compound was evaluated for anti-retroviral activity by the methods described in Examples 43 and 44. The following results were obtained. AZT controls were run simultaneously to confirm the validity of the test.

| Virus | Cell-line | Cell-tox. | ID₅₀ |
|---|---|---|---|
| HIV | CEM | >100um | 45um |
| MuLV | SC-1 | >100um | <0,1um |
| HIV | MT-4 | >500um | 1.5um |
| MuLV-R | SC-1 | >100um | 0.01um |

### Example 51

### 6-N-Pivaloyl-9-[2,3-dideoxy-4-β-(dimethylphosphono)methoxy -3-α-iodo-β-D-erythrofuranosyl]adenine

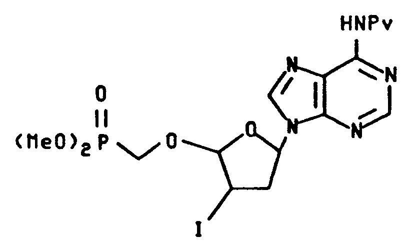

To a solution of the product of Example 45 (3.5 g, 12.2 mmol) and dimethyl hydroxymethylphosphonate (16.8 g, 120 mmol) in CH₂Cl₂ (40 ml) was added portionwise at 0°C N-iodosuccinimide (2.75 g, 12.2 mmol) and the mixture was stirred for 2 h at 0°C. The reaction was diluted with CH₂Cl₂ (50 ml), washed with water, brine, and dried over MgSO₄. The residual oil was chromatographed on silica gel using CH₂Cl₂-3% MeOH as eluent to give 8 (4.9 g, 72%) as a slightly yellow oil.
¹H NMR (CDCl₃) δ 1.36 (s, 9H), 2.89 (dd, J=6.3, 9.7 Hz, 1H), 3.20 (dd, J-6.3, 10.3 Hz, 1H), 3.7-3.9 (m, 8H), 4.49 (d, J-5.4 Hz, 1H), 5.50 (s, 1H), 6.86 (t, J=7.2 Hz, 1H), 8.29 (s, 1H), 8.50 (broad s, 1H), 8.74 (s, 1H).

### Example 52

### 6-N-Pivaloyl-9-[2,3-dideoxy-2-3-didehydro-4-β-(dimethylphosphono)methoxy-β-D-erythrofuranosyl]adenine

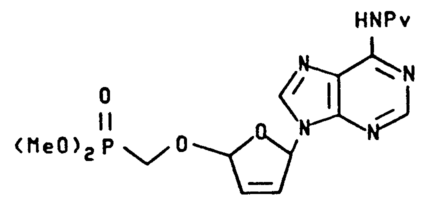

A solution of the product of Example 51 (1.7 g, 3.0 mmol) and 1,8-diazabicyclo [5,4,0] under-7-ene (912 mg, 6.0 mmol) in CHCl₃ (20 mL) was heated at 65°C for 2 h. The reaction was washed with ice-cold 20% H₃PO₄, brine, dried over MgSO₄ and evaporated in vacuo. The residual oil was purified on silica gel using CH₂Cl₂-3% MeOH to give 5 (1.2 g, 90%) as a colorless oil. This material was identical with the product of Example 48.

### Example 53

### 1-[2,3-Dideoxy-4-β-(phosphonomethoxy)-β-D-erythrofuranosyl]thymine disodium salt

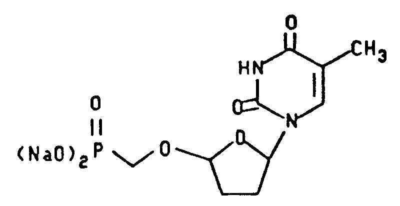

A mixture of the product of Example 25 (200 mg, 0.57 mmol) and 10% palladium on active carbon (180 mg) in water (20 ml) was hydrogenated for 30 min. under 2.13 bar (30 psi) H₂ pressure in the Parr hydrogenator. The catalyst was filtered through celite with the aid of a water wash. Water was removed by lyophilization to give the desired product (205 mg), 100%) as a white amorphous powder:
UV max (H₂O) 268 nm (ξ 8844);
¹H NMR (D₂O) δ 1.86 (s, 3H), 2.0-2.4 (m, 4H), 3.41 (dd, J=8.1, 12.9 Hz, 1H), 3.62 (dd, J=8.1, 12.9Hz, 1H), 5.32 (t, J-2.7Hz, 1H), 6.24 (t, J=7.0 Hz, 1H), 7.60 (s, 1H);
¹³C NMR (D₂O) δ 18.496, 35.384, 38.041, 72.628, (d, J=150 H₃), 93.249, 113.423, 118.884, 159.494, 174.169,
Anal. Calcd. for C₁₀H₁₃N₂O₇ PNa₂ 1 1/2 H₂O: C, 31.83; H, 4.24; N, 7.42.
Found: C, 31.62; H, 3.95; N, 7.28

### Example 54

### 6-N-Pivaloyl-9-[4-β-(dimethylphosphono)methoxy-β-D-erythrofuranosyl]adenine

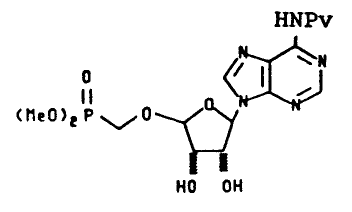

To a solution of phenylboric acid (860 mg, 7.0 mmol) and 4-methylmorpholine N-oxide (900 mg, 7.5 mmol) in CH₂Cl₂ (20 ml) was added at 23°C osmium tetroxide (25 mg) followed by the product of Example 52 (2.75 g, 6.4 mmol) in CH₂Cl₂ (10 ml). The mixture was stirred for 2h and 10% aqueous sodium bisulfite (4 ml) was added. After stirring for 1h, the CH₂Cl₂ was separated, washed with brine and dried over MgSO₄. Evaporation of solvent gave a white oil which was dissolved in acetone (15 ml) and 1,3-dipropanol (760 mg, 10 mmol). All volatiles were removed in vacuo and the resulting oil was chromatographed over silica gel using the above named compound CH₂Cl₂-7% MeOH as eluent to give (2.3 g, 76%) as a white foam:
¹H NMR (CDCl₃) δ 1.28 (s, 9H), 3.72 (d, J=10.5Hz, 6H), 3.73 (dd, J=10.6, 13.5Hz, 1H), 3.95 (d, J=10.6, 13.5 Hz, 1H) 4.91 (dd,J=4.5, 6.3 Hz, 1H), 4.33 (d, J=4.5 Hz, 1H), 5.09 (s, 1H), 6.38 (d, J=6.3Hz, 1H), 8.35 (s, 1H), 8.49 (s, 1H), 8.83 (broad s, 1H).

### Example 55

### 9-[4-β-(Methoxyhydroxyphosphinyl)methoxy-β-D-erythrofuranosyl]adenine ammonium salt

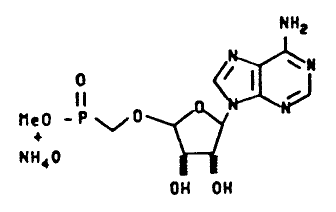

A solution of the product of Example 54 (2.2g, 4.8 mmol) and sodium methoxide (1.49, 26 mmol) in methanol (50 ml) was stirred at 23oC for 7 h under nitrogen. Volatiles were removed in vacuo and the residual oil was dissolved in water (20 ml). The aqueous solution was heated at 35°C for 10 h. The reaction was carefully neutralized to pH 5.0 by dropwise addition of 2N-HCl in an ice bath. The solution was then readjusted to 8.0 with concentrated NH₄OH and volatiles were removed in vacuo. The solid residue was purified by C₁₈ reverse-phase column using water -5% CH₃Cl as eluent under 0.56 bar (8 psi) pressure to give 11 (1.3g, 75%):
UV max (H₂O) 260 nm (ξ 10,019);
¹H NMR (D₂O) 3.56 (d, J-10.5 Hz, 3H), 3.61 (dd, J=10, 12.9 Hz, 1H), 3.83 (dd, J=10, 12.9 Hz, 1H), 4.38 (d, J-11.0 Hz, 1H), 5.0 (dd, J-6.2, 11.0 Hz, 1H), 5.19 (s, 1H);
¹³C NMR (D₂O) δ 53.897 (d, J-6.0 Hz), 64.192 (d, J-150 Hz), 75.589, 75,909, 111.096, 141,824, 151.079, 154,785, 157.354.
Anal. Calc. for CH₁₁H₁₄N₅O₇ p. 1 3/4 NaCl: C, 28.46; H, 3.47; N, 15.08.
Found: C, 28.59; H, 3.43; N, 14.72.

### Example 56

### 9-[4-β-(phosphonomethoxy)methoxy-β-D-erythrofuranosyl]adenine ammonium salt

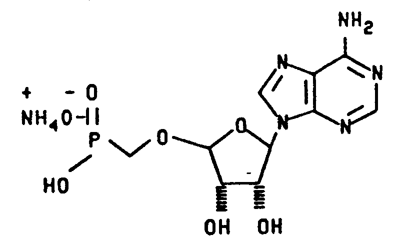

A solution of the product of Example 55 (1.5 g, 4.1 mmol) and trimethylsilyl bromide (7 ml) in DMF (30 ml) was stirred at 23°C for 6 h under nitrogen. The volatiles were removed in vacuo and the residue was dissolved in concentrated NH₄OH (3 ml). Water was evaporated in vacuo and the residual solid was purified by C₁₈ reverse-phase column using water as eluent to give 12 (600 mg, 40%) as a white amorphous powder:
UV max (H₂O) 262 nm ( ξ 12,640);
¹H NMR (D₂O) δ 3.56 (dd, J=10.0, 12.9 Hz, 1H), 3.79 (dd, J=10.0, 12.9Hz, 1H), 4.31 (d, J=11.0 Hz, 1H), 4.92 (dd, J=6.0, 11.0 Hz, 1H), 5.17 (s, 1H), 6.08 (d, J=0Hz, 1H), 8.24 (s, 1H), 8.25 (s, 1H).
¹³C NMR (D₂O) δ 66.296 (d, J=157 Hz), 75.881, 76.967, 88.982, 111.148, 119.927, 142.208, 150.689, 153,560, 156.330.
Anal. Calcd. for C₁₀H₁₇N₆O₇ P H₂O: C, 30.05; H, 5.26, N. 21.03.
Found: C, 30.09; H, 5.06; N, 20.38

## Claims (Claims for the following Contracting State(s): AT, BE, CH, DE, DK, FR, GB, GR, IE, IT, LI, LU, MC, NL, PT, SE)

1. A Phosphonomethoxymethoxymethyl purine or pyrimidine derivative of the formula I: 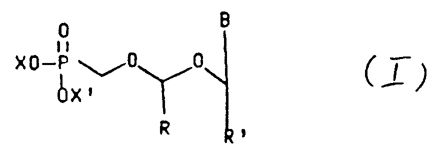 wherein
X and X' independently are hydrogen, alkyl having 1 to 6 carbon atoms, or the cation of a salt-forming base,
R and R' independently are hydrogen, alkyl with 1 to 6 carbon atoms, hydroxyalkyl with 1 to 6 carbon atoms or alkanoyl having 2 to 7 carbon atoms,
B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine,
and pharmaceutically acceptable salts thereof.

2. A phosphonomethoxymethoxymethyl purine or pyrimidine derivative according to claim 1, wherein X and X' independently are H, ethyl, methyl, or Na, and
R and R' independently are H or CH₃.

3. A purine derivative according to claim 2 wherein B is guanine or substituted guanine, and R', X and X' are H.

4. A phosphonomethoxymethoxymethyl purine or pyrimidine derivative according to claim 1, wherein said derivative is selected from the group consisting of (a) 9-[(phosphonomethoxy) methoxymethyl]guanine disodium salt, (b) 1-[(phosphonomethoxy) methoxymethyl]cytosine disodium salt, (c) 9-[(phosphonomethoxy)methoxymethyl] adenine disodium salt, (d) 9-[1-(phosphonomethoxy)ethoxy methyl] guanine disodium salt, (e)9-[(2-hydroxy-1-phosphonomethoxyethoxy)methyl]guanine disodium salt, (f) 9-[(2-hydroxy-1-phosphonomethoxyethoxy)methyl]adenine disodium salt, (g) 9-[(2-hydroxy-1-phosphonomethoxyethoxy)methyl]cytosine disodium salt and (h) 9-[(2-hydroxy-1-phosphonomethoxyethoxy)methyl]thymine disodium salt.

5. A dihydro-2-furyl or tetrahydro-2-furyl-1-substituted pyrimidine or 9-substituted purine derivative of the formula II: 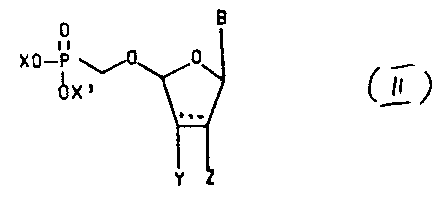 wherein
the broken line refers to an optional bond, X and X' independently are H, alkyl with 1 to 6 carbon atoms, or the cation of a salt-forming base,
Y and Z independently are H, OH, unsubstituted or substituted alkyl having 1 to 6 carbon atoms, and
Y and Z together are an oxygen atom or a methylene group in which event the broken line is absent,
B is a 9-substituted purine or 1-substituted pyrimidine base selected from the group consisting of xanthine, substituted xanthine, guanine, substituted guanine, purine, substituted purine, cytosine, substituted cytosine, thymine, uracil, substituted uracil, adenine and substituted adenine,
and pharmaceutically acceptable salts thereof.

6. A purine or pyrimidine derivative according to claims 5, wherein Y and Z independently are alkyl having 1 to 6 carbon atoms substituted by a substituent selected from the group consisting of halogen, hydroxy, amino, or azido.

7. A purine derivative according to claim 5 wherein Z and Y are H, the broken line is a bond, B is adenine or substituted adenine, and X and X' are independently H, alkyl with 1 to 6 carbon atoms, or the cation of a salt-forming base.

8. A purine or pyrimidine derivative according to claim 5, wherein said derivative is selected from the group consisting of
(a) 1-(5-phosphonometnoxytetrahydro-2-furyl)thymine disodium salt,
(b) 1-[2,3-dideoxy-2,3-didehydro-4-phosphonomethoxy-beta-D-erythrofuranosyl]thymine disodium salt,
(c) 1-[2,3-dideoxy-2,3-didehydro-4-beta-(methylphosphono)methoxy-beta-D-erythrofuranosyl]thymine sodium salt,
(d) 1-[2,3-dideoxy-4-beta-(methylphosphono)methoxy-beta-D-erythrofuranosyl]thymine sodium salt,
(e) 9-[2,3-dideoxy-2,3-didehydro-4-beta-(methylphosphono)methoxy-beta-D-erythrofuranosyl]adenine sodium salt,
(f) 9-[2,3-dideoxy-2,3-didehydro-4-beta-(methylphosphono)methoxy-beta-D-erythrofuranosyl]guanine sodium salt,
(g) 1-[2,3-dideoxy-2,3-didehydro-4-beta-(methylphosphono)methoxy-beta-D-erythrofuranosyl]cytosine sodium salt,
(h) 1-[4-beta-(dimethylphosphono)methoxy-beta-D-erythrofuranosyl]thymine,
(i) 9-[4-beta-(dimethylphosphono)methoxy-beta-D-erythrofuranosyl]adenine.
(j) 9-[4-beta-(dimethylphosphono)methoxy-beta-D-erythrofuranosyl]guanine.
(k) 1-[4-beta-(dimethylphosphono)methoxy-beta-D-erythrofuranosyl]cytosine,
(l) 1-[4-beta-(methylphosphono)methoxy-beta-D-erythrofuranosyl]thymine,
(m) 9-[4-beta-(methylphosphono)methoxy-beta-D-erythrofuranosyl]adenine,
(n) 9-[4-beta-(methylphosphono)methoxy-beta-D-erythrofuranosyl]guanine,
(o) 1-[4-beta-methylphosphono)methoxy-beta-D-erythrofuranosyl]cytosine,
(p) 1-[4-beta-phosphonomethoxy-beta-D-erythrofuranosyl)thymine disodium salt,
(q) 9-[4-beta-phosphonomethoxy-beta-D-erythrofuranosyl)adenine disodium salt,
(r) 1-[4-beta-phosphonomethoxy-beta-D-erythrofuranosyl)-cytosine disodium salt,
(s) 1-[4-beta-phosphonomethoxy-D-erythrofuranosyl)-cytosine disodium salt,
(t) 1-[2-deoxy-4-beta-(diethylphosphano)methoxy-beta-D-erythrofuranosyl]thymine,
(u) 9-[2-deoxy-4-beta-(diethylphosphono)methoxy-beta-D-erythrofuranosyl]adenine.
(v) 9-[2-deoxy-4-beta-(diethylphosphono)methoxy-beta-D-erythrofuranosyl]guanine,
(w) 1-[2-deoxy-4-beta-(diethylphosphono)methoxy-beta-D-erythrofuranosyl]cytosine,
(x) 1-(2-deoxy-4-beta-phosphonomethoxy-beta-D-erythrofuranosyl)thymine disodium salt,
(y) 9-(2-deoxy-4-beta-phosphonomethoxy-beta-D-erythrofuranosyl)adenine monoammonium salt,
(z) 9-(2-deoxy-4-beta-phosphonomethoxy-beta-D-erythrofuranosyl)guanine disodium salt,
(aa) 1-(2-deoxy-4-beta-phosphonomethoxy-beta-D-erythrofuranosyl)cytosine disodium salt,
(ab) 9-(2-deoxy-4-beta-(methylphosphono)methoxy-beta-D-erythrofuranosyl)adenine sodium salt, and
(ac) 9-[2,3-dideoxy-2,3-didehydro-4-β-phosphonomethoxy-β-D-erythrofuranosyl]adenine mono-ammonium salt.

9. A purine or pyrimidine derivative of the formula III: 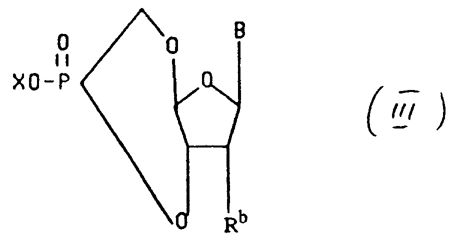 wherein
X is H, alkyl with 1 to 6 carbon atoms, R^{b} is H or OH and B is a base selected from the group consisting of xanthine, substituted xanthine, guanine, substituted guanine, purine, substituted purine, cytosine, substituted cytosine, uracil, substituted uracil, thymine, adenine and substituted adenine, and pharmaceutically acceptable salts thereof.

10. A phosphonomethoxymethoxymethyl purine/pyrimidine derivative having a cyclic phosphonate group of the formula IV: 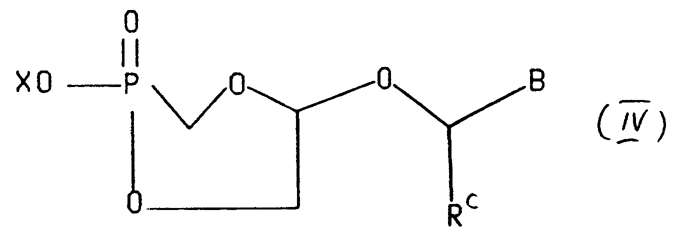 wherein
X is H, alkyl with 1 to 6 carbon atoms, R^{c} is H, alkyl with 1 to 6 carbon atoms, hydroxyalkyl with 1 to 6 carbon atoms, or alkanoyl having 2 to 7 carbon atoms, and B is a purine or pyrimidine base selected from the group consisting of xanthine, substituted xanthine, guanine, substituted cytosine, uracil, substituted uracil, thymine, adenine and substituted adenine, and pharmaceutically acceptable salts thereof.

11. A pharmaceutical composition comprising as an active ingredient an anti-viral or anti-retroviral or anti-neoplastic amount of at least one compound according to any one of claims 1 to 10 in admixture with a solid, liquid or gaseous diluent.

12. The use of at least one compound according to any one of claims 1 to 10 for preparing a pharmaceutical composition for treating an infection with a virus or retrovirus or for treating a tumor.

13. An oligonucleotide derived from a 5'-phosphonomethoxytetrahydrofuran-2'-yl purine/pyrimidine derivative according to any one of claims 5 to 8.

14. A compound of the formula V: 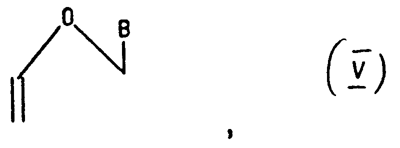 wherein
B is a purine or pyrimidine base selected from the group consisting of xanthine, substituted xanthine, guanine, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, cytosine, substituted cytosine, thymine, uracil, substituted uracil, adenine and substituted adenine.

15. A compound of the formula VI: 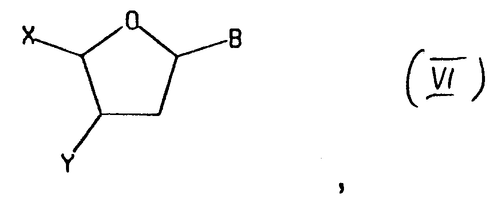 wherein
X is a halogen, Y is 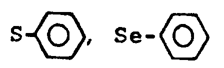 or a halogen and
B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

16. A compound of the formula VII: 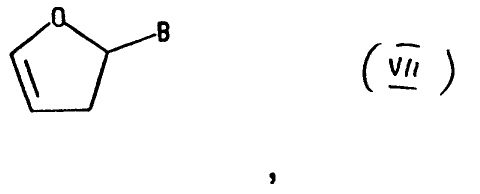 wherein
B is a purine or pyrimidine base selected from the group consisting of guanine, substituted guanine, cytosine, and substituted cytosine, 5-substituted uracil, for example, 5-chlorouracil, 5-bromouracil, 5-ethyluracil, 5-iodouracil, 5-propyluracil and 5-vinyluracii, 5-fluorouracil being excepted, and substituted adenine, for example, 3-deazaadenine.

17. A compound of the formula VIII: 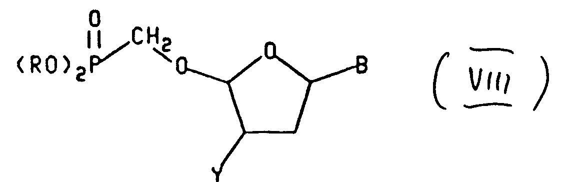 wherein
Y is a halogen, 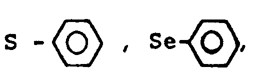 R is hydrogen or alkyl with 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

18. A process for preparing a compound of the formula 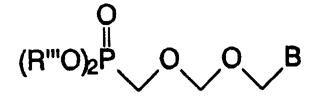 comprising reacting a compound of the formula 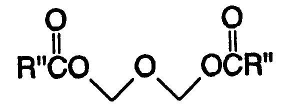 with a silylated purine or a silylated pyrimidine in the presence of a Lewis acid to form a compound of the formula 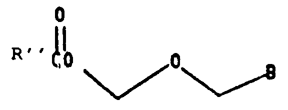 and reacting said 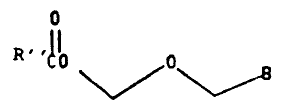 with a compound of the formula 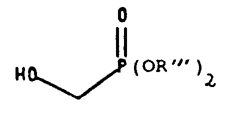 in the presence of a Lewis acid, wherein R'' is an alkyl having 1 to 6 carbon atoms or an unsubstituted or substituted aryl, R''' is hydrogen or alkyl with 1 to 6 carbon atoms and B is a pruine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

19. A process for preparing a compound of the formula 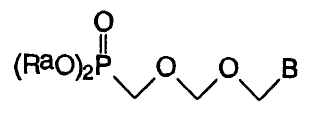 comprising reacting a compound of the formula 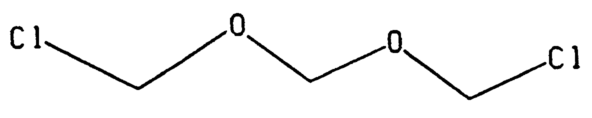 with a compound of the formula 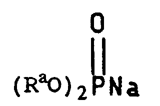 to form a compound of the formula 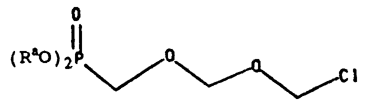 and reacting said 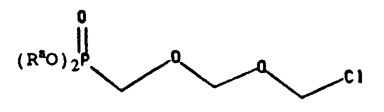 with a purine or pyrimidine base, wherein R^{a} is an alkyl having 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

20. A process for preparing a compound of the formula 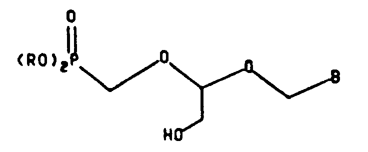 comprising reacting a compound of the formula 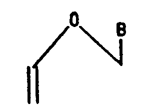 with a compound of the formula 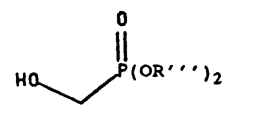 in the presence of a peracid, wherein R''' is hydrogen or alkyl with 1 to 6 carbon atoms and B is a purine or pyrimidine base selected from the group consisting of xanthine, substituted xanthine, guanine, substituted guanine, pruine, substituted purine, cytosine, substituted cytosine, thymine, uracil, substituted uracil, adenine and substituted adenine.

21. A process for preparing a compound of the formula 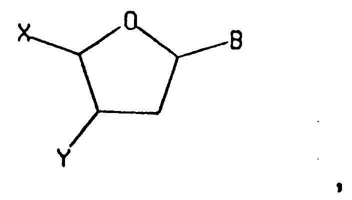 comprising reacting a compound 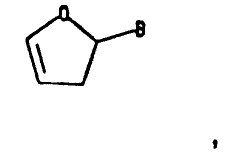 with X-Y, wherein
X is a halogen,
Y is 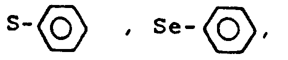 or a halogen, and
X-Y is a halogen, 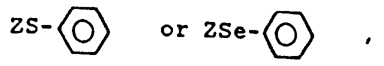 wherein Z is a halogen and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

22. A process for preparing a compound of the formula 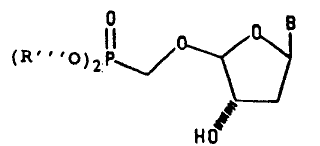 comprising reacting a compound of the formula 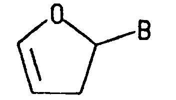 with a compound of the formula 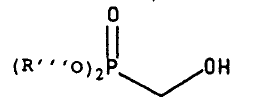 with a peracid, wherein R''' is hydrogen or alkyl with 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substitued uracil, adenine and substituted adenine.

23. A process for preparing the pharmaceutical composition of claim 11 which comprises incorporating at least one compound according to anyone of claims 1 to 10 into a solid, liquid or gaseous diluent.

## Claims (Claims for the following Contracting State(s): ES)

1. A process for preparing a phosphonomethoxymethoxymethyl purine or pyrimidine derivative of the formula I: 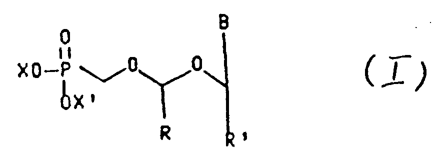 wherein
X and X' independently are hydrogen, alkyl having 1 to 6 carbon atoms, or the cation of a salt-forming base, R and R' independently are hydrogen, alkyl with 1 to 6 carbon atoms, hydroxyalkyl with 1 to 6 carbon atoms or alkanoyl having 2 to 7 carbon atoms, B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine, and pharmaceutically acceptable salts thereof,
comprising reacting a compound of the formula 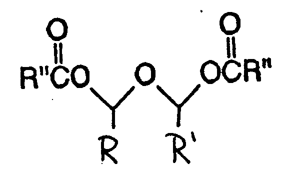 with a silylated purine or a silylated pyrimidine in the presence of a Lewis acid to form a compound of the formula 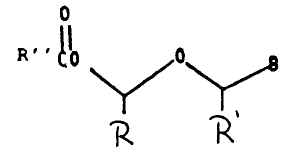 and reacting said 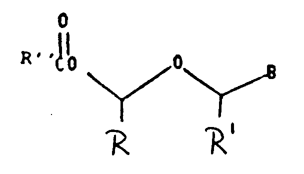 with a compound of the formula 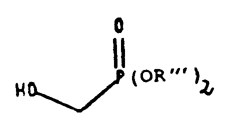 in the presence of a Lewis acid, wherein R'' is an alkyl having 1 to 6 carbon atoms or an unsubstituted or substituted aryl, R''' is hydrogen or alkyl with 1 to 6 carbon atoms and B, R and R' have the above meanings, optionally followed by introduction of the desired residue X and/or X'.

2. A process according to claim 1 for preparing a phosphonomethoxymethoxymethyl purine or pyrimidine derivative prepared in claim 1, wherein X and X' independently are H, ethyl, methyl, or Na, and R and R' independently are H or CH₃.

3. A process according to claim 2 for preparing a purine derivative as prepared in claim 2 wherein B is guanine or substituted guanine, and R', X and X' are H.

4. A process according to claim 1 for preparing a phosphonomethoxymethoxymethyl purine or pyrimidine derivative as prepared in claim 1, wherein said derivative is selected from the group consisting of (a) 9-[(phosphonomethoxy)methoxymethyl]guanine disodium salt, (b) 1-[(phosphonomethoxy)methoxymethyl]cytosine disodium salt, (c) 9-[(phosphonomethoxy)methoxymethyl]adenine disodium salt, (d) 9-[1-(phosphonomethoxy)ethoxymethyl]guanine disodium salt, (e)9-[(2-hydroxy-1-phosphonomethoxyethoxy)methyl]guanine disodium salt, (f) 9-[(2-hydroxy-1-phosphonomethoxyethoxy)methyl)adenine disodium salt, (g) 9-[(2-hydroxy-1-phosphonomethoxyethoxy methyl]cytosine disodium salt and (h) 9-[(2-hydroxy-1-phosphonomethoxyethoxy)methyl]thymine disodium salt.

5. A process for preparing any one of compounds as prepared in claims 1 to 4 comprising reacting a compound of the formula 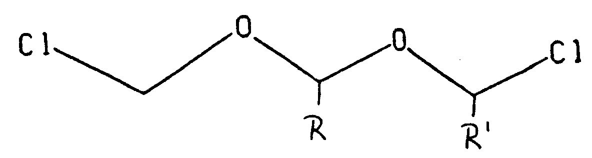 with a compound of the formula 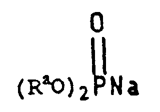 to form a compound of the formula 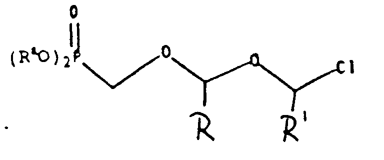 and reacting said 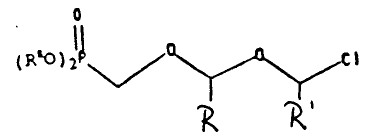 with a purine or pyrimidine base, wherein R and R' have the meanings defined in claim 1 to 4 and R^{a} is an alkyl having 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine, optionally followed by introduction of the desired residue X and/or X'.

6. A process for preparing a compound of the formula I 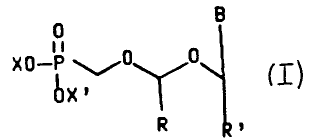 wherein
X and X' independently are hydrogen, alkyl having 1 to 6 carbon atoms, or the cation of a salt-forming base, R is CH₂OH, R' is hydrogen, and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine, and pharmaceutically acceptable salts thereof, comprising reacting a compound of the formula 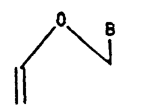 with a compound of the formula 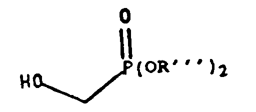 in the presence of a peracid, wherein R''' is hydrogen or alkyl with 1 to 6 carbon atoms and B has the above meanings, optionally followed by introduction of the desired residue X and/or X'.

7. A process for preparing a compound of the formula I 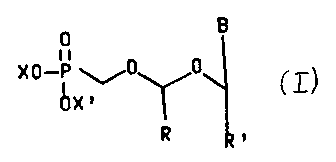 wherein
X and X' independently are hydrogen, alkyl having 1 to 6 carbon atoms, or the cation of a salt-forming base, R is CH₃, R' is hydrogen, and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine, and pharmaceutically acceptable salts thereof,
comprising reacting a compound of the formula 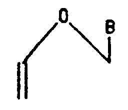 with a compound of the formula 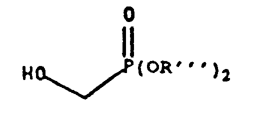 wherein
R''' is hydrogen or alkyl with 1 to 6 carbon atoms and B has the above meanings, optionally followed by introduction of the desired residue X and/or X'.

8. A process for preparing a dihydro-2-furyl or tetrahydro-2-furyl-1-substituted pyrimidine or 9-substituted purine derivative of the formula II: 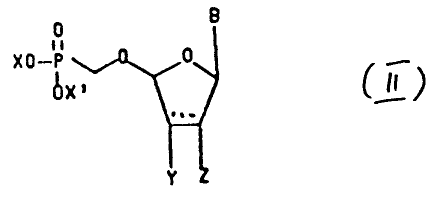 wherein
the broken line refers to an optional bond, X and X' undependently are H, alkyl with 1 to 6 carbon atoms, or the cation of a salt-forming base, Y and Z independently are H, OH, unsubstituted or substituted alkyl having 1 to 6 carbon atoms, and Y and Z together are an oxygen atom or a methylene group in which event the broken line is absent, B is a 9-substituted purine or 1-substituted pyrimidine base selected from the group consisting of xanthine, substituted xanthine, guanine, substituted substitued guanine, purine, substituted purine, cytosine, substituted cytosine, thymine, uracil, substituted uracil, adenine and substituted adenine, and pharmaceutically acceptable salts thereof,
comprising reacting a compound of the formulae 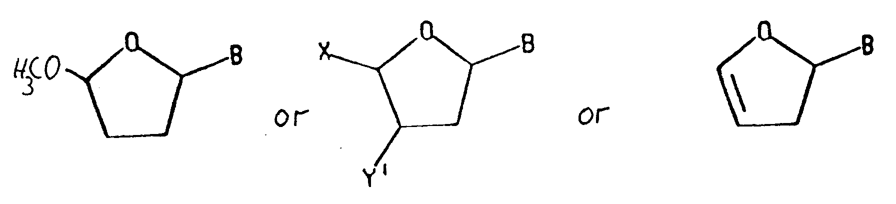 with a compound of the formula 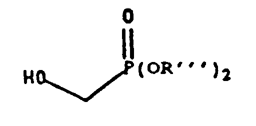 wherein
R''' is hydrogen or alkyl with 1 to 6 carbon atoms, X is halogen, Y' is S-phenyl, Se-phenyl, or halogen, and B has the above meanings, optionally followed by introduction of desired residues X, X', Y, and/or Z as well as a single or double bond as indicated by the broken line in above formula II.

9. A process according to claim 8 for preparing a purine or pyrimidine derivative as prepared in claim 8, wherein Y and Z independently are alkyl having 1 to 6 carbon atoms substituted by a substituent selected from the group consisting of halogen, hydroxy, amino, or azido.

10. A process according to claim 8 for preparing a purine derivative as prepared in claim 8 wherein Z and Y are H, the broken line is a bond, B is adenine or substituted adenine, and X and X' are independently H, alkyl with 1 to 6 carbon atoms, or the cation of a salt-forming base.

11. A process according to claim 8 for preparing a purine or pyrimidine derivative as prepared in claim 8, wherein said derivative is selected from the group consisting of
(a) 1-(5-phosphonomethoxytetrahydro-2-furyl)thymine disodium salt,
(b) 1-[2,3-dideoxy-2,3-didehydro-4-phosphonomethoxy-beta-D-erythrofuranosyl]thymine disodium salt,
(c) 1-[2,3-dideoxy-2,3-didehydro-4-beta-(methylphosphono)methoxy-beta-D-erythrofuranosyl]thymine sodium salt,
(d) 1-[2,3-dideoxy-4-beta-(methylphosphono)methoxy-beta-D-erythrofuranosyl]thymine sodium salt,
(e) 9-[2,3-dideoxy-2,3-didehydro-4-beta-(methylphosphono)methoxy-beta-D-erythrofuranosyl]adenine sodium salt,
(f) 9-[2,3-dideoxy-2,3-didehydro-4-beta-(methylphosphono)methoxy-beta-D-erythrofuranosyl]guanine sodium salt,
(g) 1-[2,3-dideoxy-2,3-didehydro-4-beta-(methylphosphono)methoxy-beta-D-erythrofuranosyl]cytosine sodium salt,
(h) 1-[4-beta-(dimethylphosphono)methoxy-beta-D-erythrofuranosyl]thymine,
(i) 9-[4-beta-(dimethylphosphono)methoxy-beta-D-erythrofuranosyl)adenine.
(j) 9-[4-beta-(dimethylphosphono)metrhoxy-beta-D-erythrofuranosyl]guanine,
(k) 1-[4-beta-(dimethylphosphono)methoxy-beta-D-erythrofuranosyl)cytosine,
(l) 1-[4-beta-(methylphosphono)methoxy-beta-D-erythrofuranosyl]thymine,
(m) 9-[4-beta-(methylphosphono)methoxy-beta-D-erythrofuranosyl]adenine,
(n) 9-[4-beta-(methylphosphono)methoxy-beta-D-erythrofuranosyl]guanine,
(o) 1-[4-beta-methylphosphono)methoxy-beta-D-erythrofuranosyl]cytosine,
(p) 1-[4-beta-phosphonomethoxy-beta-D-erythrofuranosyl)thymine disodium salt,
(q) 9-[4-beta-phosphonomethoxy-beta-D-erythrofuranosyl)adenine disodium salt,
(r) 1-[4-beta-phosphonomethoxy-beta-D-erythrofuranosyl)cytosine disodium salt,
(s) 1-[4-beta-phosphonomethoxy-D-erythrofuranosyl)cytosine disodium salt,
(t) 1-[2-deoxy-4-beta-(diethylphosphono)methoxy-beta-D-erythrofuranosyl]thymine,
(u) 9-[2-deoxy-4-beta-(diethylphosphono)methoxy-beta-D-erythrofuranosyl]adenine,
(v) 9-[2-deoxy-4-beta-(diethylphosphono)methoxy-beta-D-erythrofuranosyl]guanine,
(w) 1-[2-deoxy-4-beta-(diethylphosphono)methoxy-beta-D-erythrofuranosyl]cytosine,
(x) 1-(2-deoxy-4-beta-phosphonomethoxy-beta-D-erythrofuranosyl)thymine disodium salt,
(y) 9-(2-deoxy-4-beta-phosphonomethoxy-beta-D-erythrofuranosyl)adenine monoammonium salt,
(z) 9-(2-deoxy-4-beta-phosphonomethoxy-beta-D-erythrofuranosyl)guanine disodium salt,
(aa) 1-(2-deoxy-4-beta-phosphonomethoxy-beta-D-erythrofuranosyl)cytosine disodium salt,
(ab) 9-(2-deoxy-4-beta-(methylphosphono)methoxy-beta-D-erythrofuranosyl)adenine sodium salt, and
(ac) 9-[2,3-dideoxy-2,3-didehydro-4-β-phosphonomethoxy-β-D-erythrofuranosyl]adenine monoammonium salt.

12. A process according to claim 8 further comprising converting a compound of the formula 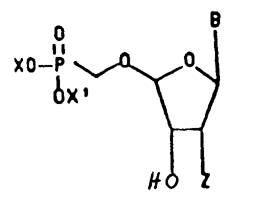 to a purine or pyrimidine derivative of the formula III: 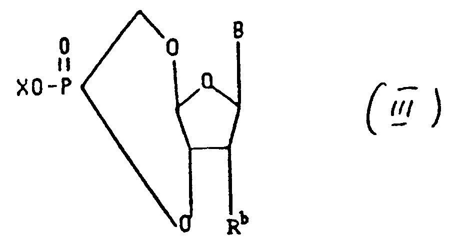 wherein
X and X' are H, alkyl with 1 to 6 carbon atoms or the cation of a salt-forming base, Z and R^{b} independently are H or OH, and B is a base selected from the group consisting of xanthine, substituted xanthine, guanine, substituted guanine, purine, substituted purine, cytosine, substituted cytosine, uracil, substituted uracil, thymine, adenine and substituted adenine, and pharmaceutically acceptable salts thereof.

13. A process according to claims 1 or 6 further comprising converting a compound of the formula 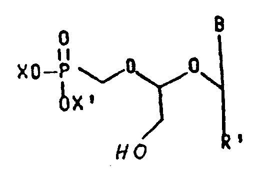 to a phosphonomethoxymethoxymethyl purine/pyrimidine derivative having a cyclic phoshponate group of the formula IV: 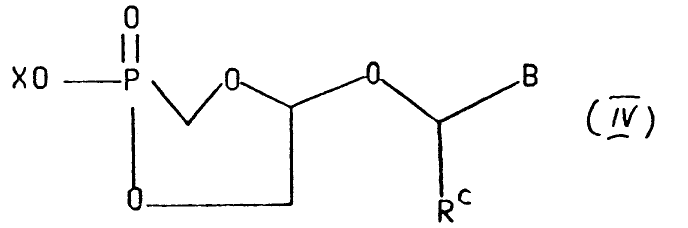 wherein
X is H, alkyl with 1 to 6 carbon atoms, or the cation of a salt-forming base, R' and R^{c} independently are H, alkyl with 1 to 6 carbon atoms, hydroxyalkyl with 1 to 6 carbon atoms, or alkanoyl having 2 to 7 carbon atoms, and B is a purine or pyrimidine base selected from the group consisting of xanthine, substituted xanthine, guanine, substituted cytosine, uracil, substituted uracil, thymine, adenine und substituted adenine, and pharmaceutically acceptable salts thereof.

14. The use of at least one compound prepared in any one of claims 1 to 13 for preparing a pharmaceutical composition for treating an infection with a virus or retrovirus or for treating a tumor.

15. A process for preparing an oligonucleotide derived from a 5'-phosphonomethoxytetrahydrofuranyl-2'-purine/pyrimidine derivative as prepared in any one of claims 8 to 11.

16. A process for preparing a compound of the formula V: 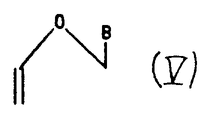 wherein
B is a purine or pyrimidine base selected from the group consisting of xanthine, substituted xanthine, guanine, 8-bromoguanine, 8-chloroguanine, 8-aminoguanine, 8-hydrazinoguanine, 8-hydroxyguanine, 8-methylguanine, 8-thioguanine and 3-deazaguanine, purine, substituted purine, cytosine, substituted cytosine, thymine, uracil, substituted uracil, adenine and substituted adenine,
comprising elimination of H-Y' from a compound of the formula 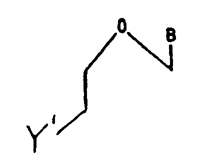 wherein
Y' is S-Ph, Se-Ph or a halogen and B has the above meanings.

17. A process for preparing a compound of the formula 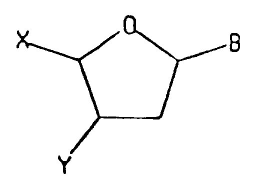 comprising reacting a compound 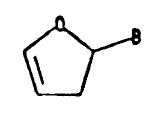 with X-Y, wherein
X is a halogen,
Y is S-Ph, Se-Ph, or a halogen, and
X-Y is a halogen, ZS-Ph or ZSe-Ph, wherein Z is a halogen and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

18. A process for preparing a compound of the formula VIII: 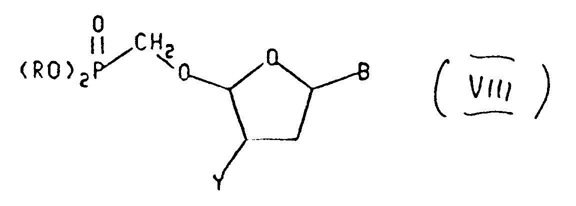 wherein
Y is a halogen, S-Ph, Se-Ph, R is hydrogen or alkyl with 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine,
comprising reacting a compound of the formula 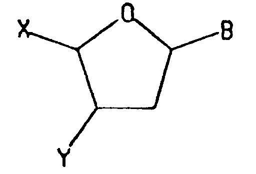 with a compound of the formula 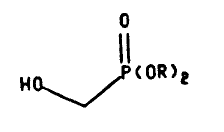 wherein
X is a halogen, and Y, R and B have the above meanings.

19. A process for preparing a compound of the formula 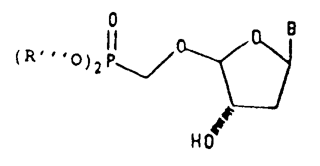 comprising reacting a compound of the formula 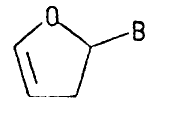 with a compound of the formula 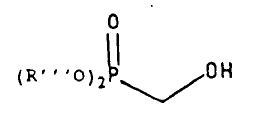 with a peracid, wherein R''' is hydrogen or alkyl with 1 to 6 carbon atoms and B is a purine or pyrimidine base selected from the group consisting of xanthine, substituted xanthine, guanine, substituted guanine, purine, substituted purine, cytosine, substituted cytosine, thymine, uracil, substituted uracil, adenine and substituted adenine.

20. A process for preparing a pharmaceutical composition which comprises incorporating at least one compound prepared in any one of claims 1 to 13 into a solid, liquid or gaseous diluent.

## Patentansprüche (Patentansprüche für folgende(n) Vertragsstaat(en): AT, BE, CH, DE, DK, FR, GB, GR, IE, IT, LI, LU, MC, NL, PT, SE)

1. Phosphonomethoxymethoxymethylpurin- oder -pyrimidinderivat der Formel I: 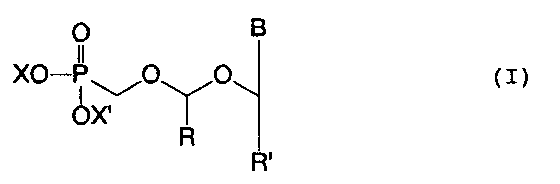 worin
X und X' unabhängig voneinander für Wasserstoff, Alkyl mit 1 bis 6 Kohlenstoffatomen oder das Kation einer salzbildenden Base stehen,
R und R' unabhängig voneinander für Wasserstoff, Alkyl mit 1 bis 6 Kohlenstoffatomen, Hydroxyalkyl mit 1 bis 6 Kohlenstoffatomen oder Alkanoyl mit 2 bis 7 Kohlenstoffatomen stehen,
B für eine Purin- oder Pyrimidinbase, ausgewählt unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, steht,
und pharmazeutisch verträgliche Salze davon.

2. Phosphonomethoxymethoxymethylpurin- oder -pyrimidinderivat nach Anspruch 1, worin X und X' unabhängig voneinander für H, Ethyl, Methyl oder Na stehen und R und R' unabhängig voneinander für H oder CH₃ stehen.

3. Purinderivat nach Anspruch 2, worin B für Guanin oder substituiertes Guanin steht und R', X und X' für H stehen.

4. Phosphonomethoxymethoxymethylpurin- oder -pyrimidinderivat nach Anspruch 1, wobei das Derivat ausgewählt ist unter (a) 9-[(Phosphonomethoxy)methoxymethyl]guanin-Dinatriumsalz, (b) 1-[(Phosphonomethoxy)methoxymethyl]cytosin-Dinatriumsalz, (c) 9-[(Phosphonomethoxy)methoxymethyl]adenin-Dinatriumsalz, (d) 9-(1-(Phosphonomethoxy)ethoxymethyl]guanidin-Dinatriumsalz, (e) 9-[(2-Hydroxy-1-phosphonomethoxyethoxy)methyl]guanin-Dinatriumsalz, (f) 9-[(2-Hydroxy-1-phosphonomethoxyethoxy)methyl]adenin-Dinatriumsalz, (g) 9-[(2-Hydroxy-1-phosphonomethoxyethoxy)methyl]cytosin-Dinatriumsalz und (h) 9-[(2-Hydroxy-1-phosphonomethoxyethoxy)methyl]thymin-Dinatriumsalz.

5. Dihydro-2-furyl- oder Tetrahydro-2-furyl-1-substituiertes Pyrimidin- oder 9-substituiertes Purinderivat der Formel II: 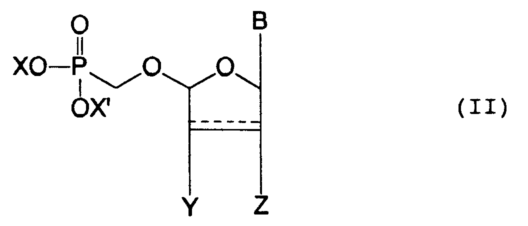 worin
die gestrichelte Linie für eine gegebenenfalls vorhandene Bindung steht, X und X' unabhängig voneinander für H, Alkyl mit 1 bis 6 Kohlenstoffatomen oder das Kation einer salzbildenden Base stehen,
Y und Z unabhängig voneinander für H, OH, gegebenenfalls substituiertes Alkyl mit 1 bis 6 Kohlenstoffatomen stehen und
Y und Z zusammen für ein Sauerstoffatom oder eine Methylengruppe stehen, wobei dann die gestrichelte Linie nicht vorhanden ist,
B für eine 9-substituierte Purin- oder eine 1-substituierte Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin,
und pharmazeutisch verträgliche Salze davon.

6. Purin- oder Pyrimidinderivat nach Anspruch 5, worin Y und Z unabhängig voneinander für Alkyl mit 1 bis 6 Kohlenstoffatomen stehen, das mit einem unter Halogen, Hydroxy, Amino oder Azido ausgewählten Substituenten substituiert ist.

7. Purinderivat nach Anspruch 5, worin Z und Y für H stehen, die gestrichelte Linie für eine Bindung steht, B für Adenin oder substituiertes Adenin steht, und X und X' unabhängig voneinander für H, Alkyl mit 1 bis 6 Kohlenstoffatomen, oder das Kation einer salzbildenden Base stehen.

8. Purin- oder Pyrimidinderivat nach Anspruch 5, wobei das Derivat ausgewählt ist unter
(a) 1-(5-Phosphonomethoxytetrahydro-2-furyl)thymin-Dinatriumsalz,
(b) 1-[2,3-Dideoxy-2,3-didehydro-4-phosphonomethoxy-β-D-erythrofuranosyl]thymin-Dinatriumsalz,
(c) 1-[2,3-Dideoxy-2,3-didehydro-4-β-(methylphosphono)methoxy-β-D-erythrofuranosyl]thymin-Natriumsalz,
(d) 1-[2,3-Dideoxy-4-β-(methylphosphono)methoxy-β-D-erythrofuranosyl]thymin-Natriumsalz,
(e) 9-[2,3-Dideoxy-2,3-didehydro-4-β-(methylphosphono)methoxy-β-D-erythrofuranosyl]adenin-Natriumsalz,
(f) 9-[2,3-Dideoxy-2,3-didehydro-4-β-(methylphosphono)methoxy-β-D-erythrofuranosyl]guanin-Natriumsalz,
(g) 1-[2,3-Dideoxy-2,3-didehydro-4-β-(methylphosphono)methoxy-β-D-erythrofuranosyl]cytosin-Natriumsalz,
(h) 1-[4-β-(Dimethylphosphono)methoxy-β-D-erythrofuranosyl]thymin,
(i) 9-[4-β-(Dimethylphosphono)methoxy-β-D-erythrofuranosyl]adenin,
(j) 9-[4-β-(Dimethylphosphono)methoxy-β-D-erythrofuranosyl]guanin,
(k) 1-[4-β-(Dimethylphosphono)methoxy-β-D-erythrofuranosyl]cytosin,
(l) 1-[4-β-(Methylphosphono)methoxy-β-D-erythrofuranosyl]thymin,
(m) 9-[4-β-(Methylphosphono)methoxy-β-D-erythrofuranosyl]adenin,
(n) 9-[4-β-(Methylphosphono)methoxy-β-D-erythrofuranosyl]guanin,
(o) 1-[4-β-(Methylphosphono)methoxy-β-D-erythrofuranosyl]cytosin,
(p) 1-[4-β-Phosphonomethoxy-β-D-erythrofuranosyl]-thymin-Dinatriumsalz,
(q) 9-[4-β-Phosphonomethoxy-β-D-erythrofuranosyl]adenin-Dinatriumsalz,
(r) 1-[4-β-Phosphonorethoxy-β-D-erythrofuranosyl]cytosin-Dinatriumsalz,
(s) 1-[4-β-Phosphonomethoxy-D-erythrofuranosyl]cytosin-Dinatriumsalz,
(t) 1-[2-Deoxy-4-β-(diethylphosphono)methoxy-β-D-erythrofuranosyl]thymin,
(u) 9-[2-Deoxy-4-β-(diethylphosphono)methoxy-β-D-erythrofuranosyl]adenin,
(v) 9-[2-Deoxy-4-β-(diethylphosphono)methoxy-β-D-erythrofuranosyl]guanin,
(w) 1-[2-Deoxy-4-β-(diethylphosphono)methoxy-β-D-erythrofuranosyl]cytosin,
(x) 1-(2-Deoxy-4-β-phosphonomethoxy-β-D-erythrofuranosyl)thymin-Dinatriumsalz,
(y) 9-(2-Deoxy-4-β-phosphonomethoxy-β-D-erythrofuranosyl)adenin-Monoammoniumsalz,
(z) 9-(2-Deoxy-4-β-phosphonomethoxy-β-D-erythrofuranosyl)guanin-Dinatriumsalz,
(aa) 1-(2-Deoxy-4-β-phosphonomethoxy-β-D-erythrofuranosyl)cytosin-Dinatriumsalz,
(ab) 9-(2-Deoxy-4-β-(methylphosphono)methoxy-β-D-erythrofuranosyl)adenin-Natriumsalz und
(ac) 9-[2,3-Dideoxy-2,3-didehydro-4-β-phssphonomethoxy-β-D-erythrofuranosyl]adenin-Monoammoniumsalz.

9. Purin- oder Pyrimidinderivat der Formel III: 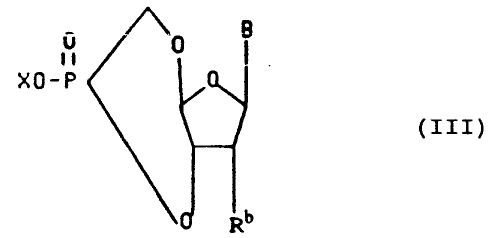 worin
X für H oder Alkyl mit 1 bis 6 Kohlenstoffatomen steht, R^{b} für H oder OH steht und B für eine Base steht, die ausgewählt ist unter Xanthin, substituiertem Xanthin, Guanin, substituiertem Guanin, Purin, substituiertem Purin, Cytosin, substituiertem Cytosin, Uracil, substituiertem Uracil, Thymin, Adenin und substituiertem Adenin, und pharmazeutisch verträgliche Salze davon.

10. Phosphonomethoxymethoxymethylpurin/pyrimidinderivat mit einer cyclischen Phosphonatgruppe der Formel IV: 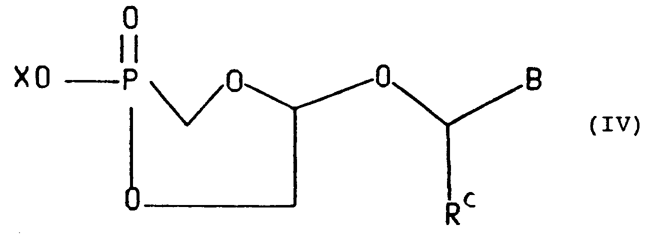 worin
X für H oder Alkyl mit 1 bis 6 Kohlenstoffatomen steht, R^{c} für H, Alkyl mit 1 bis 6 Kohlenstoffatomen, Hydroxyalkyl mit 1 bis 6 Kohlenstoffatomen oder Alkanoyl mit 2 bis 7 Kohlenstoffatomen steht und B für eine Purin- oder Pyrimidinbase steht, die ausgewählt ist unter Xanthin, substituiertem Xanthin, Guanin, substituiertem Cytosin, Uracil, substituiertem Uracil, Thymin, Adenin und substituiertem Adenin, und pharmazeutisch verträgliche Salze davon.

11. Pharmazeutisches Mittel, umfassend als aktiven Bestandteil eine antivirale oder antiretrovirale oder antineoplastische Menge wenigstens einer Verbindung nach einem der Ansprüche 1 bis 10 im Gemisch mit einem festen, flüssigen oder gasförmigen Verdünnungsmittel.

12. Verwendung wenigstens einer Verbindung nach einem der Ansprüche 1 bis 10 zur Herstellung eines pharmazeutischen Mittels zur Behandlung einer Infektion mit einem Virus oder Retrovirus oder zur Behandlung eines Tumors.

13. Oligonukleotid, abgeleitet aus einem 5'-Phosphonomethoxytetrahydrofuran-2'-yl-purin/pyrimidin-Derivat nach einem der Ansprüche 5 bis 8.

14. Verbindung der Formel V: 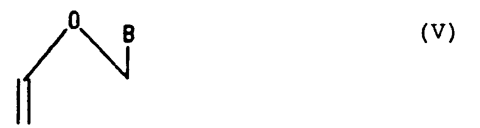 worin
B für eine Purin- oder Pyrimidinbase steht, die ausgewählt ist unter Xanthin, substituiertem Xanthin, Guanin, 8-Bromguanin, 8-Chlorguanin-, 8-Aminoguanin, 8-Hydrazinoguanin, 8-Hydroxyguanin, 8-Methylguanin, 8-Thioguanin und 3-Deazaguanin, Purin, substituiertem Purin, Cytosin, substituiertem Cytosin, Thymin, Uracil, substituiertem Uracil, Adenin und substituiertem Adenin.

15. Verbindung der Formel VI: 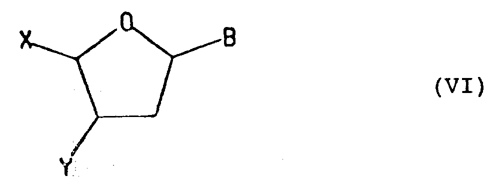 worin
X für ein Halogen, Y für 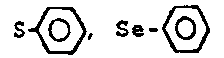 oder ein Halogen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin.

16. Verbindung der Formel VII: 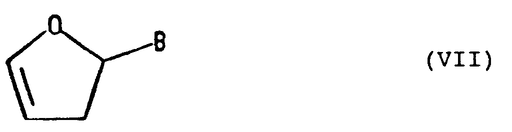 worin
B für eine Purin- oder Pyrimidinbase steht, die ausgewählt ist unter Guanin, substituiertem Guanin, Cytosin, substituiertem cytosin, 5-substituiertem Uracil, z. B. 5-Chloruracil, 5-Bromuracil, 5-Ethyluracil, 5-Ioduracil, 5-Propyluracil und 5-Vinyluracil, wobei 5-Fluoruracil ausgenommen ist, und substituiertem Adenin, z. B. 3-Deazaadenin.

17. Verbindung der Formel VIII: 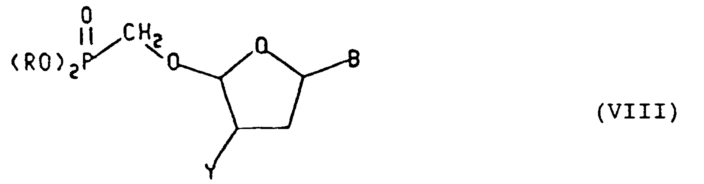 worin
Y für ein Halogen, 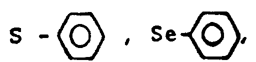 R für Wasserstoff oder Alkyl mit 1 bis 6 Kohlenstoffatomen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin.

18. Verfahren zur Herstellung einer Verbindung der Formel 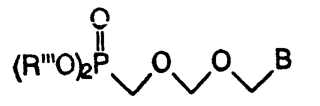 umfassend die Umsetzung einer Verbindung der Formel 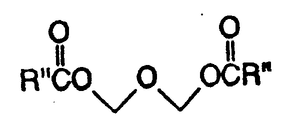 mit einem silylierten Purin oder einem silylierten Pyrimidin in Anwesenheit einer Lewissäure zu einer Verbindung der Formel 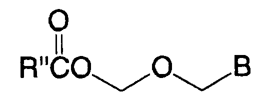 und die Umsetzung von 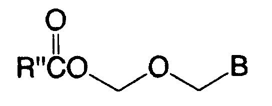 mit einer Verbindung der Formel 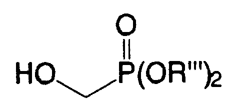 worin
R'' für eine Alkylgruppe mit 1 bis 6 Kohlenstoffatomen oder unsubstituiertes oder substituiertes Aryl steht, R''' für Wasserstoff oder Alkyl mit 1 bis 6 Kohlenstoffatomen steht und B für eine Purin-oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, in Gegenwart einer Lewissäure.

19. Verfahren zur Herstellung einer Verbindung der Formel 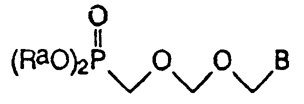 umfassend die Umsetzung einer Verbindung der Formel 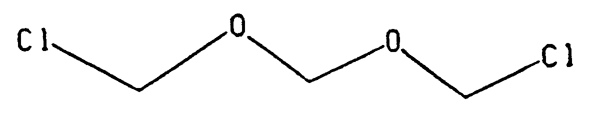 mit einer Verbindung der Formel 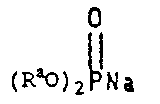 zu einer Verbindung der Formel 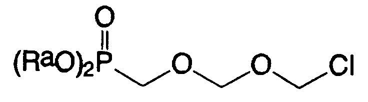 und die Umsetzung von 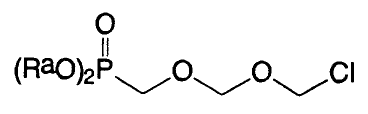 worin
R^{a} für eine Alkylgruppe mit 1 bis 6 Kohlenstoffatomen steht und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, mit einer Purin- oder Pyrimidinbase.

20. Verfahren zur Herstellung einer Verbindung der Formel 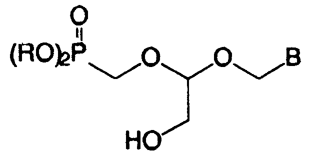 umfassend die Umsetzung einer Verbindung der Formel 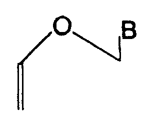 mit einer Verbindung der Formel 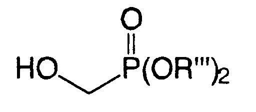 worin
R''' für Wasserstoff oder Alkyl mit 1 bis 6 Kohlenstoffatomen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, in Gegenwart einer Persäure.

21. Verfahren zur Herstellung einer Verbindung der Formel 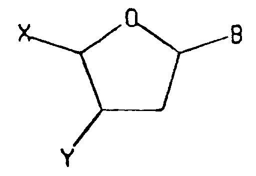 umfassend die Umsetzung einer Verbindung 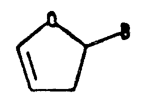 mit X-Y, worin
X für ein Halogen steht,
Y für 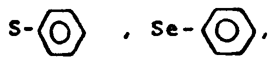 oder ein Halogen steht und
X-Y für ein Halogen, 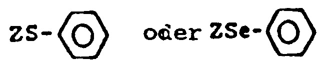 steht, wobei Z für ein Halogen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin.

22. Verfahren zur Herstellung einer Verbindung der Formel 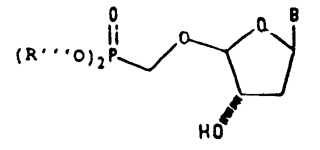 umfassend die Umsetzung einer Verbindung der Formel 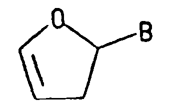 mit einer Verbindung der Formel 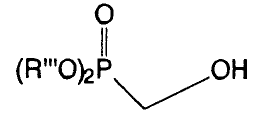 worin
R''' für Wasserstoff oder Alkyl mit 1 bis 6 Kohlenstoffatomen und B für eine Purin- oder Pyrimidinbase steht, die ausgewählt ist unter Xanthin, substituiertem Xanthin, Guanin, substituiertem Guanin, Purin, substituiertem Purin, cytosin, substituiertem Cytosin, Thymin, Uracil, substituiertem Uracil, Adenin und substituiertem Adenin, mit einer Persäure.

23. Verfahren zur Herstellung des pharmazeutischen Mittels nach Anspruch 11, umfassend die Aufnahme wenigstens einer Verbindung nach einem der Ansprüche 1 bis 10 in ein festes, flüssiges oder gasförmiges Verdünnungsmittel.

## Patentansprüche (Patentansprüche für folgende(n) Vertragsstaat(en): ES)

1. Verfahren zur Herstellung eines Phosphonomethoxymethoxymethylpurin- oder -pyrimidinderivates der Formel I: 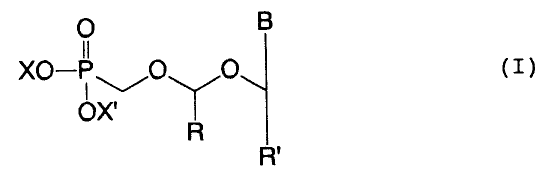 worin
X und X' unabhängig voneinander für Wasserstoff, Alkyl mit 1 bis 6 Kohlenstoffatomen oder das Kation einer salzbildenden Base stehen,
R und R' unabhängig voneinander für Wasserstoff, Alkyl mit 1 bis 6 Kohlenstoffatomen, Hydroxyalkyl mit 1 bis 6 Kohlenstoffatomen oder Alkanoyl mit 2 bis 7 Kohlenstoffatomen stehen, B für eine Purin- oder Pyrimidinbase, ausgewählt unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, steht,
und pharmazeutisch verträglicher Salze davon,
umfassend die Umsetzung einer Verbindung der Formel 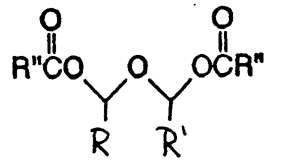 mit einem silylierten Purin oder einem silylierten Pyrimidin in Anwesenheit einer Lewissäure zu einer Verbindung der Formel 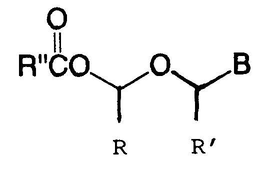 und die Umsetzung von 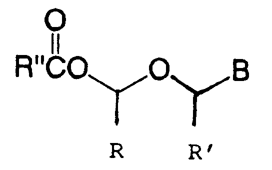 mit einer Verbindung der Formel 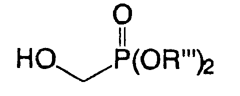 worin
R'' für eine Alkylgruppe mit 1 bis 6 Kohlenstoffatomen oder unsubstituiertes oder substituiertes Aryl steht, R''' für Wasserstoff oder Alkyl mit 1 bis 6 Kohlenstoffatomen steht und B, R und R' die obigen Bedeutungen besitzen, in Gegenwart einer Lewissäure, gegebenenfalls mit anschließender Einführung des gewünschten Restes X und/oder X'.

2. Verfahren nach Anspruch 1 zur Herstellung eines nach Anspruch 1 hergestellten Phosphonomethoxymethoxymethylpurin- oder -pyrimidinderivates, worin X und X' unabhängig voneinander für H, Ethyl, Methyl oder Na stehen und R und R' unabhängig voneinander für H oder CH₃ stehen.

3. Verfahren nach Anspruch 2 zur Herstellung eines nach Anspruch 2 hergestellten Purinderivates, worin B für Guanin oder substituiertes Guanin steht und R', X und X' für H stehen.

4. Verfahren nach Anspruch 1 zur Herstellung eines nach Anspruch 1 hergestellten Phosphonomethoxymethoxymethylpurin- oder -pyrimidinderivates, wobei das Derivat ausgewählt ist unter (a) 9-[(Phosphonomethoxy)methoxymethyl)guanin-Dinatriumsalz, (b) 1-[(Phosphonomethoxy)methoxymethyl]cytosin-Dinatriumsalz, (c) 9-[(Phosphonomethoxy)methoxymethyl]adenin-Dinatriumsalz, (d) 9-[1-(Phosphonomethoxy)ethoxymethyl]guanidin-Dinatriumsalz, (e) 9-[(2-Hydroxy-1-phosphonomethoxyethoxy)methyl]guanin-Dinatriumsalz, (f) 9-[(2-Hydroxy-1-phosphonomethoxyethoxy)methyl]adenin-Dinatriumsalz, (g) 9-[(2-Hydroxy-1-phosphonomethoxyethoxy)methyl]cytosin-Dinatriumsalz und (h) 9-[(2-Hydroxy-1-phosphonomethoxyethoxy)methyl]thymin-Dinatriumsalz.

5. Verfahren zur Herstellung einer der nach einem der Ansprüche 1 bis 4 hergestellten Verbindungen,
umfassend die Umsetzung einer Verbindung der Formel 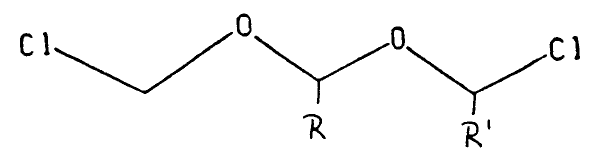 mit einer Verbindung der Formel 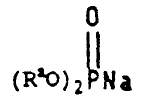 zu einer Verbindung der Formel 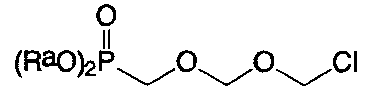 und die Umsetzung von 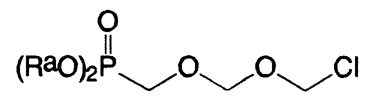 worin
R und R' die in Anspruch 1 bis 4 angegebenen Bedeutungen besitzen, R^{a} für eine Alkylgruppe mit 1 bis 6 Kohlenstoffatomen steht und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituierten Uracil, Adenin und substituiertem Adenin, mit einer Purin- oder Pyrimidinbase, gegebenenfalls mit anschließender Einführung des gewünschten Restes X und/oder X'.

6. Verfahren zur Herstellung einer Verbindung der Formel I: 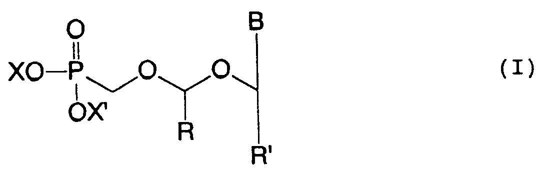 worin
X und X' unabhängig voneinander für Wasserstoff, Alkyl mit 1 bis 6 Kohlenstoffatomen oder das Kation einer salzbildenden Base stehen, R für CH₂OH steht, R' für Wasserstoff steht und B für eine Purin- oder Pyrimidinbase, ausgewählt unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, steht,
und pharmazeutisch verträglicher Salze davon,
umfassend die Umsetzung einer Verbindung der Formel 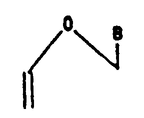 mit einer Verbindung der Formel 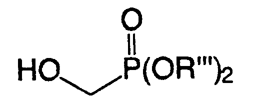 worin
R''' für Wasserstoff oder Alkyl mit 1 bis 6 Kohlenstoffatomen und B die obigen Bedeutungen besitzt, in Gegenwart einer Persäure, gegebenenfalls mit anschließender Einführung des gewünschten Restes X und/oder X'.

7. Verfahren zur Herstellung einer Verbindung der Formel I: 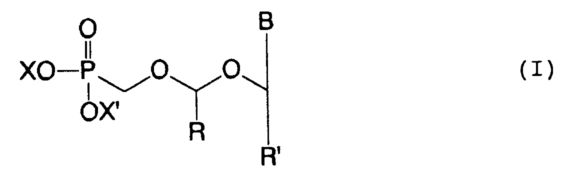 worin
X und X' unabhängig voneinander für Wasserstoff, Alkyl mit 1 bis 6 Kohlenstoffatomen oder das Kation einer salzbildenden Base stehen, R für CH₂OH steht, R' für Wasserstoff steht und B für eine Purin- oder Pyrimidinbase, ausgewählt unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, steht,
und pharmazeutisch verträglicher Salze davon,
umfassend die Umsetzung einer Verbindung der Formel 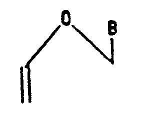 mit einer Verbindung der Formel 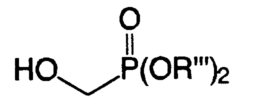 worin
R''' für Wasserstoff oder Alkyl mit 1 bis 6 Kohlenstoffatomen und B die obigen Bedeutungen besitzt, gegebenenfalls mit anschließender Einführung des gewünschten Restes X und/oder X'.

8. Verfahren zur Herstellung eines Dihydro-2-furyl- oder Tetrahydro-2-furyl-1-substituierten Pyrimidin- oder 9-substituierten Purinderivates der Formel II: 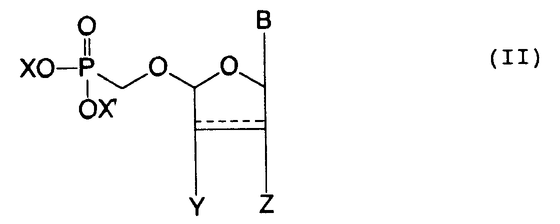 worin
die gestrichelte Linie für eine gegebenenfalls vorhandene Bindung steht, X und X' unabhängig voneinander für H, Alkyl mit 1 bis 6 Kohlenstoffatomen oder das Kation einer salzbildenden Base stehen, Y und Z unabhängig voneinander für H, OH, unsubstituiertes oder substituiertes Alkyl mit 1 bis 6 Kohlenstoffatomen stehen und Y und Z zusammen für ein Sauerstoffatom oder eine Methylengruppe stehen, wobei dann die gestrichelte Linie nicht vorhanden ist, B für eine 9-substituierte Purin- oder eine 1-substituierte Pyrimidinbase steht, die ausgewählt ist unter Xanthin, substituierter Xanthin, Guanin, substituiertem Guanin, Purin, substituiertem Purin, Cytosin, substituiertem Cytosin, Thymin, Uracil, substituiertem Uracil, Adenin und substituiertem Adenin, und pharmazeutisch verträglicher Salze davon,
umfassend die Umsetzung einer Verbindung der Formeln 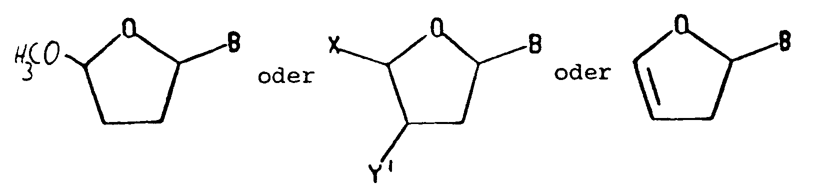 mit einer Verbindung der Formel 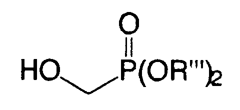 worin
R''' für Wasserstoff oder Alkyl mit 1 bis 6 Kohlenstoffatomen, X für Halogen, Y' für S-Phenyl, Se-Phenyl oder Halogen steht und B die obigen Bedeutungen besitzt, gegebenenfalls mit anschließender Einführung der gewünschten Reste X, X', Y und/oder Z sowie einer Einfach- oder Doppelbindung, die in obiger Formel II durch die gestrichelte Linie dargestellt ist.

9. Verfahren nach Anspruch 8 zur Herstellung eines nach Anspruch 8 hergestellten Purin- oder Pyrimidinderivates, worin Y und Z unabhängig voneinander für Alkyl mit 1 bis 6 Kohlenstoffatomen stehen, das mit einem unter Halogen, Hydroxy, Amino oder Azido ausgewählten Substituenten substituiert ist.

10. Verfahren nach Anspruch 8 zur Herstellung eines nach Anspruch 8 hergestellten Purinderivates, worin Z und Y für H stehen, die gestrichelte Linie für eine Bindung steht, B für Adenin oder substituiertes Adenin steht, und X und X' unabhängig voneinander für H, Alkyl mit 1 bis 6 Kohlenstoffatomen, oder das Kation einer salzbildenden Base stehen.

11. Verfahren nach Anspruch 8 zur Herstellung eines nach Anspruch 8 hergestellten Purin- oder Pyrimidinderivates, wobei das Derivat ausgewählt ist unter
(a) 1-(5-Phosphonomethoxytetrahydro-2-furyl)thymin-Dinatriumsalz,
(b) 1-[2,3-Dideoxy-2,3-didehydro-4-phosphonomethoxy-β-D-erythrofuranosyl]thymin-Dinatriumsalz,
(c) 1-[2,3-Dideoxy-2,3-didehydro-4-β-(methylphosphono)-methoxy-β-D-erythrofuranosyl]thymin-Natriumsalz,
(d) 1-[2,3-Dideoxy-4-β-(methylphosphono)methoxy-β-D-erythrofuranosyl]thymin-Natriumsalz,
(e) 9-[2,3-Dideoxy-2,3-didehydro-4-β-(methylphosphono)-methoxy-β-D-erythrofuranosyl]adenin-Natriumsalz,
(f) 9-[2,3-Dideoxy-2,3-didehydro-4-β-(methylphosphono)-methoxy-β-D-erythrofuranosyl]guanin-Natriumsalz,
(g) 1-[2,3-Dideoxy-2,3-didehydro-4-β-(methylphosphono)-methoxy-β-D-erythrofuranosyl]cytosin-Natriumsalz,
(h) 1-[4-β-(Dimethylphosphono)methoxy-β-D-erythrofuranosyl]thymin,
(i) 9-[4-β-(Dimethylphosphono)methoxy-β-D-erythrofuranosyl]adenin,
(j) 9-[4-β-(Dimethylphosphono)methoxy-β-D-erythrofuranosyl]guanin,
(k) 1-[4-β-(Dimethylphosphono)methoxy-β-D-erythrofuranosyl]cytosin,
(1) 1-[4-β-(Methylphosphono)methoxy-β-D-erythrofuranosyl]thymin,
(m) 9-[4-β-(Methylphosphono)methoxy-β-D-erythrofuranosyl]adenin,
(n) 9-[4-β-(Methylphosphono)methoxy-β-D-erythrofuranosyl]guanin,
(o) 1-[4-β-(Methylphosphono)methoxy-β-D-erythrofuranosyl]cytosin,
(p) 1-[4-β-Phosphonomethoxy-β-D-erythrofuranosyl]-thymin-Dinatriumsalz,
(q) 9-[4-β-Phosphonomethoxy-β-D-erythrofuranosyl]adenin-Dinatriumsalz,
(r) 1-[4-β-Phosphonomethoxy-β-D-erythrofuranosyl]cytosin-Dinatriumsalz,
(s) 1-[4-β-Phosphonomethoxy-D-erythrofuranosyl]cytosin-Dinatriumsalz,
(t) 1-[2-Deoxy-4-β-(diethylphosphono)methoxy-β-D-erythrofuranosyl]thymin,
(u) 9-[2-Deoxy-4-β-(diethylphosphono)methoxy-β-D-erythrofuranosyl]adenin,
(v) 9-[2-Deoxy-4-β-(diethylphosphono)methoxy-β-D-erythrofuranosyl]guanin,
(w) 1-[2-Deoxy-4-β-(diethylphosphono)methoxy-β-D-erythrofuranosyl]cytosin,
(x) 1-(2-Deoxy-4-β-phosphonomethoxy-β-D-erythrofuranosyl)thymin-Diratriumsalz,
(y) 9-(2-Deoxy-4-β-phosphonomethoxy-β-D-erythrofuranosyl)adenin-Monoammoniumsalz,
(z) 9-(2-Deoxy-4-β-phosphonomethoxy-β-D-erythrofuranosyl)guanin-Dinatriumsalz,
(aa) 1-(2-Deoxy-4-β-phosphonomethoxy-β-D-erythrofuranosyl)cytosin-Dinatriumsalz,
(ab) 9-(2-Deoxy-4-β-(methylphosphono)methoxy-β-D-erythrofuranosyl)adenin-Natriumsalz und
(ac) 9-[2,3-Dideoxy-2,3-didehydro-4-β-phosphonomethoxy-β-D-erythrofuranosyl]adenin-Monoammoniumsalz.

12. Verfahren nach Anspruch 8, zusätzlich umfassend die Umwandlung einer Verbindung der Formel 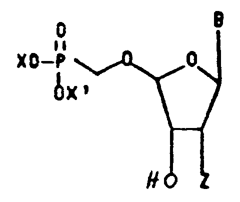 in ein Purin- oder Pyrimidinderivat der Formel III: 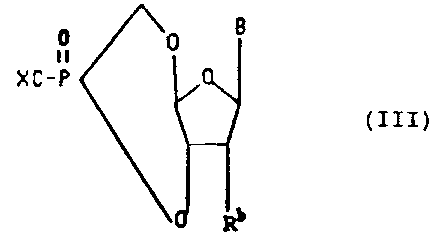 worin
X und X' für H, Alkyl mit 1 bis 6 Kohlenstoffatomen oder das Kation einer salzbildenden Base stehen, Z und R^{b} unabhängig voneinander für H oder OH stehen und B für eine Base steht, die ausgewählt ist unter Xanthin, substituiertem Xanthin, Guanin, substituiertem Guanin, Purin, substituiertem Purin, Cytosin, substituiertem Cytosin, Uracil, substituiertem Uracil, Thymin, Adenin und substituiertem Adenin, und pharmazeutisch verträgliche Salze davon.

13. Verfahren nach einem der Ansprüche 1 oder 6, zusätzlich umfassend die Umwandlung einer Verbindung der Formel 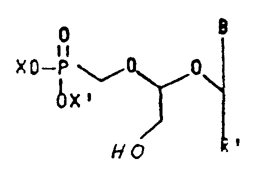 in ein Phosphonomethoxymethoxymethylpurin/pyrimidinderivat mit einer cyclischen Phosphonatgruppe der Formel IV: 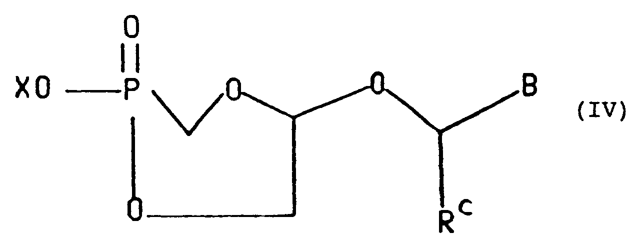 worin
X für H, Alkyl mit 1 bis 6 Kohlenstoffatomen steht, oder das Kation einer salzbildenden Base steht, R' und R^{c} unabhängig voneinander für H, Alkyl mit 1 bis 6 Kohlenstoffatomen, Hydroxyalkyl mit 1 bis 6 Kohlenstoffatomen oder Alkanoyl mit 2 bis 7 Kohlenstoffatomen stehen und B für eine Purin- oder Pyrimidinbase steht, die ausgewählt ist unter Xanthin, substituiertem Xanthin, Guanin, substituiertem Cytosin, Uracil, substituiertem Uracil, Thymin, Adenin und substituiertem Adenin, und pharmazeutisch verträgliche Salze davon.

14. Verwendung wenigstens einer Verbindung nach einem der Ansprüche 1 bis 13 zur Herstellung eines pharmazeutischen Mittels zur Behandlung einer Infektion mit einem Virus oder Retrovirus oder zur Behandlung eines Tumors.

15. Verfahren zur Herstellung eines aus einem 5'-Phosphonomethoxytetrahydrofuran-2'-yl-purin/pyrimidin-Derivat nach einem der Ansprüche 8 bis 11 abgeleiteten Oligonukleotides.

16. Verfahren zur Herstellung einer Verbindung der Formel V: 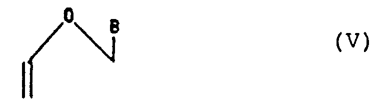 worin
B für eine Purin- oder Pyrimidinbase steht, die ausgewählt ist unter Xanthin, substituiertem Xanthin, Guanin, 8-Bromguanin, 8-Chlorguanin-, 8-Aminoguanin, 8-Hydrazinoguanin, 8-Hydroxyguanin, 8-Methylguanin, 8-Thioguanin und 3-Deazaguanin, Purin, substituiertem Purin, Cytosin, substituiertem Cytosin, Thymin, Uracil, substituiertem Uracil, Adenin und substituiertem Adenin,
umfassend die Eliminierung von H-Y' aus einer Verbindung der Formel 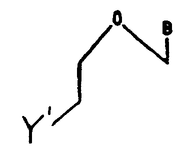 worin
Y' für S-Ph, Se-Ph oder ein Halogen steht und B die obigen Bedeutungen besitzt.

17. Verfahren zur Herstellung einer Verbindung der Formel: 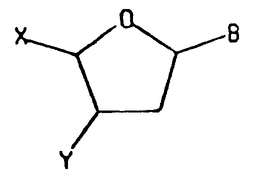 umfassend die Umsetzung einer Verbindung der Formel 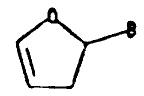 mit X-Y,
worin
X für ein Halogen,
Y für S-Ph, Se-Ph oder ein Halogen steht
und
X-Y für ein Halogen, ZS-Ph oder ZSe-Ph steht, worin Z für ein Halogen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin.

18. Verfahren zur Herstellung einer Verbindung der Formel VIII: 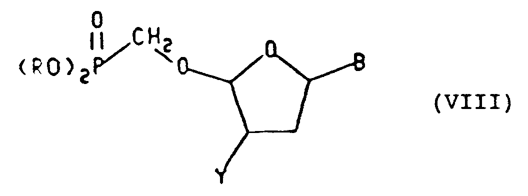 worin
Y für ein Halogen, S-Ph oder Se-Ph steht, R für Wasserstoff oder Alkyl mit 1 bis 6 Kohlenstoffatomen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin,
umfassend die Umsetzung einer Verbindung der Formel 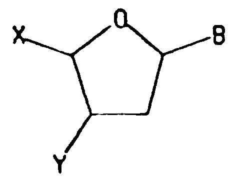 mit einer Verbindung der Formel 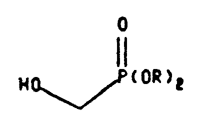 worin
X für Halogen steht und Y, R und B die obigen Bedeutungen besitzen.

19. Verfahren zur Herstellung einer Verbindung der Formel 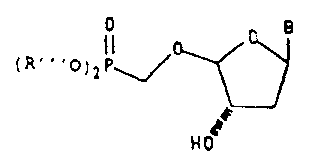 umfassend die Umsetzung einer Verbindung der Formel 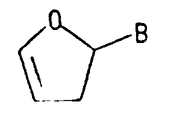 mit einer Verbindung der Formel 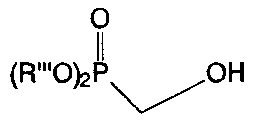 worin
R''' für Wasserstoff oder Alkyl mit 1 bis 6 Kohlenstoffatomen und B für eine Purin- oder Pyrimidinbase steht, die ausgewählt ist unter Xanthin, substituiertem Xanthin, Guanin, substituiertem Guanin, Purin, substituiertem Purin, Cytosin, substituiertem Cytosin, Thymin, Uracil, substituiertem Uracil, Adenin und substituiertem Adenin, mit einer Persäure.

20. Verfahren zur Herstellung eines pharmazeutischen Mittels, umfassend die Aufnahme wenigstens einer nach einem der Ansprüche 1 bis 13 hergestellten Verbindung in ein festes, flüssiges oder gasförmiges Verdünnungsmittel.

## Revendications (Revendications pour l'(les) Etat(s) contractant(s) suivant(s): AT, BE, CH, DE, DK, FR, GB, GR, IE, IT, LI, LU, MC, NL, PT, SE)

1. Dérivé de phosphonométhoxyméthoxyméthyl purine ou pyrimidine de la formule I : 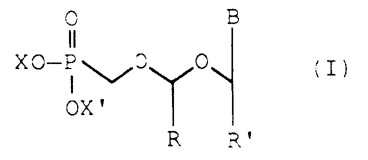 où
X et X' sont indépendamment hydrogène, alkyle ayant 1 à 6 atomes de carbone, ou bien le cation d'une base formant un sel,
R et R' sont indépendamment hydrogène, alkyle ayant 1 à 6 atomes de carbone, hydroxyalkyle ayant 1 à 6 atomes de carbone ou alcanoyle ayant 2 à 7 atomes de carbone,
B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée,
et leurs sels pharmaceutiquement acceptables.

2. Dérivé de phosphonométhoxyméthoxyméthyl purine ou pyrimidine selon la revendication 1, où X et X' sont indépendamment H, éthyle, méthyle, ou Na, et
R et R' sont indépendamment H ou CH₃.

3. Dérivé de purine selon la revendication 2 où B est guanine ou guanine substituée, et R', X et X' sont H.

4. Dérivé de phosphonométhoxyméthoxyméthyl purine ou pyrimidine selon la revendication 1, où ledit dérivé est sélectionné dans le groupe consistant en (a) sel disodique de 9-[(phosphonométhoxy)méthoxyméthyl]guanine, (b) sel disodique de 1-[(phosphonométhoxy)méthoxyméthyl]cytosine, (c) sel disodique de 9-[(phosphonométhoxy)méthoxyméthyl]adénine, (d) sel disodique de 9-[1-(phosphonométhoxy)éthoxy méthyl]guanine, (e) sel disodique de 9-[(2-hydroxy-1-phosphonométhoxyéthoxy)méthyl]guanine, (f) sel disodique de 9-[(2-hydroxy-1-phosphonométhoxyéthoxy)méthyl]adénine, (g) sel disodique de 9-[(2-hydroxy-1-phosphonométhoxyéthoxy)méthyl]cytosine et (h) sel disodique de 9-[(2-hydroxy-1-phosphonométhoxyéthoxy)méthyl]thymine.

5. Dérivé de pyrimidine dihydro-2-furyle ou tétrahydro-2-furyl-1-substituée ou de purine 9-substituée de la formule II : 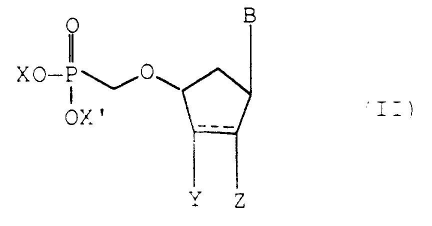 où
la ligne interrompue se rapporte à une liaison facultative, X et X' sont indépendamment H, alkyle avec 1 à 6 atomes de carbone ou bien le cation d'une base formant un sel,
Y et Z sont indépendamment H, OH, alkyle non substitué ou substitué ayant 1 à 6 atomes de carbone, et
Y et Z sont ensemble un atome d'oxygène ou un groupe méthylène auquel cas la ligne interrompue est absente,
B est une base de purine 9-substituée ou de pyrimidine 1-substituée sélectionnée dans le groupe consistant en xanthine, xanthine substituée, guanine, guanine substituée, purine, purine substituée, cytosine, cytosine substituée, thymine, uracile, uracile substitué, adénine et adénine substituée,
et leurs sels pharmaceutiquement acceptables.

6. Dérivé de purine ou de pyrimidine selon la revendication 5, où Y et Z sont indépendamment alkyle ayant 1 à 6 atomes de carbone, substitué par un substituant sélectionné dans le groupe consistant en halogène, hydroxy, amino, ou azido.

7. Dérivé de purine selon la revendication 5 où Z et Y sont H, la ligne interrompue est une liaison, B est adénine ou adénine substituée, et X et X' sont indépendamment H, alkyle avec 1 à 6 atomes de carbone, ou bien le cation d'une base formant un sel.

8. Dérivé de purine ou de pyrimidine selon la revendication 5 où ledit dérivé est sélectionné dans le groupe consistant en :
(a) sel disodique de 1-(5-phosphonométhoxytétrahydro-2-furyl)thymine,
(b) sel disodique de 1-[2,3-didésoxy-2,3-didéshydro-4-phosphonométhoxy-bêta-D-érythrofuranosyl] thymine,
(c) sel de sodium de 1-[2,3-didésoxy-2,3-didéshydro-4-bêta-(méthylphosphono)-méthoxy-bêta-D-érythrofuranosyl]thymine,
(d) sel de sodium de 1-[2,3-didésoxy-4-bêta(méthylphosphono)méthoxy-bêta-D-érythrofuranosyl]thymine,
(e) sel de sodium de 9-[2,3-didésoxy-2,3-didéshydro-4-béta-(méthylphosphono)-méthoxy-bêta-D-érythrofuranosyl]adénine,
(f) sel de sodium de 9-[2,3-didésoxy-2,3-didéshydro-4-bêta-(méthylphosphono)-méthoxy-bêta-D-érythrofuranosyl]guanine,
(g) sel de sodium de 1-[2,3-didésoxy-2,3-didéshydro-4-bêta-(méthylphosphono)-méthoxy-bêta-D-érythrofuranosyl]cytosine,
(h) 1-[4-bêta-(diméthylphosphono)méthoxy-bêta-D-érythrofuranosyl]thymine,
(i) 9-[4-bêta-(diméthylphosphono)méthoxy-bêta-D-érythrofuranosyl]adénine.
(j) 9-[4-bêta-(diméthylphosphono)méthoxy-bêta-D-érythrofuranosyl]guanine.
(k) 1-[4-bêta-(diméthylphosphono)méthoxy-bêta-D-érythrofuranosyl]cytosine,
(l) 1-[4-bêta-(méthylphosphono)méthoxy-bêta-D-érythrofuranosyl]thymine,
(m) 9-[4-bêta-(méthylphosphono)méthoxy-bêta-D-érythrofuranosyl]adénine,
(n) 9-[4-bêta-(méthylphosphono)méthoxy-bêta-D-érythrofuranosyl]guanine,
(o) 1-[4-bêta-(méthylphosphono)méthoxy-bêta-D-érythrofuranosyl]cytosine,
(p) sel disodique de 1-[4-bêta-phosphonométhoxy-bêta-D-érythrofuranosyl]thymine,
(q) sel disodique de 9-[4-bêta-phosphonométhoxy-bêta-D-érythrofuranosyl]adénine,
(r) sel disodique de 1-[4-bêta-phosphonométhoxy-bêta-D-érythrofuranosyl]cytosine,
(s) sel disodique de 1-[4-bêta-phosphonométhoxy-D-érythrofuranosyl]cytosine,
(t) 1-[2-désoxy-4-bêta-(diéthylphosphono)méthoxy-bêta-D-érythrofuranosyl]thymine,
(u) 9-[2-désoxy-4-bêta-(diéthylphosphono)méthoxy-bêta-D-érythrofuranosyl]adénine.
(v) 9-[2-désoxy-4-bêta-(diéthylphosphono)méthoxy-bêta-D-érythrofuranosyl]guanine.
(w) 1-9-[2-désoxy-4-bêta-(diéthylphosphono) méthoxy-bêta-D-érythrofuranosyl]cytosine.
(x) sel disodique de 1-(2-désoxy-4-bêta-phosphonométhoxy-bêta-D-érythrofuranosyl]thymine,
(y) sel de monoammonium de 9-(2-désoxy-4-bêta-phosphonométhoxy-bêta-D-érythrofuranosyl]adénine,
(z) sel disodique de 9-(2-désoxy-4-bêta-phosphonométhoxy-bêta-D-érythrofuranosyl]guanine,
(aa) sel disodique de 1-(2-désoxy-4-bêta-phosphonométhoxy-bêta-D-érythrofuranosyl]cytosine,
(ab) sel de sodium de 9-(2-désoxy-4-bêta-(méthylphosphono)méthoxy-bêta-D-érythrofuranosyl)adénine, et
(ac) sel de monoammonium de 9-[2,3-didésoxy-2,3-didéshydro-4-bêta-phosphono-méthoxy-bêta-D-érythrofuranosyl]adénine.

9. Dérivé de purine ou de pyrimidine de la formule III : 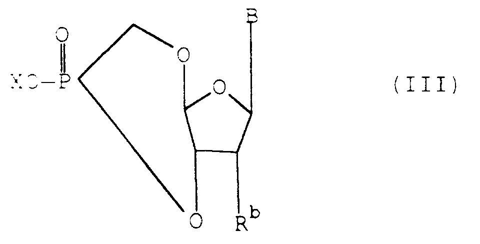 où
X est H, alkyle avec 1 à 6 atomes de carbone, R^{b} est H ou OH et B est une base sélectionnée dans le groupe consistant en xanthine, xanthine substituée, guanine, guanine substituée, purine, purine substituée, cytosine, cytosine substituée, uracile, uracile substitué, thymine, adénine et adénine substituée, et leurs sels pharmaceutiquement acceptables.

10. Dérivé de phosphonométhoxyméthoxyméthyl purine/pyrimidine ayant un groupe phosphonate cyclique de la formule IV : 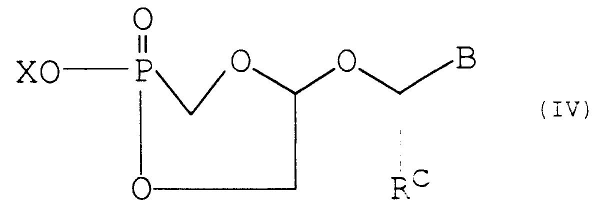 où
X est H, alkyle avec 1 à 6 atomes de carbone, R^{c} est H, alkyle avec 1 à 6 atomes de carbone, hydroxyalkyle avec 1 à 6 atomes de carbone, ou alcanoyle ayant 2 à 7 atomes de carbone, et B est une base de purine ou de pyrimidine sélectionnée dans le groupe consistant en xanthine, xanthine substituée, guanine, cytosine substituée, uracile, uracile substitué, thymine, adénine et adénine substituée, et leurs sels pharmaceutiquement acceptables.

11. Composition pharmaceutique comprenant comme ingrédient actif une quantité anti-virale ou anti-rétrovirale ou anti-néoplastique d'au moins un composé selon l'une quelconque des revendications 1 à 10 en mélange avec un diluant solide, liquide ou gazeux.

12. Utilisation d'au moins un composé selon l'une quelconque des revendications 1 à 10 pour la préparation d'une composition pharmaceutique pour le traitement d'une infection par un virus ou un rétrovirus ou pour le traitement d'une tumeur.

13. Oligonucléotide provenant d'un dérivé de 5'-phosphonométhoxytétrahydrofuran-2'-yl-purine/pyrimidine selon l'une quelconque des revendications 5 à 8.

14. Composé de la formule V : 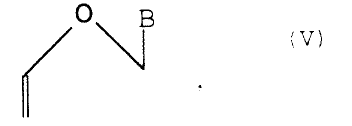 où
B est une base de purine ou de pyrimidine sélectionnée dans le groupe consistant en xanthine, xanthine substituée, guanine, 8-bromoguanine, 8-chloroguanine, 8-aminoguanine, 8-hydrazinoguanine, 8-hydroxyguanine, 8-méthylguanine, 8-thioguanine et 3-désazaguanine, purine, purine substituée, cytosine, cytosine substituée, thymine, uracile, uracile substitué, adénine et adénine substituée.

15. Composé de la formule VI : 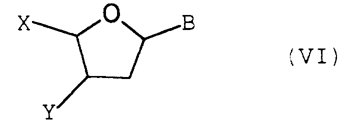 où
X est un halogène, Y est 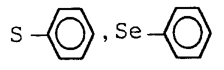 ou un halogène et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

16. Composé de la formule VII : 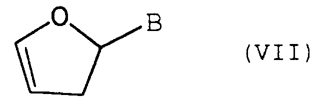 où
B est une base de purine ou de pyrimidine sélectionnée dans le groupe consistant en guanine, guanine substituée, cytosine, et cytosine substituée, 5-uracile substitué, par exemple 5-chlorouracile, 5-bromouracile, 5-éthyluracile, 5-iodouracile, 5-propyluracile et 5-vinyluracile, 5-fluorouracile étant excepté, et adénine substituée, par exemple, 3-déazaadénine.

17. Composé de la formule VIII : 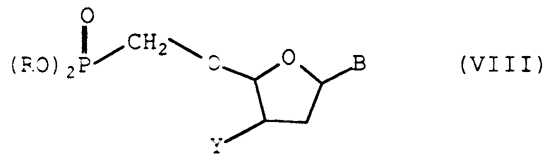 où
Y est un halogène, 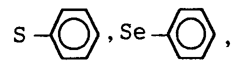 R est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

18. Procédé de préparation d'un composé de la formule 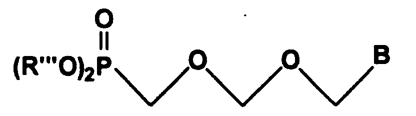 comprenant la réaction d'un composé de la formule 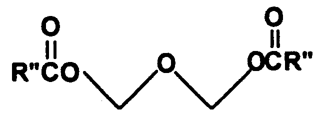 avec une purine silylée ou une pyrimidine silylée en présence d'un acide de Lewis pour former un composé de la formule 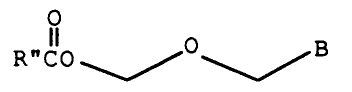 et réaction dudit 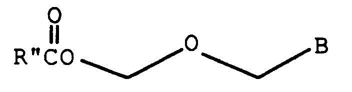 avec un composé de la formule 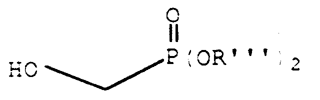 en présence d'un acide de Lewis, où R'' est un alkyle ayant 1 à 6 atomes de carbone ou un aryle non substitué ou substitué, R''' est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

19. Procédé de préparation d'un composé de la formule 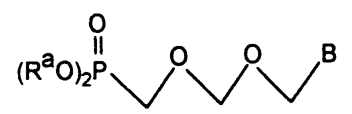 comprenant la réaction d'un composé de la formule 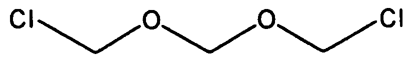 avec un composé de la formule 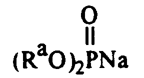 pour former un composé de la formule 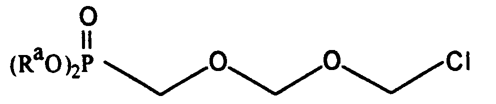 et réaction dudit 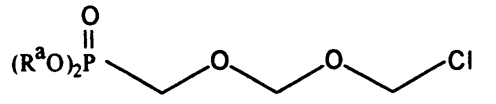 avec une base de purine ou de pyrimidine, où R^{a} est un alkyle ayant 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

20. Procédé de préparation d'un composé de formule 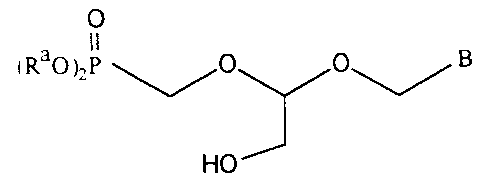 comprenant la réaction d'un composé de la formule 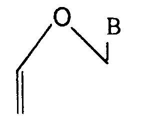 avec un composé de la formule 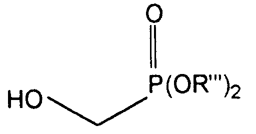 en présence d'un péracide, où R''' est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

21. Procédé de préparation d'un composé de la formule 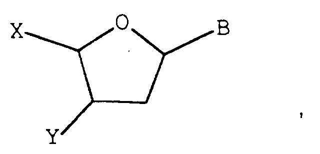 comprenant la réaction d'un composé 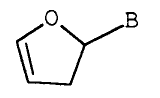 avec X-Y, où
X est un halogène,
Y est 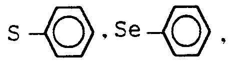 ou un halogène,
et
X-Y est un halogène, 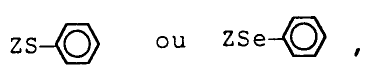 où Z est un halogène et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

22. Procédé de préparation d'un composé de formule 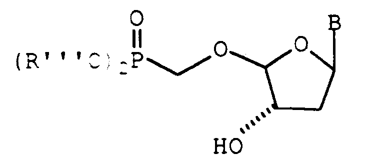 comprenant la réaction d'un composé de formule 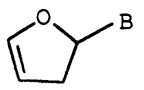 avec un composé de formule 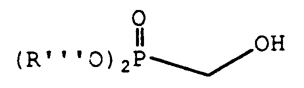 avec un péracide, où R''' est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

23. Procédé de préparation de la composition pharmaceutique de la revendication 11 qui comprend l'incorporation d'au moins un composé selon l'une quelconque des revendications 1 à 10 dans un diluant solide, liquide ou gazeux.

## Revendications (Revendications pour l'(les) Etat(s) contractant(s) suivant(s): ES)

1. Procédé de préparation d'un dérivé de phosphonométhoxyméthoxyméthyl purine ou pyrimidine de la formule I : 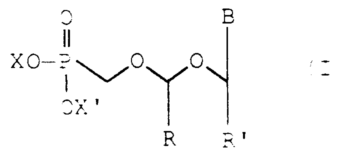 où
X et X' sont indépendamment hydrogène, alkyle ayant 1 à 6 atomes de carbone, ou bien le cation d'une base formant un sel, R et R' sont indépendamment hydrogène, alkyle ayant 1 à 6 atomes de carbone, hydroxyalkyle ayant 1 à 6 atomes de carbone ou alcanoyle ayant 2 à 7 atomes de carbone, B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée, et leurs sels pharmaceutiquement acceptables,
comprenant la réaction d'un composé de la formule 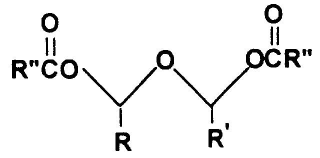 avec une purine silylée ou une pyrimidine silylée en présence d'un acide de Lewis pour former un composé de la formule 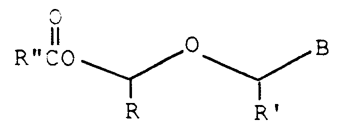 et la réaction dudit 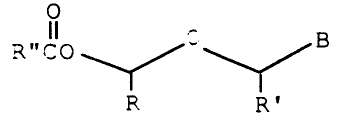 avec un composé de la formule 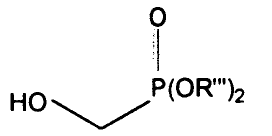 en présence d'un acide de Lewis, où R'' est un alkyle ayant 1 à 6 atomes de carbone ou un aryle non substitué ou substitué, R''' est hydrogène ou alkyle avec 1 à 6 atomes de carbone et B, R et R' ont les significations ci-dessus, avec ensuite facultativement l'introduction du résidu X et/ou X' souhaité.

2. Procédé selon la revendication 1 pour la préparation d'un dérivé de phosphonométhoxyméthoxyméthyl purine ou pyrimidine préparé à la revendication 1, où X et X' sont indépendamment H, éthyle, méthyle, ou Na, et R et R' sont indépendamment H ou CH₃.

3. Procédé selon la revendication 2 pour la préparation d'un dérivé de purine tel que préparé à la revendication 2 où B est guanine ou guanine substituée, et R', X et X' sont H.

4. Procédé selon la revendication 1 pour la préparation d'un dérivé de phosphonométhoxyméthoxyméthyl purine ou pyrimidine tel que préparé à la revendication 1, où ledit dérivé est sélectionné dans le groupe consistant en (a) sel disodique de 9-[(phosphonométhoxy) méthoxyméthyl]guanine, (b) sel disodique de 1-[(phosphonométhoxy)méthoxyméthyl]cytosine, (c) sel disodique de 9-[(phosphonométhoxy)méthoxyméthyl]adénine, (d) sel disodique de 9-[1-(phosphonométhoxy)éthoxyméthyl]guanine, (e) sel disodique de 9-[(2-hydroxy-1-phosphonométhoxyéthoxy)méthyl]guanine, (f) sel disodique de 9-[(2-hydroxy-1-phosphonométhoxyéthoxy)méthyl]adénine, (g) sel disodique de 9-[(2-hydroxy-1-phosphonométhoxyéthoxy)méthyl]cytosine et (h) sel disodique de 9-[(2-hydroxy-1-phosphonométhoxyéthoxy)méthyl]thymine.

5. Procédé de préparation de l'un des composés tel que préparé aux revendications 1 à 4 comprenant la réaction d'un composé de la formule 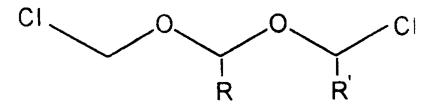 avec un composé de la formule 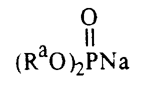 pour former un composé de la formule 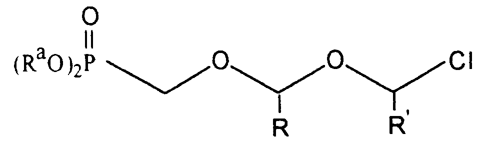 et réaction dudit 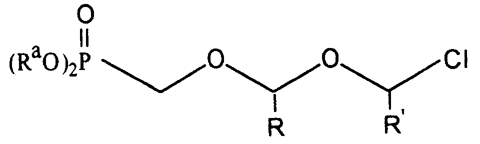 avec une base de purine ou de pyrimidine, où R et R' ont les significations définies à la revendication 1 à 4 et R^{a} est un alkyle ayant 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée, facultativement avec ensuite introduction du résidu X et/ou X' souhaité.

6. Procédé de préparation d'un composé de la formule I 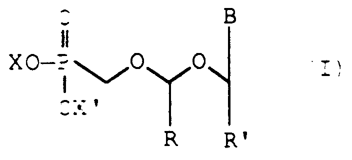 où
X et X' sont indépendamment hydrogène, alkyle ayant 1 à 6 atomes de carbone, ou bien le cation d'une base formant un sel, R est CH₂OH, R' est hydrogène, et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée, et leurs sels pharmaceutiquement acceptables, comprenant la réaction d'un composé de la formule 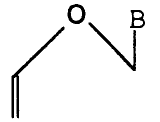 avec un composé de la formule 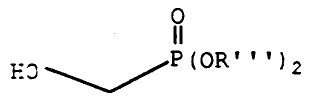 en présence d'un péracide, où R''' est hydrogène ou alkyle avec 1 à 6 atomes de carbone et B a les significations ci-dessus avec ensuite facultativement introduction du résidu X et/ou X' souhaité.

7. Procédé de préparation d'un composé de formule I 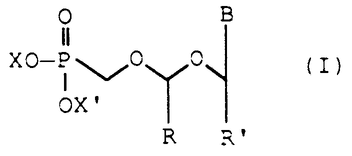 où
X et X' sont indépendamment hydrogène, alkyle ayant 1 à 6 atomes de carbone, ou bien le cation d'une base formant un sel, R est CH₃, R' est hydrogène, et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée, et leurs sels pharmaceutiquement acceptables,
comprenant la réaction d'un composé de la formule 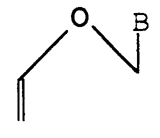 avec un composé de la formule 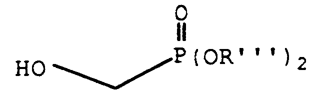 où
R''' est hydrogène ou alkyle avec 1 à 6 atomes de carbone et B a la signification ci-dessus, avec ensuite facultativement l'introduction du résidu X et/ou X' souhaité.

8. Procédé de préparation d'un dérivé de pyrimidine dihydro-2-furyle ou tétrahydro-2-furyl-1-substitué ou purine 9-substitué formule II : 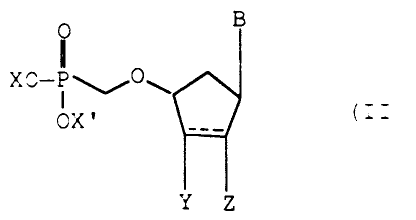 où
la ligne interrompue indique une liaison facultative, X et X' sont indépendamment H, alkyle avec 1 à 6 atomes de carbone ou bien le cation d'une base formant un sel, Y et Z sont indépendamment H, OH, alkyle non substitué ou substitué ayant 1 à 6 atomes de carbone, et Y et Z sont ensemble un atome d'oxygène ou un groupe méthylène auquel cas la ligne interrompue est absente, B est une base de purine 9-substituée ou de pyrimidine 1-substituée sélectionnée dans le groupe consistant en xanthine, xanthine substituée, guanine substituée, purine, purine substituée, cytosine, cytosine substituée, thymine, uracile, uracile substitué, adénine et adénine
substituée, et leurs sels pharmaceutiquement acceptables, comprenant la réaction d'un composé des formules 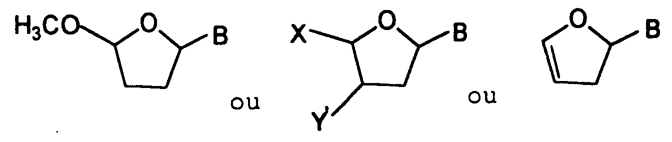 avec un composé de la formule 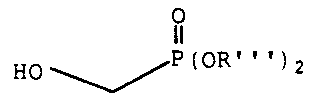 où
R''' est hydrogène ou alkyle avec 1 à 6 atomes de carbone, X est halogène, Y' est S-phényle, Se-phényle, ou halogène, et B a les significations ci-dessus, avec ensuite facultativement l'introduction des résidus souhaités X, X', Y, et/ou Z ainsi qu'une simple ou double liaison comme indiqué par la ligne interrompue dans la formule II ci-dessus.

9. Procédé selon la revendication 8 pour la préparation d'un dérivé de purine ou de pyrimidine tel que préparé à la revendication 8, où Y et Z sont indépendamment alkyle ayant 1 à 6 atomes de carbone substitué par un substituant sélectionné dans le groupe consistant en halogène, hydroxy, amino, ou azido.

10. Procédé selon la revendication 8 pour la préparation d'un dérivé de purine tel que préparé à la revendication 8 où Z et Y sont H, la ligne interrompue est une liaison, B est adénine ou adénine substituée, et X et X' sont indépendamment H, alkyle avec 1 à 6 atomes de carbone, ou bien le cation d'une base formant un sel.

11. Procédé selon la revendication 8 pour la préparation d'un dérivé de purine ou de pyrimidine tel que préparé à la revendication 8, où ledit dérivé est sélectionné dans le groupe consistant en
(a) sel disodique de 1-(5-phosphonométhoxytétrahydro-2-furyl)thymine,
(b) sel disodique de 1-[2,3-didésoxy-2,3-didéshydro-4-phosphonométhoxy-bêta-D-érythrofuranosyl] thymine,
(c) sel de sodium de 1-[2,3-didésoxy-2,3-didéshydro-4-bêta-(méthylphosphono)-méthoxy-bêta-D-érythrofuranosyl]thymine,
(d) sel de sodium de 1-[2,3-didésoxy-4-bêta(méthylphosphono)méthoxy-bêta-D-érythrofuranosyl] thymine,
(e) sel de sodium de 9-[2,3-didésoxy-2,3-didéshydro-4-bêta-(méthylphosphono)-méthoxy-bêta-D-érythrofuranosyl]adénine,
(f) sel de sodium de 9-[2,3-didésoxy-2,3-didéshydro-4-bêta-(méthylphosphono)-méthoxy-bêta-D-érythrofuranosyl]guanine,
(g) sel de sodium de 1-[2,3-didésoxy-2,3-didéshydro-4-bêta-(méthylphosphono)-méthoxy-bêta-D-érythrofuranosyl]cytosine,
(h) 1-[4-bêta-(diméthylphosphono)méthoxy-bêta-D-érythrofuranosyl]thymine,
(i) 9-[4-bêta-(diméthylphosphono)méthoxy-bêta-D-érythrofuranosyl]adénine,
(j) 9-[4-bêta-(diméthylphosphono)méthoxy-bêta-D-érythrofuranosyl]guanine.
(k) 1-[4-bêta-(diméthylphosphono)méthoxy-bêta-D-érythrofuranosyl]cytosine,
(l) 1-[4-bêta-(diméthylphosphono)méthoxy-bêta-D-érythrofuranosyl]thymine,
(m) 9-[4-bêta-(méthylphosphono)méthoxy-bêta-D-érythrofuranosyl]adénine,
(n) 9-[4-bêta-(méthylphosphono)méthoxy-bêta-D-érythrofuranosyl]guanine,
(o) 1-[4-béta-(méthylphosphono)méthoxy-bêta-D-érythrofuranosyl]cytosine,
(p) sel disodique de 1-[4-bêta-phosphonométhoxy-bêta-D-érythrofuranosyl]thymine,
(q) sel disodique de 9-[4-bêta-phosphonométhoxy-bêta-D-érythrofuranosyl]adénine,
(r) sel disodique de 1-[4-bêta-phosphonométhoxy-bêta-D-érythrofuranosyl]cytosine,
(s) sel disodique de 1-[4-bêta-phosphonométhoxy-D-érythrofuranosyl)cytosine,
(t) 1-[2-désoxy-4-bêta-(diéthylphosphono)méthoxy-bêta-D-érythrofuranosyl]thymine,
(u) 9-[2-désoxy-4-bêta-(diéthylphosphono)méthoxy-bêta-D-érythrofuranosyl]adénine.
(v) 9-[2-désoxy-4-bêta-(diéthylphosphono)méthoxy-bêta-D-érythrofuranosyl]guanine,
(w) 1-9-[2-désoxy-4-béta-(diéthylphosphano)méthoxy-bêta-D-érythrofuranosyl]cytosine,
(x) sel disodique de 1-(2-désoxy-4-bêta-phosphonométhoxy-bêta-D-érythrofuranosyl]thymine,
(y) sel de monoammonium de 9-(2-désoxy-4-bêta-phosphonométhoxy-bêta-D-érythrofuranosyl]adénine,
(z) sel disodique de 9-(2-désoxy-4-bêta-phosphonométhoxy-bêta-D-érythrofuranosyl]guanine,
(aa) sel disodique de 1-(2-désoxy-4-bêta-phosphonométhoxy-bêta-D-érythrofuranosyl]cytosine,
(ab) sel de sodium de 9-(2-désoxy-4-bêta-(méthylphosphono)méthoxy-bêta-D-érythrofuranosyl)adénine, et
(ac) sel de monoammonium de 9-[2,3-didésoxy-2,3-didéshydro-4-bêta-phosphono-méthoxy-bêta-D-érythrofuranosyl]adénine.

12. Procédé selon la revendication 8 comprenant de plus la conversion d'un composé de la formule 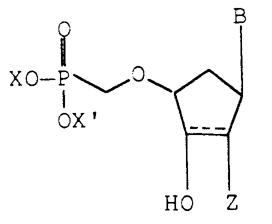 en un dérivé de purine ou de pyrimidine de la formule III: 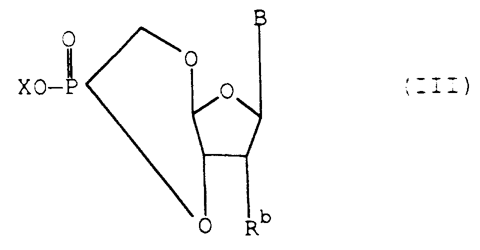 où
X et X' sont H, alkyle avec 1 à 6 atomes de carbone ou le cation d'une base formant un sel, Z et R^{b} sont indépendamment H ou OH, et B est une base sélectionnée dans le groupe consistant en xanthine, xanthine substituée, guanine, guanine substituée, purine, purine substituée, cytosine, cytosine substituée, uracile, uracile substitué, thymine, adénine et adénine substituée, et leurs sels pharmaceutiquement acceptables.

13. Procédé selon es revendications 1 ou 6 comprenant de plus la conversion d'un composé de la formule 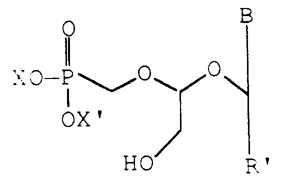 en un dérivé de phosphonométhoxyméthoxyméthyl
purine/pyrimidine ayant un groupe phosphonate cyclique de la formule IV : 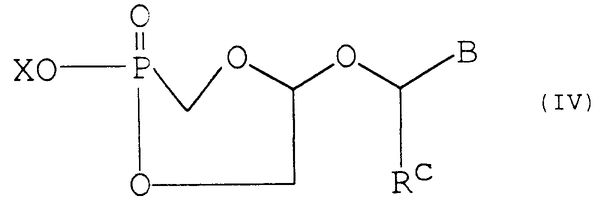 où
X est H, alkyle avec 1 à 6 atomes de carbone, ou bien le cation d'une base formant un sel, R' et R^{c} sont indépendamment H, alkyle avec 1 à 6 atomes de carbone, hydroxyalkyle avec 1 à 6 atomes de carbone, ou alcanoyle ayant 2 à 7 atomes de carbone, et B est une base de purine ou de pyrimidine sélectionnée dans le groupe consistant en xanthine, xanthine substituée, guanine, cytosine substituée, uracile, uracile substitué, thymine, adénine et adénine substituée, et leurs sels pharmaceutiquement acceptables.

14. Utilisation d'au moins un composé préparé selon l'une quelconque des revendications 1 à 13 pour la préparation d'une composition pharmaceutique pour le traitement d'une infection par un virus ou un rétrovirus ou pour le traitement d'une tumeur.

15. Procédé de préparation d'une oligonucléotide dérivé d'un dérivé de 5'-phosphonométhoxytétrahydrofuranyl-2'-purine/pyrimidine tel que préparé selon l'une quelconque des revendications 8 à 11.

16. Procédé de préparation d'un composé de la formule V: 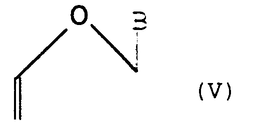 où
B est une base de purine ou de pyrimidine sélectionnée dans le groupe consistant en xanthine, xanthine substituée, guanine, 8-bromoguanine, 8-chloroguanine, 8-aminoguanine, 8-hydrazinoguanine, 8-hydroxyguanine, 8-méthylguanine, 8-thioguanine et 3-déazaguanine, purine, purine substituée, cytosine, cytosine substituée, thymine, uracile, uracile substitué, adénine et adénine substituée,
comprenant l'élimination de H-Y' d'un composé de la formule 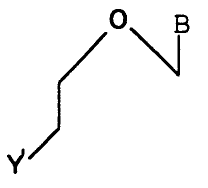 où
Y' est S-Ph, Se-Ph ou un halogène et B a les significations ci-dessus.

17. Procédé de préparation d'un composé de la formule 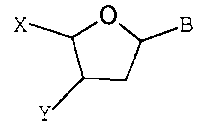 comprenant la réaction d'un composé 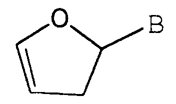 avec X-Y, où
X est un halogène,
Y est S-Ph, Se-Ph, ou un halogène, et
X-Y est un halogène, ZS-Ph ou ZSe-Ph, où Z est un halogène et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

18. Procédé de préparation d'un composé de formule VIII. 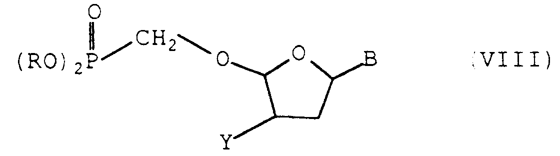 où
Y est un halogène, S-Ph, Se-Ph, R est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée,
comprenant la réaction d'un composé de la formule 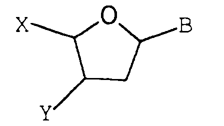 avec un composé de la formule 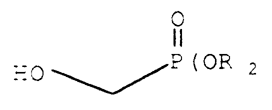 où
X est un halogène, et Y, R et B ont les significations ci-dessus.

19. Procédé de préparation d'un composé de la formule 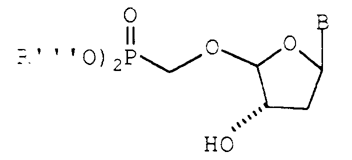 comprenant la réaction d'un composé de la formule 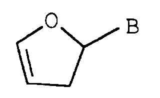 avec un composé de la formule 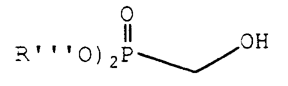 avec un péracide, où R''' est hydrogène ou alkyle avec 1 à 6 atomes de carbone et B est une base de purine ou de pyrimidine sélectionnée dans le groupe consistant en xanthine, xanthine substituée, guanine, guanine substituée, purine, purine substituée, cytosine, cytosine substituée, thymine, uracile, uracile substitué, adénine et adénine substituée.

20. Procédé de préparation d'une composition pharmaceutique qui comprend l'incorporation d'au moins un composé préparé selon l'une des revendications 1 à 13 dans un diluant solide, liquide ou gazeux.
